# EUROPEAN PATENT SPECIFICATION

(11) **EP 3 359 196 B1**
(45) Date of publication and mention of the grant of the patent: **16.03.2022**
(21) Application number: 16782374.9
(22) Date of filing: 05.10.2016
(51) Int. Cl.: A61K 45/06, A61K 31/337, A61K 31/52, A61K 31/704, A61P 35/00

(54) **RATIONAL COMBINATION THERAPY FOR THE TREATMENT OF CANCER**
RATIONALE KOMBINATIONSTHERAPIE ZUR BEHANDLUNG VON KREBS
POLYTHÉRAPIE RATIONELLE POUR LE TRAITEMENT DU CANCER

(30) Priority: 05.10.2015 US 201562237470 P
(43) Date of publication of application: 15.08.2018
(73) Proprietor: Memorial Sloan Kettering Cancer Center, New York, NY 10065 (US)
(72) Inventor: CHIOSIS, Gabriela, New York, New York 10065 (US); TALDONE, Tony, New York, New York 10065 (US); SHRESTHA, Liza, New York, New York 10065 (US); KOREN, John, New York, New York 10065 (US); GOMES-DAGAMA, Erica M., New York, New York 10065 (US); RODINA, Anna, New York, New York 10065 (US)
(74) Representative: Carpmaels & Ransford LLP
(86) International application number: PCT/US2016/055594
(87) International publication number: WO 2017/062520

(56) References cited:
- GOPA IYER ET AL: "A phase I trial of docetaxel and pulse-dose 17-allylamino-17-demethoxygeldanamycin in adult patients with solid tumors", CANCER CHEMOTHERAPY AND PHARMACOLOGY, SPRINGER, BERLIN, DE, vol. 69, no. 4, 29 November 2011 (2011-11-29), pages 1089-1097, XP035035178, ISSN: 1432-0843, DOI: 10.1007/S00280-011-1789-3
- DAVID B SOLIT ET AL: "Inhibition of Heat Shock Protein 90 Function Down-Regulates Akt Kinase and Sensitizes Tumors to Taxol", CANCER RESEARCH, AMERICAN ASSOCIATION FOR CANCER RESEARCH, US, vol. 63, 1 May 2003 (2003-05-01), pages 2139-2144, XP007906848, ISSN: 0008-5472
- Lyuba Varticovski: "Synergy of the Purine-Scaffold HSP90 Inhibitor, PU-H71, with Doxorubicin in Non-Hodgkin's Lymphoma Cell Lines", , 16 November 2007 (2007-11-16), XP055327806, Blood Retrieved from the Internet: URL:http://www.bloodjournal.org/content/11 0/11/1399?sso-checked=true [retrieved on 2016-12-09]
- Krishan Sharma: "HSP70 Inhibitor, YK5, Synergizes with Chemotherapeutic Agents and Prevents Chemoresistance in Acute Myelogenous Leukemia (AML). | Blood Journal", , 16 November 2012 (2012-11-16), XP055327813, Blood Retrieved from the Internet: URL:http://www.bloodjournal.org/content/12 0/21/2476?sso-checked=true [retrieved on 2016-12-09]
- JHAVERI KOMAL ET AL: "Advances in the clinical development of heat shock protein 90 (Hsp90) inhibitors in cancers", BIOCHIMICA ET BIOPHYSICA ACTA. MOLECULAR CELL RESEARCH, vol. 1823, no. 3, 29 October 2011 (2011-10-29), pages 742-755, XP028896528, ISSN: 0167-4889, DOI: 10.1016/J.BBAMCR.2011.10.008

## Description

### 1. BACKGROUND

Protein homeostasis is maintained by the coordinated action of the chaperome, a network of molecular chaperones and the co-chaperones and folding enzymes that assist in their function. In mammalian cells, over 200 genes encode members of the chaperome that together account for -10% of total polypeptide mass of the cell. A majority of these are heat shock proteins (HSPs), with HSP90 and HSP70 constituting 50-60% of the chaperome mass. Aberrant cellular processes such as those that enable replicative immortality in cancer can harness the chaperome to counter burdens placed by proteome malfunctions. However, the deviant stress chaperome species remain poorly characterized, thereby hampering crucial developments in disease biology.

To maintain homeostasis, cells employ intricate molecular machineries comprised of thousands of proteins programmed to execute well-defined functions. Dysregulation of these pathways, through protein mis-expression or mutation, can lead to biological advantages that confer a malignant phenotype. Although at the cellular level such dysregulation may be beneficial *(i.e.,* favoring increased survival), at the molecular level this requires cells to invest energy in maintaining the stability and function of these proteins. It is believed that to maintain these proteins in a pseudo-stable state, cancer cells co-opt molecular chaperones, including HSP90.

In support of this hypothesis, HSP90 is recognized to play important roles in maintaining the transformed phenotype. HSP90 and its associated co-chaperones assist in the correct conformational folding of cellular proteins, collectively referred to as "client proteins", many of which are effectors of signal transduction pathways controlling cell growth, differentiation, the DNA damage response, and cell survival. Tumor cell addiction to deregulated proteins (*i.e.* through mutations, aberrant expression, improper cellular translocation, etc.) can thus become critically dependent on HSP90.

The rationale for HSP90 therapy in various forms of cancers is now well-supported by preclinical and clinical studies including in disease resistant to standard therapy. For instance, studies have demonstrated a notable sensitivity of certain HER2+ tumors to HSP90 inhibitors. In these tumors, 17-AAG (also called Tanespimycin) and 17-DMAG (Alvespimycin) elicited responses even, and in particular, in patients with progressive disease after trastuzumab therapy. Other HSP90 inhibitors, such as PU-H71, when tested pre-clinically in a number of triple-negative breast cancer mouse models, delivered the most potent targeted single-agent anti-tumor effect yet reported in this difficult-to-treat breast cancer subtype.

In Iyer et al. Cancer Chemotherapy and Pharmacology, Vol. 69, No.4, 1089-1097 Nov. 2011, the administration of 17-AAG with docetaxel to patients with advanced solid tumor malignancies is described. In Solit et al. Cancer Res. Vol. 63, 2139-2144, May 2003, the administration of 17-AAG with taxol to patients with breast cancer is described.

In WO 2013/009655, it was shown that the abundance of a particular "oncogenic HSP90" species, which is not dictated by HSP90 expression alone, predicts for sensitivity to HSP90 inhibition therapy, and thus is a biomarker for HSP90 therapy. "Oncogenic HSP90" was defined as the HSP90 fraction that represents a cell stress specific form of chaperone complex, that is expanded and constitutively maintained in the tumor cell context, and that may execute functions necessary to maintain the malignant phenotype. Such roles are not only to regulate the folding of overexpressed (*i.e.* HER2), mutated (*i.e.* mB-Raf) or chimeric proteins (*i.e.* Bcr-Abl), but also to facilitate scaffolding and complex formation of molecules involved in aberrantly activated signaling complexes (*i.e.* STAT5, BCL6). While the tumor becomes addicted to survival on a network of HSP90-oncoproteins, these proteins become dependent on "oncogenic HSP90" for functioning and stability. This symbiotic interdependence suggests that addiction of tumors to HSP90 oncoproteins equals addiction to "oncogenic HSP90".

Furthermore, in WO 2013/009655, it was shown that HSP90 forms biochemically distinct complexes in malignant cells. A major fraction of cancer cell HSP90 retains "housekeeping" chaperone functions similar to normal cells, whereas a functionally distinct HSP90 pool enriched or expanded in cancer cells (*i.e.,* "oncogenic HSP90") specifically interacts with oncogenic proteins required to maintain tumor cell survival, aberrant proliferative features and invasive and metastatic behavior.

Incongruence in the make-up of HSP90-containing chaperome complexes in cancer versus normal cells was initially believed to be a manifestation of up-regulated member molecules. However, it is now proposed that epigenetic modifications dictate the composition of the stress-modified chaperome *See* Taldone, T., Ochiana, S. O., Patel, P. D. & Chiosis, G. Selective targeting of the stress chaperome as a therapeutic strategy. Trends Pharmacol Sci 35, 592-603 (2014) and Mollapour, M. & Neckers, L. Post-translational modifications of Hsp90 and their contributions to chaperone regulation. Biochimica et biophysica acta 1823, 648-655 (2012) In particular, chemical alterations such as post-translational modification, or biochemical changes via co-chaperone and adapter protein pre-recruitment, may instead be the distinguishing characteristic of the stress chaperome species. With their composition and stability likely to be highly dependent on endogenous conditions found in native tumors, these stress chaperome species have resisted investigation by current laboratory approaches in large part due to limitations of methods that disrupt or engineer the cellular environment to facilitate analysis.

Despite the recent advances in understanding the biology of HSP90 function in cancer cells, the underlying nature, structure and function of HSP90 and its role within the chaperome is still not well understood. A more fundamental understanding of the chaperome will be beneficial in developing new targeted therapies for the treatment of cancer.

### 2. SUMMARY OF DISCLOSURE

This invention provides anHSP90 inhibitor as defined by claim 1, a proteotoxic stressor as defined by claim 2 and the combination of an HSP90 inhibitor and a proteotoxic stressor as defined by claim 3 for use in methods of treating cancer according to those claims.

We show that under conditions of stress, such as malignant transformation, the chaperome becomes biochemically "rewired" to form stable, survival- facilitating, high molecular weight complexes. While normal physiological conditions induce a transient existence of the chaperome assembly, we present data highlighting the accumulation of stable, multi-chaperome conglomerates, named the epichaperome, in certain cancer cells. Specifically, we show that cancer cells have reservoirs of preformed chaperome complexes with all their accessories (co-chaperones and auxiliary factors), in anticipation of heightened activity in aberrant cells. We further show that cancer cells that have become biochemically rewired to form these multi-chaperome conglomerates (epichaperome) are dependent on the epichaperome for survival.

Additionally, we show that cancer cells can be induced to form the aforementioned stable, multi-chaperome conglomerates (epichaperome) by the induction of appropriate proteotoxic stress. In certain circumstances, the proteotoxic stress is capable of transforming the transient chaperome assembly that exists under normal physiological conditions into the epichaperome complex defined herein. Under other circumstances, the proteotoxic stress is capable of increasing the stability of an already formed epichaperome. In either case, the proteotoxic stress effectively makes the cancer cells more dependent on HSP90 and other chaperone and co-chaperone proteins in the epichaperome for survival. We show herein that the stressed cancer cells are significantly more amenable to treatment with an inhibitor of at least one of the proteins forming the epichaperome, such as an HSP90 inhibitor. Therefore, we have shown that by pre-treating a tumor with a proteotoxic stressor at a sufficient time prior to administering an inhibitor of one of the proteins forming the epichaperome (e.g., HSP90), the tumor is rendered significantly more sensitive to inhibition therapy as compared to tumors not treated with the proteotoxic stressor. Additionally, we show that pre-treating a tumor with a proteotoxic stressor at a sufficient time prior to administering an inhibitor of one of the proteins forming the epichaperome is significantly more efficacious than concurrent administration of the proteotoxic stressor and an inhibitor of one of the proteins forming the epichaperome (*e.g.*, HSP90).

There are reports of combining Taxol^{®} or doxorubicin with 17-AAG to induce apoptosis in cancer cell lines. See Münster et al.Clin. Cancer Res. Vol. 7, 2228-2236, Aug. 2001; Solit et al. Cancer Res. Vol. 63, 2139-2144, May 2003; and U.S. Patent 7,211,562 (collectively "Rosen and co-workers"). Rosen and co-workers found the efficacy of the combination to be dependent on the status of retinoblastoma protein (Rb) expression in the cell. In particular, Rosen and co-workers show that enhancement of Taxol^{®}-induced apoptosis with 17-AAG was independent of dosing schedule in mutated Rb or Rb-negative cells. Similar results were observed in xenograft experiments. However, in the present invention, methods are not dependent on Rb status and provide superior results irrespective of Rb status. Rosen and co-workers also focus on the role of cell cycle in sensitizing cancer cells to apoptosis. The present invention, however, relates to the induction of the epichaperome as a primary factor leading to the sensitization of the cancer.

Also in contrast to the present invention, Rosen and co-workers found that for some combinations, e.g. doxorubicin and 17-AAG, the sequence of administration is irrelevant. The ensuing examples, however, show that dosing schedule (i.e., sequence of treatment) provies superior results when a proteotoxic stressor (e.g., doxorubicin) is administered prior to an Hsp90 inhibitor as compared to the reverse sequence.

Rosen and co-workers also state that the combination against Rb-positive (e.g., wild-type Rb) cancer cells provides similar results when 17-AAG is given simultaneously vs. "immediately after" Taxol^{®}. See See Münster *et al.pg.* 2234. In contrast, the present invention encompasses the recognition that an Hsp90 inhibitor administered after a proteotoxic stressor provides unexpectedly superior efficacy as compared to other dosing schedules.

Accordingly, the disclosure provides evidence that increasing the proteotoxic stress on the cancer cells through administration of particular proteotoxic stressors increases the sensitivity of the cells to HSP90 inhibition therapy. The proteotoxic stressors are capable of pushing the cancer cells into a state where they have an increased reliance on HSP90 and other chaperone and co-chaperone proteins. The disclosure thereby provides compounds for use in methods of treating cancer using rational combination therapy of a proteotoxic stressor and an HSP90 inhibitor that relies on appropriate timing of the proteotoxic stressor and the HSP90 inhibitor.

The invention provides compounds according to claims 1 to 3 for use in methods for treating cancer by administering to a cancer patient an inhibitor of HSP90 following pretreatment with a proteotoxic stressor.

The proteotoxic stressor is administered at a sufficient time prior to administration of the HSP90 inhibitor to maximize the formation of the epichaperome complex, thereby rendering the tumor most vulnerable to HSP90 inhibition therapy. On the other hand, administration of the HSP90 inhibitor at a time significantly after the epichaperome complex is formed can mitigate the effect of the HSP90 inhibitor as the tumor becomes less dependent on the epichaperome for survival.

In certain embodiments, the HSP90 inhibitor is administered at least one hour after administering an agent that induces a proteotoxic stress on the tumor cells. In other embodiments, the HSP90 inhibitor is administered at least two hours after administering an agent that induces a proteotoxic stress on the tumor cells. In other embodiments, the HSP90 inhibitor is administered at least three hours after administering an agent that induces a proteotoxic stress on the tumor cells. In other embodiments, the HSP90 inhibitor is administered at least four hours after administering an agent that induces a proteotoxic stress on the tumor cells. In other embodiments, the HSP90 inhibitor is administered at least five hours after administering an agent that induces a proteotoxic stress on the tumor cells. In other embodiments, the HSP90 inhibitor is administered at least six hours after administering an agent that induces a proteotoxic stress on the tumor cells. In other embodiments, the HSP90 inhibitor is administered at least seven hours after administering an agent that induces a proteotoxic stress on the tumor cells. In other embodiments, the HSP90 inhibitor is administered at least eight hours after administering an agent that induces a proteotoxic stress on the tumor cells. In other embodiments, the HSP90 inhibitor is administered at least nine hours after administering an agent that induces a proteotoxic stress on the tumor cells. In other embodiments, the HSP90 inhibitor is administered at least ten hours after administering an agent that induces a proteotoxic stress on the tumor cells. In other embodiments, the HSP90 inhibitor is administered at least twelve hours after administering an agent that induces a proteotoxic stress on the tumor cells. In other embodiments, the HSP90 inhibitor is administered at least eighteen hours after administering an agent that induces a proteotoxic stress on the tumor cells. In other embodiments, the HSP90 inhibitor is administered twenty four hours after administering an agent that induces a proteotoxic stress on the tumor cells. In the invention, the HSP90 inhibitor is administered no more than twenty four hours after administering an agent that induces a proteotoxic stress on the tumor cells. In certain embodiments, the proteotoxic stressor is administered at a time in the range between any of the foregoing embodiments, e.g., between about one and three hours prior to the administration of the HSP90 inhibitor, between about two and four hours prior to the administration of the HSP90 inhibitor, between about three and five hours prior to the administration of the HSP90 inhibitor, between about two and six hours prior the administration of the HSP90 inhibitor, between about three and six hours prior to the administration of the HSP90 inhibitor, between about four and six hours prior to the administration of the HSP90 inhibitor, between about four and eight hours prior to the administration of the HSP90 inhibitor, between about four and ten hours prior to the administration of the HSP90 inhibitor, between about five and seven hours prior to the administration of the HSP90 inhibitor and so on, and so forth.

In particular embodiments, the proteotoxic agent is administered parenterally (*e.g.,* intravenously). In such embodiments, the HSP90 inhibitor is administered at a time following completion of the parenteral administration. For instance, the HSP90 inhibitor can be administered one hour, two hours, three hours, four hours, five hours, six hours, seven hours, eight hours, nine hours, ten hours, eleven hours, or twelve hours following completion of the parenteral administration of the proteotoxic agent. In certain embodiments the HSP90 inhibitor can be administered eighteen or twenty four hours following completion of the parenteral administration of the proteotoxic agent. In certain embodiments, the HSP90 is administered at a time in the range between any of the foregoing embodiments, e.g., about one to three hours following completion of the parenteral administration of the proteotoxic agent, about two to four hours following completion of the parenteral administration of the proteotoxic agent, about three to five hours following completion of the parenteral administration of the proteotoxic agent, about two to six hours following completion of the parenteral administration of the proteotoxic agent, about three to six hours following completion of the parenteral administration of the proteotoxic agent, about four to six hours following completion of the parenteral administration of the proteotoxic agent, about four to eight hours following completion of the parenteral administration of the proteotoxic agent, about four to ten hours following completion of the parenteral administration of the proteotoxic agent, about five to seven hours following completion of the parenteral administration of the proteotoxic agent and so on, and so forth.

In another embodiment, HSP90 or another chaperone protein can be induced to form a stable epichaperome complex by a means alternative to stressing the cells that induce a biochemical rewiring of the chaperome. For instance, the stability of the epichaperome complex can be increased by pre-treating cancer cells with modulators of the post-translational modification (PTM) status of HSP90. In one such embodiment, post-translational modification is achieved by limiting the amount of phosphorylation of chaperome proteins. For instance, phosphate groups can be removed by adding a phosphatase. Alternatively, a kinase inhibitor can be added at a time prior to the administration of the HSP90 inhibitor to reduce phosphorylation of the chaperone proteins. In one such embodiment, cancer cells are pretreated with the drug PD407824, an inhibitor of checkpoint kinases Chk1 and Wee1, prior to administration of an HSP90 inhibitor.

The compounds disclosed herein are used in methods to treat a variety of different cancers including but not limited to breast cancer, lung cancer including small cell lung cancer and non-small cell lung cancer, cervical cancer, colon cancer, choriocarcinoma, bladder cancer, cervical cancer, basal cell carcinoma, choriocarcinoma, colon cancer, colorectal cancer, endometrial cancer esophageal cancer, gastric cancer, head and neck cancer, acute lymphocytic cancer (ACL), myelogenous leukemia including acute myeloid leukemia (AML) and chronic myeloid chronic myeloid leukemia (CML), multiple myeloma, T-cell leukemia lymphoma, liver cancer, lymphomas including Hodgkin's disease, lymphocytic lymphomas, neuroblastomas follicular lymphoma and a diffuse large B-cell lymphoma, oral cancer, ovarian cancer, pancreatic cancer, prostate cancer, rectal cancer, sarcomas, skin cancers such as melanoma, testicular cancer, thyroid cancer, renal cancer, myeloproliferative disorders, gastrointestinal cancers including gastrointestinal stromal tumors, esophageal cancer, stomach cancer, a gallbladder cancer, anal cancer, brain tumors including gliomas, lymphomas including follicular lymphoma, and diffuse large B-cell lymphoma.

In some embodiments, the compounds disclosed herein can be used in methods to treat retinoblastoma (Rb)- deficient or Rb-negative cancers. In some embodiments, the compounds disclosed herein can be used in methods to treat breast cancer, lung cancer including small cell lung cancer and non-small cell lung cancer, cervical cancer, colon cancer, choriocarcinoma, bladder cancer, cervical cancer, basal cell carcinoma, choriocarcinoma, colon cancer, colorectal cancer, endometrial cancer esophageal cancer, gastric cancer, head and neck cancer, acute lymphocytic cancer (ACL), myelogenous leukemia including acute myeloid leukemia (AML) and chronic myeloid chronic myeloid leukemia (CML), multiple myeloma, T-cell leukemia lymphoma, liver cancer, lymphomas including Hodgkin's disease, lymphocytic lymphomas, neuroblastomas follicular lymphoma and a diffuse large B-cell lymphoma, oral cancer, ovarian cancer, pancreatic cancer, prostate cancer, rectal cancer, sarcomas, skin cancers such as melanoma, testicular cancer, thyroid cancer, renal cancer, myeloproliferative disorders, gastrointestinal cancers including gastrointestinal stromal tumors, esophageal cancer, stomach cancer, a gallbladder cancer, anal cancer, brain tumors including gliomas, lymphomas including follicular lymphoma, or diffuse large B-cell lymphoma, wherein the cancer is a retinoblastoma (Rb)- deficient or Rb-negative cancer. In some embodiments, the compounds disclosed herein can be used in methods to treat small cell lung cancer, triple-negative breast cancer, HPV-positive head and neck cancer, retinoblastoma, bladder cancer, prostate cancer, osteosarcoma, or cervical cancer, wherein the cancer is a retinoblastoma (Rb)- deficient or Rb-negative cancer.

In some embodiments, the compounds disclosed herein can be used in methods to treat retinoblastoma (Rb)-expressing, Rb-positive, and/ or Rb wild type cancers. In some embodiments, the compounds disclosed herein are used in methods to treat cancers other than retinoblastoma, osteosarcoma, or small-cell lung cancer. In some embodiments, the compounds disclosed herein are used in methods to treat cancers other than breast cancers which are Rb-positive. In some embodiments, the compounds disclosed herein are used in methods to treat cancers other than breast cancers which overexpress HER2. In some embodiments, the compounds disclosed herein are used in methods to treat cancers other than breast cancers which are Rb-positive and overexpress HER2. It will be appreciated that a determination of HER2 overexpression may utilize comparison to an appropriate reference, which in some embodiments is a breast cancer with intermediate, low, or nondetectable HER2 expression, or in other embodiments is another cancer with intermediate, low, or nondetectable HER2 expression.

In the invention, the proteotoxic stressor to be administered prior to the HSP90 is a chemotherapeutic agent. The chemotherapeutic reagent is a microtubule stabilizing agent. The chemotherapeutic agent is provided at a dose that is capable of increasing the levels of epichaperome formed in the cells. In one embodiment, the chemotherapeutic agent may be given at a dosage that is typically administered to cancer patients. In some embodiments, the chemotherapeutic agent (e.g., a taxane such as Abraxane^{®}) is given at the same dosage reflected in the prescribing information (e.g., drug label) for the chemotherapeutic agent. In another embodiment, the chemotherapeutic agent may be given at a dosage that less than the amount typically administered to cancer patients. For instance, the dosage of the chemotherapeutic agent administered to cancer patients can be 80% of the amount, 70% of the amount, 60% of the amount, 50% of the amount, 40% of the amount, 30% of the amount, or 20% of the amount reflected in the prescribing information for the chemotherapeutic agent. In certain embodiments, the chemotherapeutic agent can be administered in an amount between any of the foregoing embodiments, *e.g.*, between 20% and 80% of the amount reflected in the prescribing information for the chemotherapeutic agent, between 40% and 80% of the amount reflected in the prescribing information for the chemotherapeutic agent, between 50% and 70% of the amount reflected in the prescribing information for the chemotherapeutic agent, between 50% and 60% of the amount reflected in the prescribing information for the chemotherapeutic agent, and so on, and so forth.

In the invention, the chemotherapeutic agent to be administered prior to the HSP90 inhibitor is a microtubule stabilizing agent. Particular microtubule stabilizing agents include but are not limited to docetaxel, paclitaxel, cabazitaxel, ixabepilone, vincristine, laulimalide, discodermolids, and epothilones. In one embodiment, the proteotoxic stressor is a protein-bound paclitaxel composition such as Abraxane^{®}. In other embodiment, the proteotoxic stressor is cremaphor-based paclitaxel composition (*e.g.*, Taxol^{®}).

In one embodiment, the subject is undergoing or previously underwent administration of one or more chemotherapeutic agents prior to administration of a dosing regimen in accordance with the disclosure. If the patient is currently on a dosing regimen of a particular chemotherapeutic agent, the dosing regimen may need to be modified in accordance with the disclosure. Accordingly, the disclosure provides compounds for use in methods of treating cancer, said methods comprising administering to a cancer patient an inhibitor of HSP90, the patient having received a proteotoxic stressor a sufficient time prior to administration of the HSP90 inhibitor to increase the formation of the epichaperome.

In one embodiment of the present disclosure, the HSP90 inhibitor to be administered following administration of the proteotoxic stressor is an HSP90 inhibitor that binds directly and preferentially to an oncogenic form of HSP90 (i.e oncogenic HSP90) present in the cancer cells of the patient. In the invention, the HSP90 inhibitor to be administered following administration of the proteotoxic stressor is 8-(6-Iodo- benzo[1,3]dioxol-5-ylsulfanyl)-9-(3-isopropylamino-propyl)-9H-purin-6-ylamine (PU-H71) or pharmaceutically acceptable salt thereof (*e.g.,* HCl salt). PU-H71 can be administered intravenously to a human patient at a dosage ranging from about 5 mg/m² to about 350 mg/m² according to a dosing schedule selected from once weekly, twice weekly, three times weekly, four times weekly, or five times weekly. In the invention, the proteotoxic stressor is generally administered at a predetermined time prior to each administration of PU-H71. In particular embodiments, PU-H71 is administered intravenously to a human patient at a dosage from about 20 mg/m² to about 60 mg/m² according to a dosing schedule selected from once weekly, twice weekly, three times weekly, four times weekly, or five times weekly. In other embodiments, PU-H71 is administered intravenously to a human patient at a dosage from about 60 mg/m² to about 150 mg/m² according to a dosing schedule selected from once weekly, twice weekly, three times weekly, four times weekly, or five times weekly. In other embodiments, PU-H71 is administered intravenously to a human patient at a dosage from about 200 mg/m² to about 350 mg/m² according to a dosing schedule selected from once weekly, twice weekly, three times weekly, four times weekly, or five times weekly. In other embodiments, PU-H71 is administered intravenously to a human patient at a dosage from about 250 mg/m² to about 300 mg/m² according to a dosing schedule selected from once weekly, twice weekly, three times weekly, four times weekly, or five times weekly. In other embodiments, PU-H71 is administered intravenously to a human patient at a dosage from about 250 mg/m² to about 300 mg/m² according to a twice weekly dosing schedule. In other embodiments, PU-H71 is administered intravenously to a human patient at a dosage from about 300 mg/m² to about 350 mg/m² according to a once weekly dosing schedule.

PU-H71 can be administered intravenously to a human patient at a dosage ranging from about 5 mg/m² to about 350 mg/m² according to a dosing schedule selected from once per week, once every two weeks, once every three weeks, or once every four weeks. In accordance with the disclosure, the proteotoxic stressor is generally administered at a predetermined time prior to each administration of PU-H71. In particular embodiments, PU- H71 is administered intravenously to a human patient at a dosage from about 20 mg/m² to about 60 mg/m² according to a dosing schedule selected from once per week, once every two weeks, once every three weeks, or once every four weeks. In other embodiments, PU- H71 is administered intravenously to a human patient at a dosage from about 60 mg/m² to about 150 mg/m² according to a dosing schedule selected from once per week, once every two weeks, once every three weeks, or once every four weeks. In other embodiments, PU- H71 is administered intravenously to a human patient at a dosage from about 200 mg/m² to about 350 mg/m² according to a dosing schedule selected from once per week, once every two weeks, once every three weeks, or once every four weeks. In other embodiments, PU- H71 is administered intravenously to a human patient at a dosage from about 250 mg/m² to
about 300 mg/m² according to a dosing schedule selected from once per week, once every two weeks, once every three weeks, or once every four weeks. In other embodiments, PU-H71 is administered intravenously to a human patient at a dosage from about 250 mg/m² to about 300 mg/m² according to a once every three weeks dosing schedule. In other embodiments, PU-H71 is administered intravenously to a human patient at a dosage from about 300 mg/m² to about 350 mg/m² according to a once every three weeks dosing schedule.

In one aspect, the disclosure provides compounds for use in methods of treating cancer by administering a combination of a proteotoxic stressor (or modulator of the post-translational modification of HSP90) and an HSP90 inhibitor over a treatment cycle of between 7 days and 31 days, wherein the proteotoxic stressor (or modulator of the post-translational modification of HSP90) and the HSP90 inhibitor are administered at least once over said cycle, and wherein each administration of said proteotoxic stressor (or modulator of the post-translational modification of HSP90) is followed by administration of said HSP90 inhibitor. In accordance with the disclosure, administration of the HSP90 inhibitor commences following an increase in epichaperome formation induced by the proteotoxic stressor (or modulator of the post-translational modification of HSP90). In some embodiments, the HSP90 inhibitor is administered at least one hour after administering an agent that induces a proteotoxic stress (or modulator of the post-translational modification of HSP90) on the tumor cells. In other embodiments, the HSP90 inhibitor is administered at least two hours after administering an agent that induces a proteotoxic stress (or modulator of the post-translational modification of HSP90) on the tumor cells. In other embodiments, the HSP90 inhibitor is administered at least three hours after administering an agent that induces a proteotoxic stress (or modulator of the post-translational modification of HSP90) on the tumor cells. In other embodiments, the HSP90 inhibitor is administered at least four hours after administering an agent that induces a proteotoxic stress (or modulator of the post-translational modification of HSP90) on the tumor cells. In other embodiments, the HSP90 inhibitor is administered at least five hours after administering an agent that induces a proteotoxic stress (or modulator of the post- translational modification of HSP90) on the tumor cells. In other embodiments, the HSP90 inhibitor is administered at least six hours after administering an agent that induces a proteotoxic stress (or modulator of the post-translational modification of HSP90) on the tumor cells. In other embodiments, the HSP90 inhibitor is administered at least seven hours after administering an agent that induces a proteotoxic stress (or modulator of the post- translational modification of HSP90) on the tumor cells. In other embodiments, the HSP90 inhibitor is administered at least eight hours after administering an agent that induces a proteotoxic stress (or modulator of the post-translational modification of HSP90) on the tumor cells. In other embodiments, the HSP90 inhibitor is administered at least nine hours after administering an agent that induces a proteotoxic stress (or modulator of the post-translational modification of HSP90) on the tumor cells. In other embodiments, the HSP90 inhibitor is administered at least ten hours after administering an agent that induces a proteotoxic stress (or modulator of the post-translational modification of HSP90) on the tumor cells. In other embodiments, the HSP90 inhibitor is administered at least twelve hours after administering an agent that induces a proteotoxic stress (or modulator of the post- translational modification of HSP90) on the tumor cells. In other embodiments, the HSP90 inhibitor is administered at least eighteen hours after administering an agent that induces a proteotoxic stress (or modulator of the post-translational modification of HSP90) on the tumor cells. In other embodiments, the HSP90 inhibitor is administered twenty four hours after administering an agent that induces a proteotoxic stress (or modulator of the post- translational modification of HSP90) on the tumor cells. In certain embodiments, the proteotoxic stressor or modulator of the post-translational modification of HSP90) is administered at a time in the range between any of the foregoing embodiments, e.g., between about one and three hours prior to the administration of the HSP90 inhibitor, between about two and four hours prior to the administration of the HSP90 inhibitor, between about three and five hours prior to the administration of the HSP90 inhibitor, between about two and six hours prior the administration of the HSP90 inhibitor, between about three and six hours prior to the administration of the HSP90 inhibitor, between about four and six hours prior to the administration of the HSP90 inhibitor, between about four and eight hours prior to the administration of the HSP90 inhibitor, between about four and ten hours prior to the administration of the HSP90 inhibitor, between about five and seven hours prior to the administration of the HSP90 inhibitor and so on, and so forth.

In some embodiments, the treatment cycle may be a 21 day cycle, with the proteotoxic stressor (or modulator of the post-translational modification of HSP90) and the HSP90 inhibitor administered only on day 1 of the cycle. In some embodiments, the treatment cycle may be a 21 day cycle, with the proteotoxic stressor (or modulator of the post-translational modification of HSP90) and the HSP90 inhibitor to be administered twice, three times, four times, five times, six times, seven times or eight times during the cycle. In some embodiments, the treatment cycle may be a 14 day cycle, with the proteotoxic stressor (or modulator of the post-translational modification of HSP90) and the HSP90 inhibitor administered once, twice, three times, four times, five times, or six times over the cycle. In some embodiments, the treatment cycle may be a 28 day cycle, with the proteotoxic stressor (or modulator of the post-translational modification of HSP90) and the HSP90 inhibitor to be administered twice, three times, four times, five times, six times, seven times, eight times, nine times, or ten times during the cycle. In some such embodiments, the proteotoxic stressor (or modulator of the post-translational modification of HSP90) and the HSP90 inhibitor may be administered on days 1, 8, and 15 of the 28 treatment cycle. In other embodiments, the treatment cycle may be a 7 day cycle, with the proteotoxic stressor (or modulator of the post-translational modification of HSP90) and the HSP90 inhibitor to be administered on day 1 of the cycle.

In certain embodiments, the HSP90 inhibitor (or modulator of the post-translational modification of HSP90) is administered only on days when the proteotoxic stressor (or modulator of the post-translational modification of HSP90) is administered. In other embodiments, the HSP90 inhibitor is administered on days when the proteotoxic stressor (or modulator of the post-translational modification of HSP90) is administered and on days when the proteotoxic stressor (or modulator of the post-translational modification of HSP90) is not administered. For instance, in a two week cycle, the HSP90 inhibitor and the proteotoxic stressor (or modulator of the post-translational modification of HSP90) may be administered on days 1 and 8 of the cycle and the HSP90 inhibitor can be administered by itself on days 4 and 11 of the cycle.

In another aspect, which is not part of the present invention, the disclosure provides methods of determining whether the epichaperome is present in a tumor. The Applicant has discovered that the larger the amount of formation of the epichaperome in the cancer cells, the more dependant the cancer cells are on the epichamerome for survival and proliferation. The present disclosure provides biochemical assays that enable the detection of the epichaperome in tumors. In this manner, the epichaperome provides a biochemical signature that can be exploited as a biomarker.

Due to its occurrence in several tumour types irrespective of genetic or tissue attributes, this biomarker provides a means of patient stratification for the identification of tumours that will respond to several HSP90 and HSP70 inhibitor drugs already in clinical or late preclinical evaluations. The disclosure also demonstrates that tumors that have occurrence of the epichaperome are much more amenable to treatment to a pharmacological agent that targets chaperone and co-chaperone members (*e.g.*, HSP90 or HSP70) than tumors that do not have the epichaperome complex. Moreover, the abundance of the epichaperome complex, as measures by the biochemical techniques disclosed herein, is indicative of the vulnerability of the cancer cells to respond to pharmacological agents targeting chaperome members.

In one embodiment, which is not part of the claimed invention, the presence of the epichaperome complex or lack thereof is determined using a capillary-based platform that combines isoelectric focusing with immunoblotting capabilities. This methodology uses an immobilized pH gradient to separate native multimeric protein complexes based on their isoelectric point (pI), and then allows for probing of immobilized complexes with specific antibodies. Moreover, it does so using only minute amounts of sample, enabling the investigation of primary specimens. In another embodiment, which is not part of the claimed invention, the presence of the epichaperome complex or lack thereof is determined using native PAGE.

### 3. BRIEF DESCRIPTION OF FIGURES

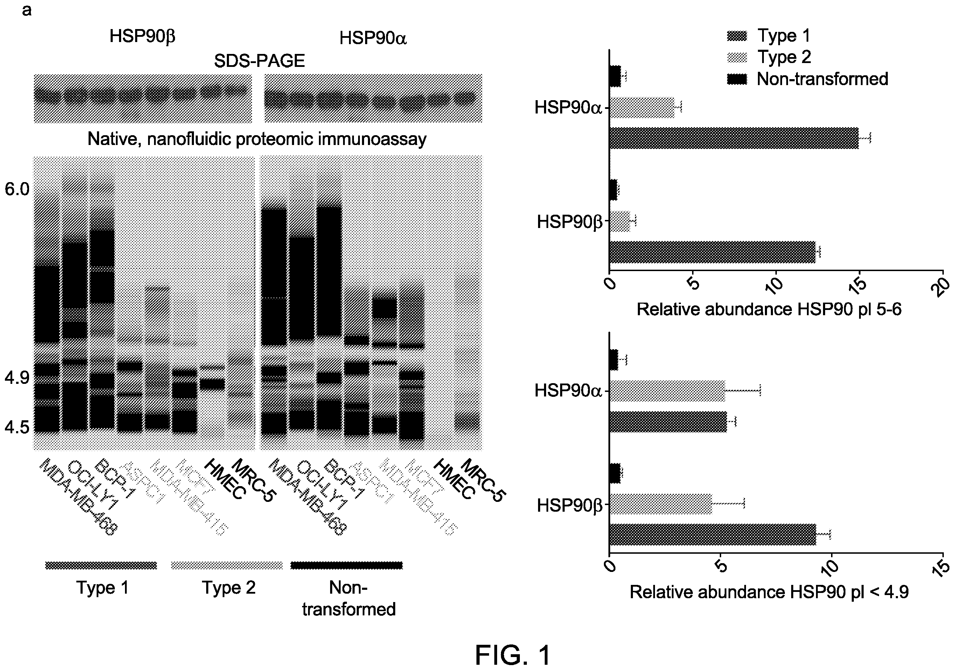

**FIGURES 1a-1d** show certain cancer cells but not all are enriched in stable, multi-chaperome complexes of which HSP90 is a key component. **1a,1b)** Cell homogenates were analyzed under native capillary isoelectric focusing separation conditions and probed with antibodies against HSP90α and HSP90β. PT; primary tumor, NPT; normal tissue adjacent to tumor. Mean ± s.d. from two technical replicates is shown. 1**c**) The biochemical profile of HSP90 in cultured cells and in primary tumors. TNBC, triple-negative breast cancer. **1d)** The biochemical profile of several chaperome members under native and denaturing gel conditions. IB, immunoblotting. All data are representative of two or three independent experiments.

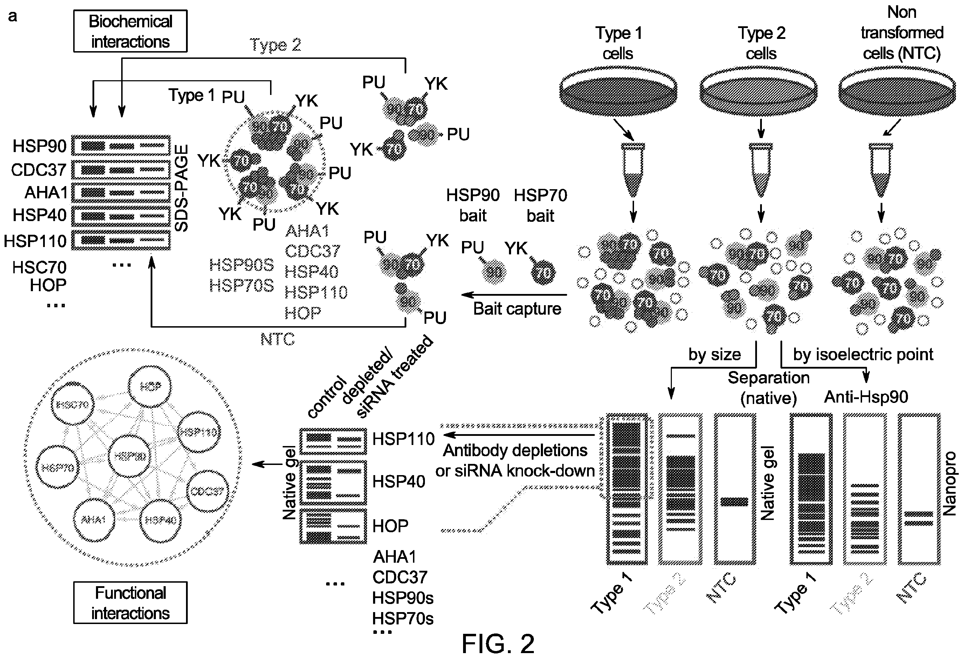

**FIGURES 2a-2c** show interconnected, stable multimeric chaperome complexes are found in type 1 cancer cells. **2a)** Schematic for inquiry into functional and biochemical relationships between key chaperome members in type 1, 2, and non-transformed cells. **2b)** Changes in multimeric chaperone complexes in type 1 cancer cell homogenates depleted with antibodies against HSP70, HSC70, and HOP, and for cells in which AHA-1 was knocked-down by a specific siRNA. **2c)** The cargo or interacting proteins of HSP90 and HSP70 isolated by their respective chemical baits from indicated cell homogenates. Levels of proteins in the homogenate and isolate were probed by Western blot. All data were repeated independently twice or trice with representative shown.

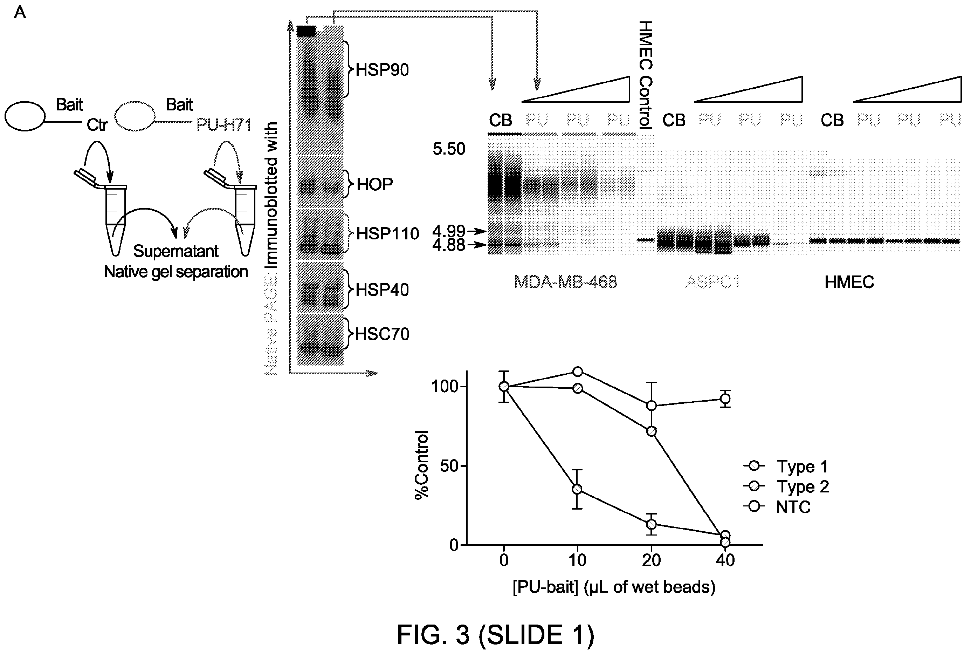

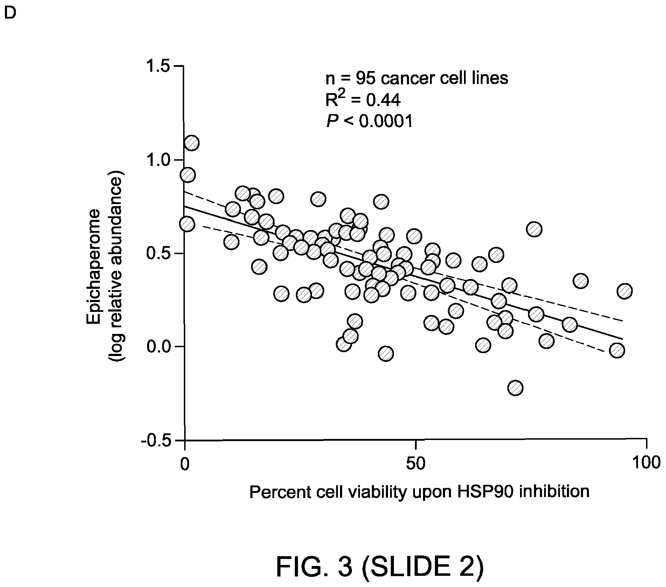

**FIGURES 3a-3g** demonstrate the epichaperome facilitates cancer cell survival. **3a)** Lysates of the indicated cells MDAMB468 (Type 1, high epichaperome, sensitive to HSP90 inhibitors), ASPC1 (Type 2, low to no epichaperome, resistant to HSP90 inhibitors) and HMEC (non-transformed) were incubated with increasing amounts of PU-H71-beads (PU-H71 attached to a solid support) or with control beads (beads with an inert chemical attached) **3b)-3d)** Correlative analysis of epichaperome abundance as measured by PU-FITC capture and cell viability upon a 48 h treatment with PU-H71 as measured by Annexin V staining. Each data point represents a cell line; data are the mean from two technical replicates. **3e)** PU-H71-sensitivity of cells in which AHA-1 levels were reduced by siRNA or scramble control. Cells were treated for 24 h with increasing concentrations of PU-H71. Mean ± s.d. from two technical replicates and representative Western blots are shown. **3f)** Genetic lesions corresponding to **FIG. 3d****. 3g)** Apoptotic sensitivity of type 1 and type 2 cancer cells treated 48 h with the indicated HSP90 agents, as measured by Annexin V staining.

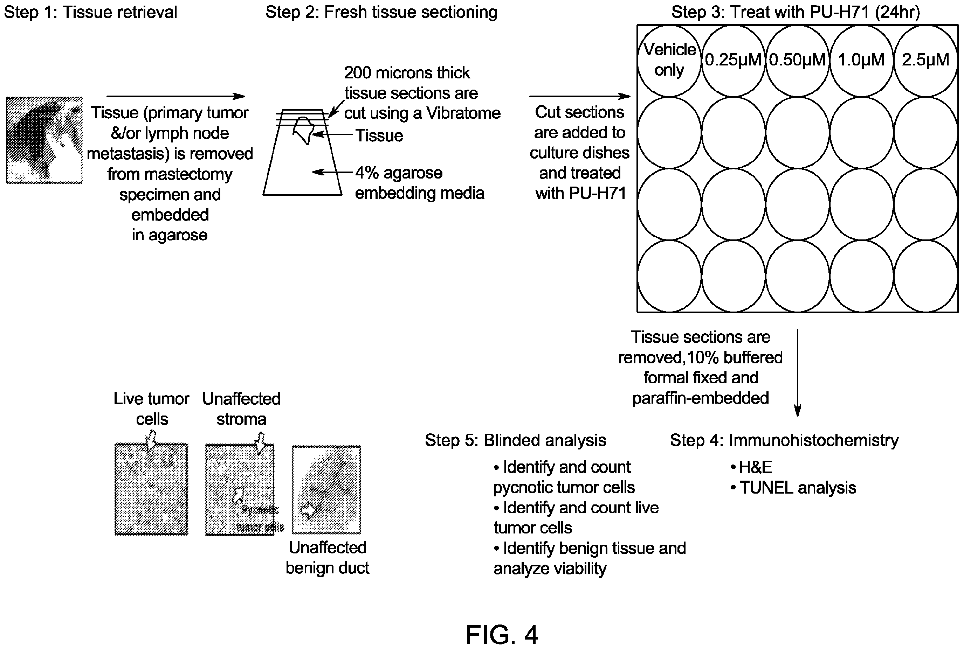

**FIGURE 4** shows treatment schematic *ex vivo* studies of Example 6.

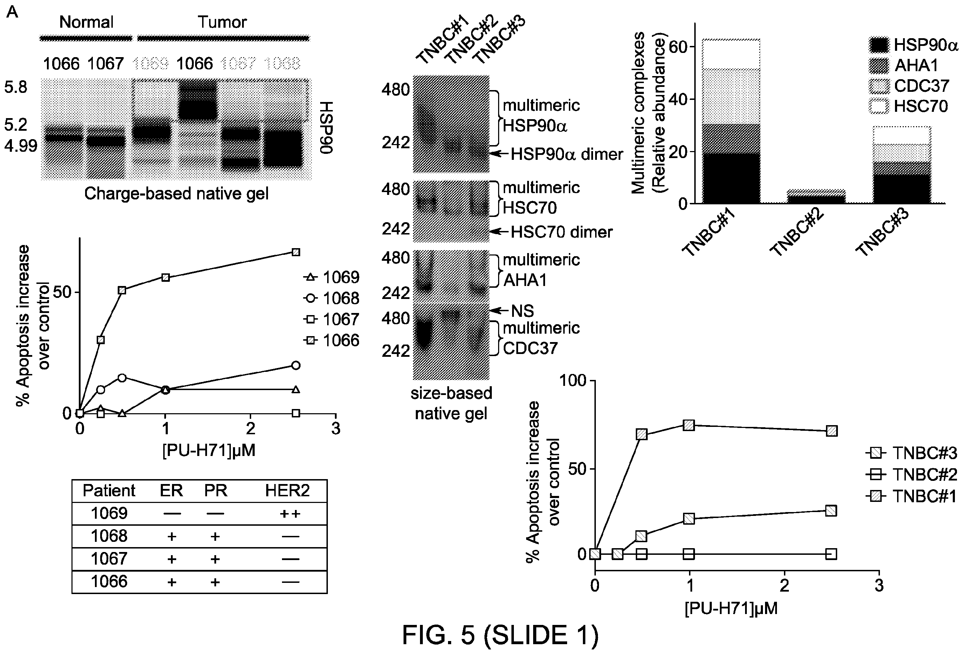

**FIGURES 5a- 5e** demonstrate *ex vivo* studies which show epichaperome positive tumors are sensitive to HSP90 inhibition. **5a)** shows the sensitivity and epichaperome expression of primary breast cancer specimens treated ex vivo with the HSP90 inhibitor PU-H71. **5b)** representative examples of primary breast cancer specimens treated *ex vivo* with PU-H71. **5c)** representative examples of acute myeloid leukemia specimens treated *ex vivo* with PU-H71. **5d)** sensitivity of acute myeloid leukemia specimens treated *ex vivo* with the HSP90 inhibitor PU-H71. **5e)** further shows shows the sensitivity and epichaperome expression of primary breast cancer specimens treated ex vivo with the HSP90 inhibitor PU-H71.

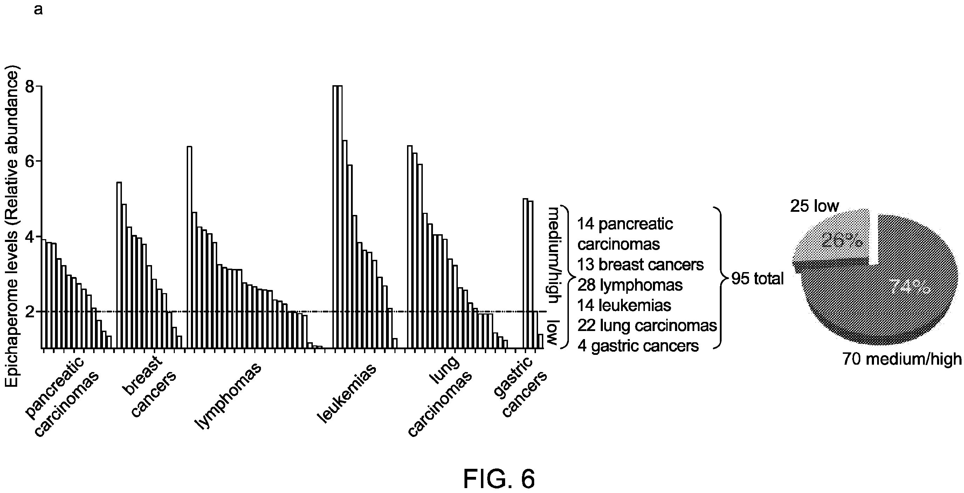

**FIGURES 6a-6c** demonstrate that not all tumours depend on the epichaperome but more than half do. **a-c)** Epichaperome abundance determined by PU-FITC in a panel of 95 cancer cell lines **(a)** and 40 primary AMLs **(b)** and by PU-PET in 50 solid tumours (c). For PU-PET, cross-sectional CT and PU-PET images of representative solid tumours are shown each at the same transaxial plane. Location of the tumours is indicated by arrows.

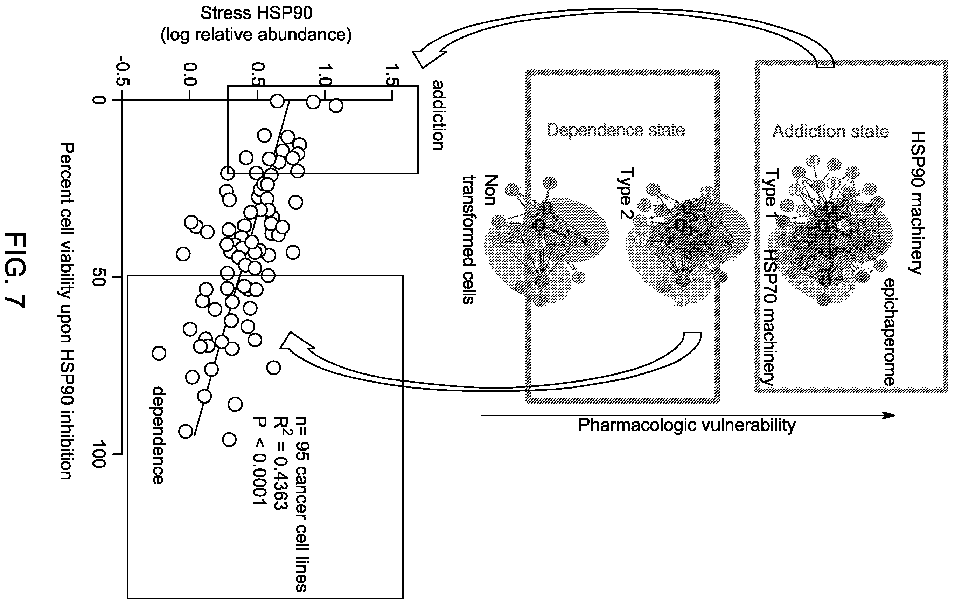

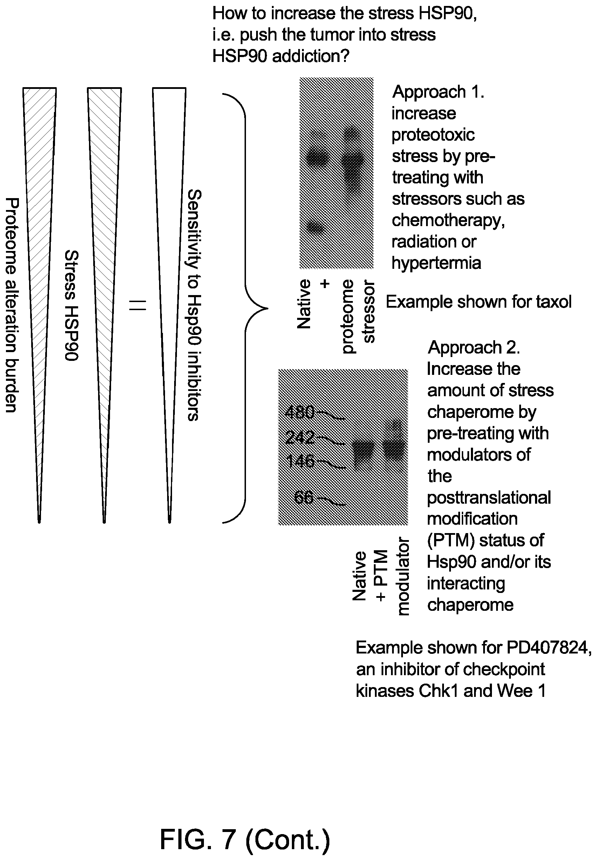

**FIGURE 7** shows that that increasing the stress of cancer cells by introduction of proteotoxic stressor increases the pharmacologic vulnerability of the cancer cells. The diagram on the left shows that when cells are pushed into a state of HSP90 addiction following introduction of the proteotoxic stressor, the stable epichaperome complex is formed. As shown on the bottom left, cells in the stressed state (*i.e.,* greater epichaperome formation) are more vulnerable to HSP90 therapy. The diagram on the right shows several means to increase epichaperome formation, including adding a chemotherapeutic agent or pre-treating with a modulator of the posttranslational modification (PTM) status of HSP90 and/or its interacting chaperome.

**FIGRUES 8a-8f** show treatment of breast and pancreatic cancer cells with a taxane before the addition of an HSP90 inhibitor (exemplified for PU-H71) leads to an increase in the stress chaperome levels and increased cytotoxicity when compared to either compound alone or added in the reverse sequence. **8a, 8b)** The biochemical profile of HSP90 and several chaperome members under native and denaturing gel conditions in cells treated with vehicle, a positive control, paclitaxel or bortezomib. **8c)** viability of MiaPaCa2 pancreatic cancer cells treated with the sequential combinations of vehicle, PU-H71 and Docetaxel as indicated. Cell viability at 72h was measured using the Sulforhodamine B assay. Note that PU before taxane is antagonistic. **8d)** The biochemical profile of HSP90 under native conditions. Cells were pretreated with paclitaxel for 1h then drug was washed off to mimic *in vivo* conditions. Note that the epichaperome levels (labeled top > dimer) follow a bell shape with a peak noted at 5-7h, then decrease to endogenous levels by 24h. **8e, 8f),** The biochemical profile of HSP90 under native and denaturing gel conditions in the indicated cancer cells (breast, pancreatic and lung are shown) treated with the indicated chemotherapeutic agents. Experimental conditions that resulted in the increase of the epichaperome are indicated by an asterisk.

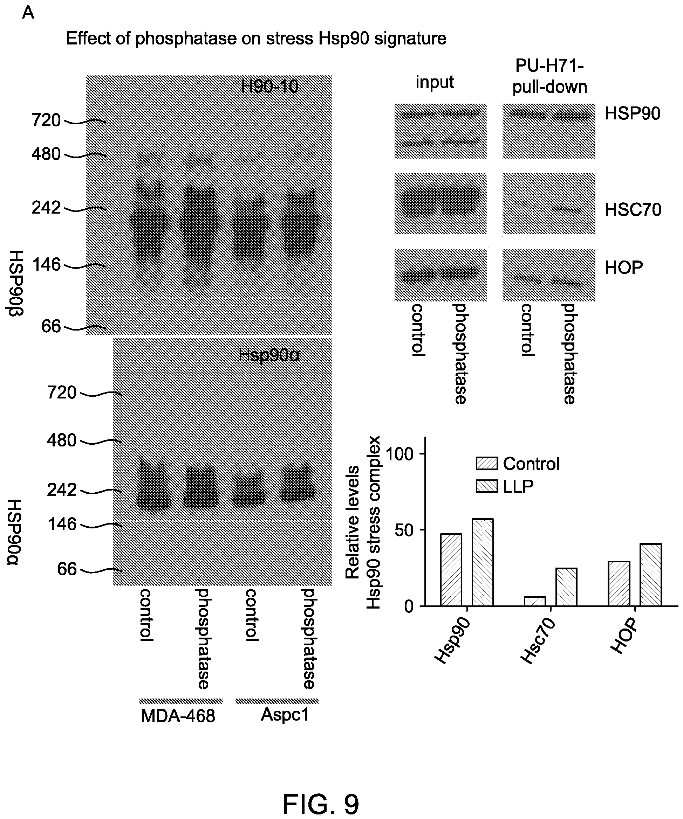

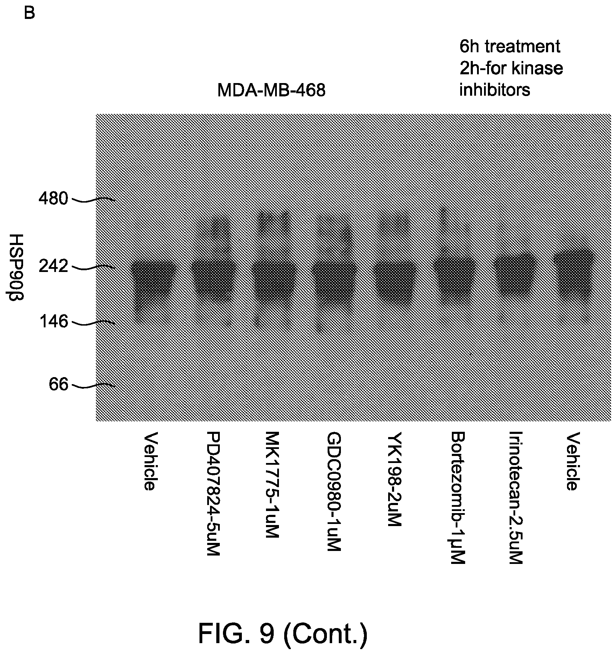

**FIGURES 9a** **and** **9b****.** demonstrate modulation of the epichaperome by post-translational modification alteration - effect of phosphorylation. **9a)** The biochemical profile of HSP90 under native and denaturing gel conditions in cancer cell lysates treated with vehicle or a phosphatase (LPP). On the right, the binding profile of PU-H71 attached to a solid support. Note an increase in the stress HSP90 complexes in lysates treated with LPP, suggesting the potential role for phosphorylation in inhibiting or limiting the formation of the epichaperome. **9b)** The biochemical profile of HSP90 under native gel conditions in the indicated breast cancer cell treated with the indicated kinase inhibitors and chemotherapeutic agents. Note that inhibition of certain kinases, but not all, results in an increase in the cellular levels of the epichaperome. YK198 is an allosteric HSP70 inhibitor.

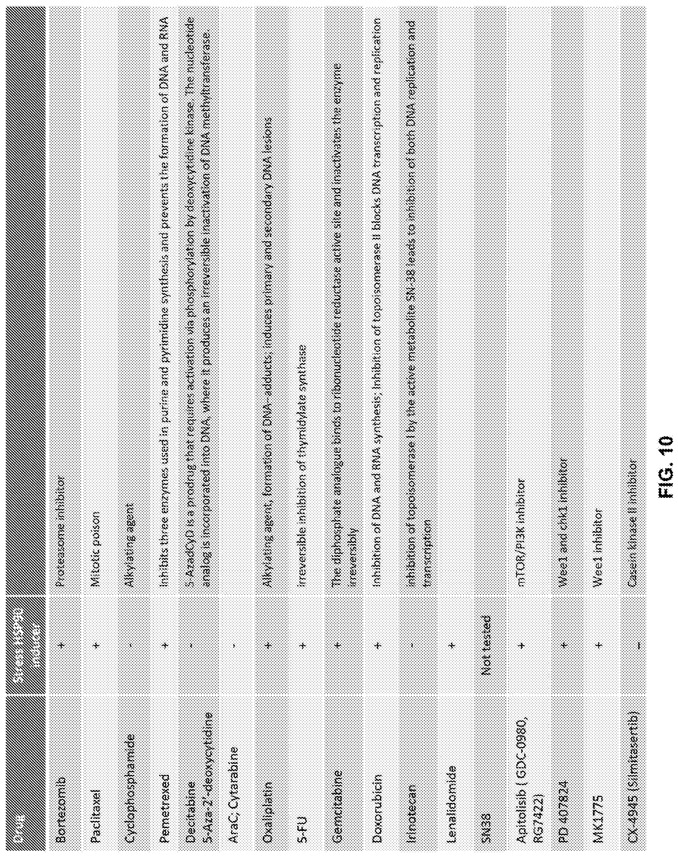

**FIGURE 10** shows particular proteotoxic stressors able to induce epichaperome formation.

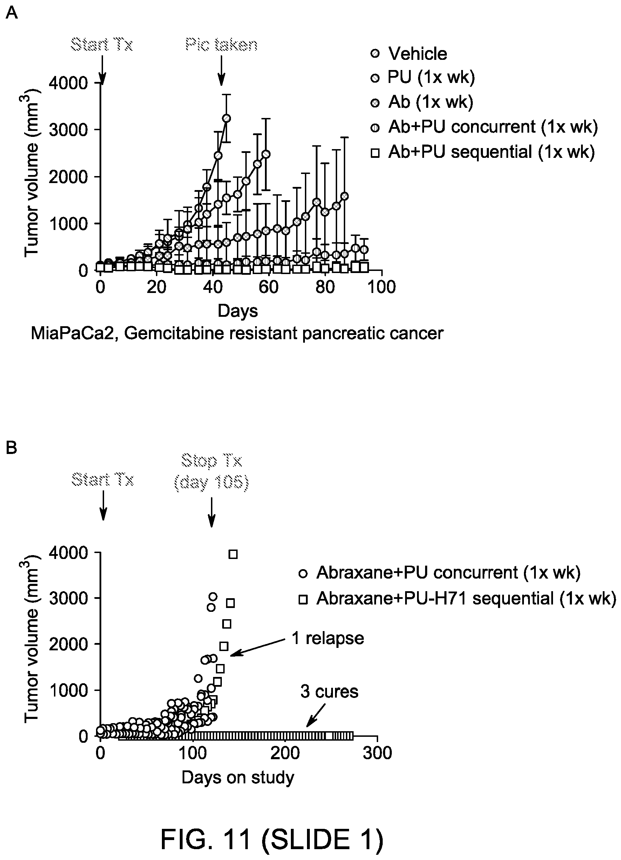

**FIGURES 11a- 11e** demonstrate an *in vivo* study to monitor the efficacy and safety of PU-H71 and Abraxane^{®} when administered under the indicated treatment paradigms to mice bearing xenografted MiaPaCa2 pancreatic cancer tumors. **11a, 11b)** Sequential denotes Abraxane^{®} followed at 6h by PU-H71. Ab, abraxane^{®}, PU, PU-H71. Tumor volume is indicated as A. average +/- SD and B. for individual mice. Agents were administered once a week (1xwk) by ip injection. **11c, 11d)** Mouse weight was monitored twice weekly. **11e)** The effect on tumor regression of Abraxane^{®} and PU-H71 administered concurrently or in the sequence Abraxane^{®} followed by PU-H71 at 1h, 3h or 6h, respectively. Measured volume **(11f)** of xenografted tumors and mouse weight **(11g)** as measured on day 28 of the indicated treatment paradigms.

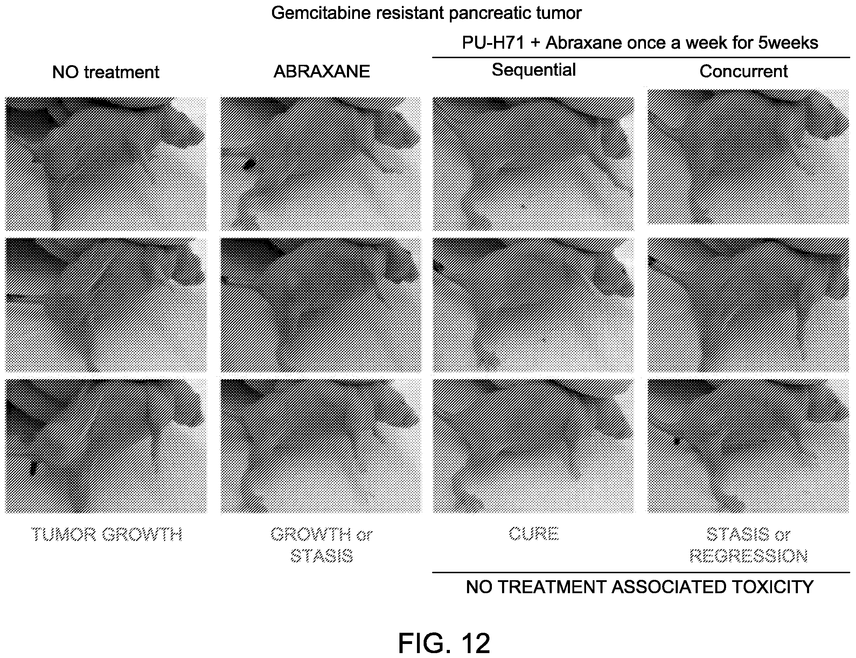

**FIGURE 12** shows pictures of representative mice taken five weeks into the treatment regimen of PU-H71 and Abraxane^{®} when administered under the indicated treatment paradigms to mice bearing xenografted MiaPaCa2 pancreatic cancer tumors. Sequential treatment of PU-H71 6h after Abraxane^{®} administration resulted in a cure while concurrent treatment of PU-H71 and Abraxane^{®} resulted in stasis or regression.

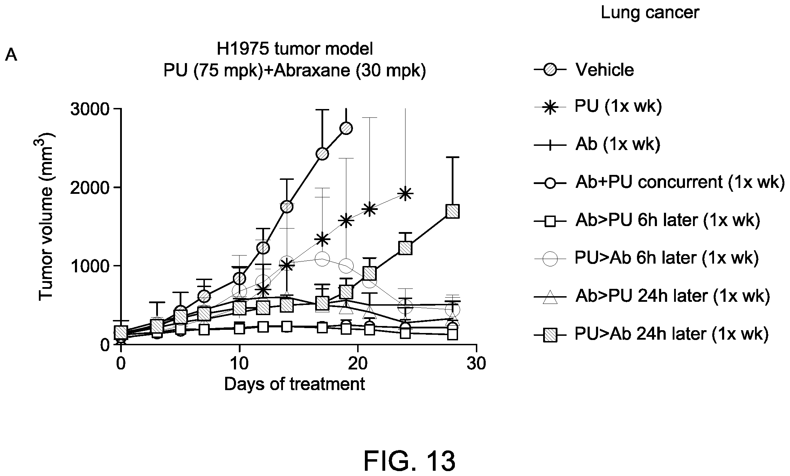

**FIGURES 13a-13e** demonstrate an *in vivo* study to monitor the efficacy and safety of PU-H71 and Abraxane when administered under the indicated treatment paradigms to mice bearing xenografted NCI-H1975 EGFR mutant non-small cell lung cancer tumors. 13a) PU-H71 was administered 6 h or 24 h before Abraxan^{®}e (PU- >Ab 6 h later; PU- >Ab 24 h later) or vice versa (Ab->PU 6 h later; Ab->PU 24 h later). Each agent was also administered alone or combined, concurrently. Agents were administered once a week (1xwk) by ip injection. **13b)** The concurrent and the Ab->PU 6 h arms were treated and monitored as indicated. Note that while on the concurrent arm, all mice relapsed around day 100, cures were noted on the sequential Ab->PU 6 h arm. 13c) The effect of Ab-> PU 6 h on tumors relapsing on Abraxane^{®}. No weight loss was recorded (not shown). Measured volume of xenografted tumors on day 14 **(13d)** and day 28 **(13e)** of indicated treatment paradigms

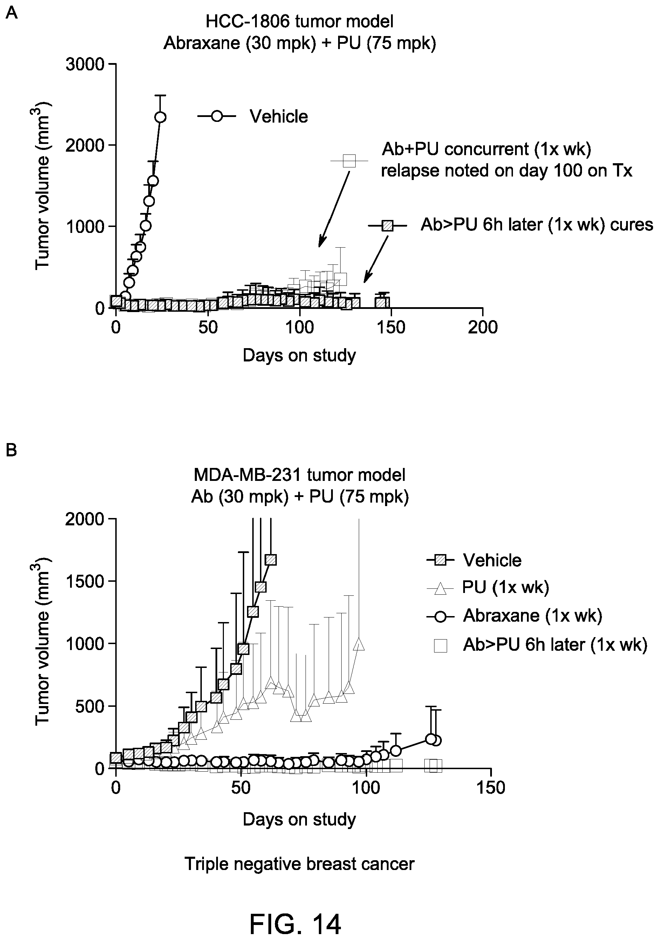

**FIGURES 14a -14d** demonstrate an *in vivo* study to monitor the efficacy and safety of PU-H71 and Abraxane^{®} when administered under the indicated treatment paradigms to mice bearing xenografted breast cancer tumors. **14a, 14c, and 14d)** shows results for the HCC-1806 tumor model; **14b)** shows results for the MDA-MB-231 tumor model.

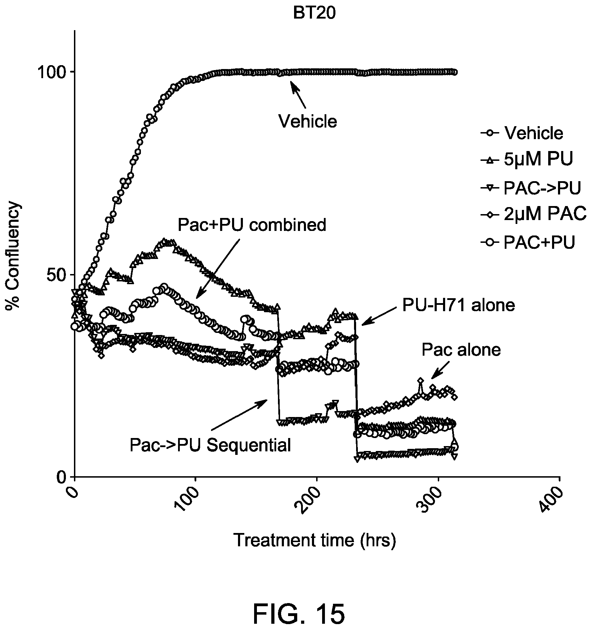

**FIGURE 15** shows results of Inucyte Kinetic growth assay (EXAMPLE 11).

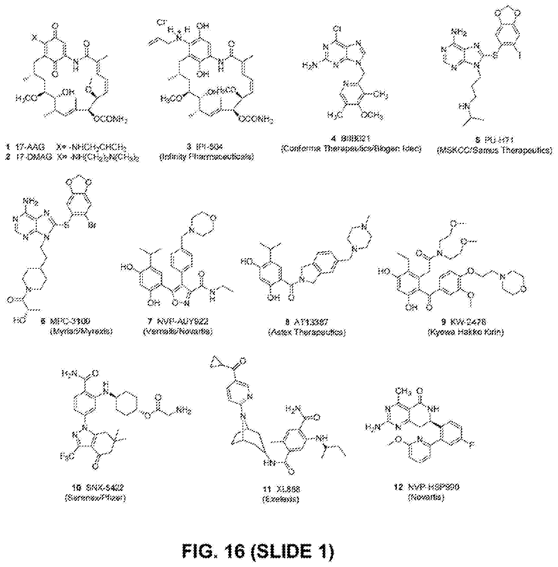

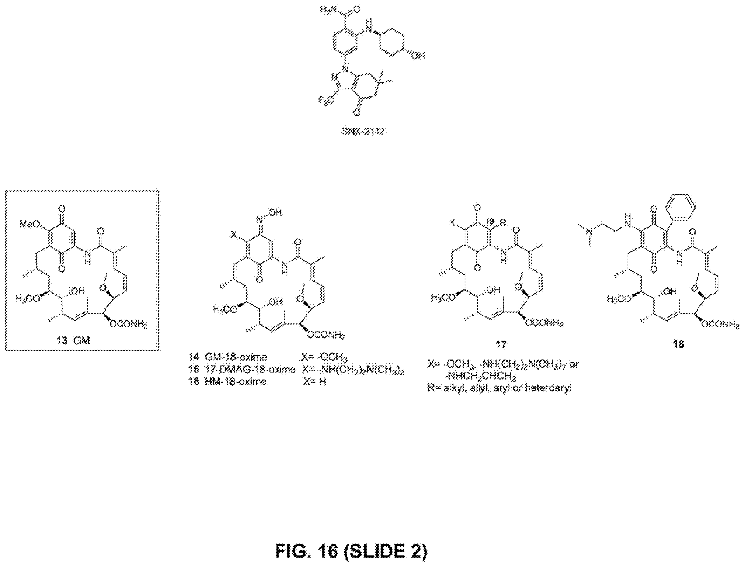

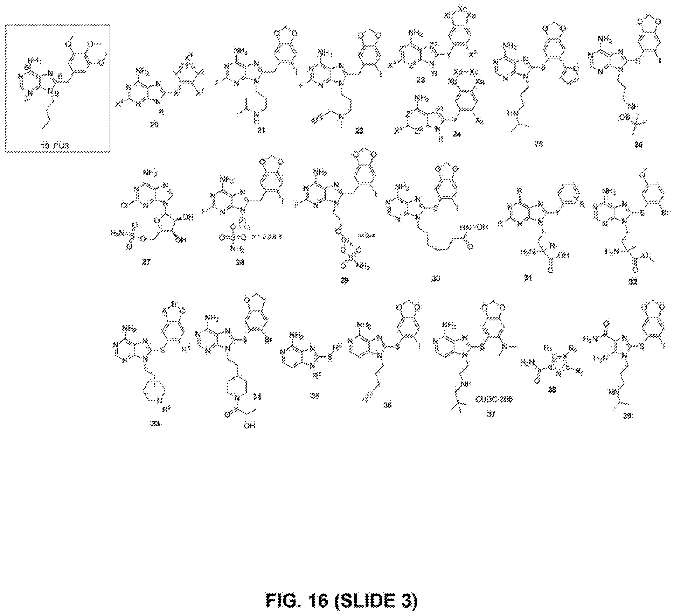

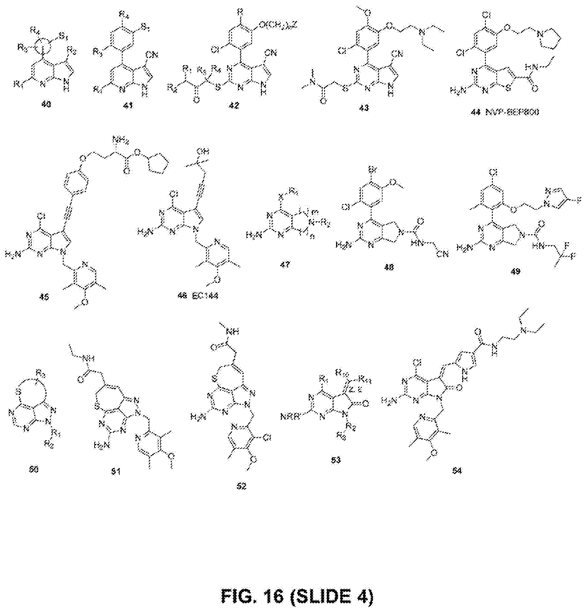

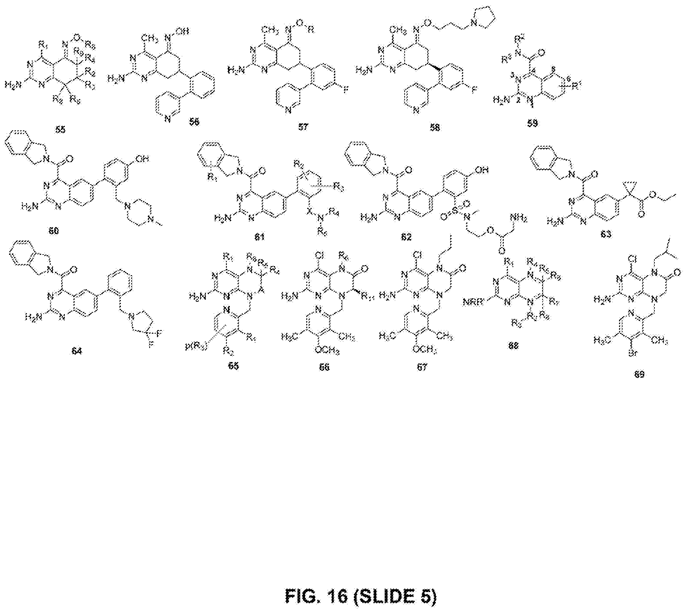

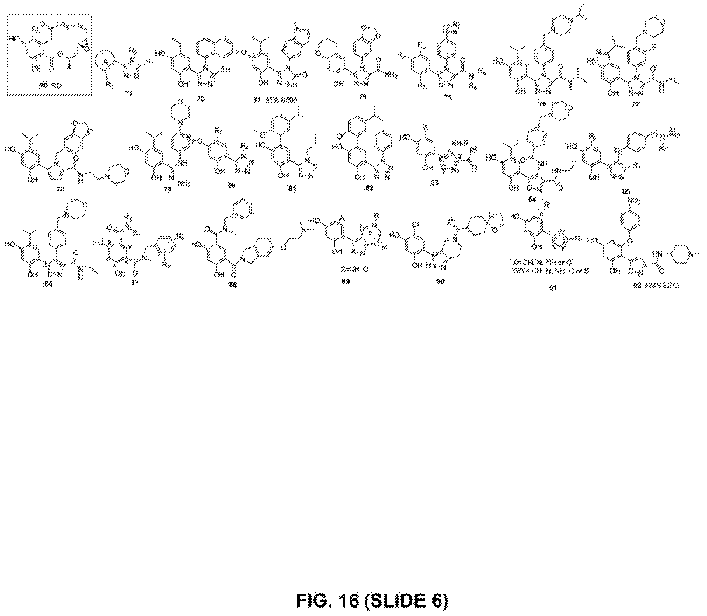

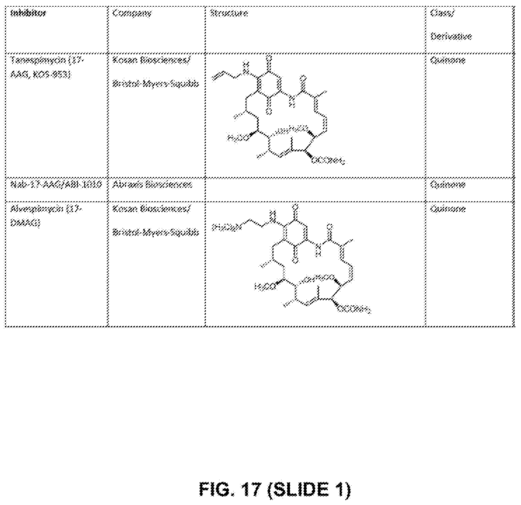

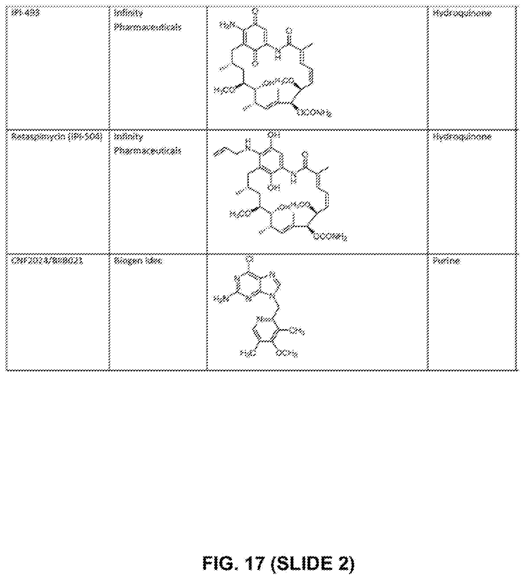

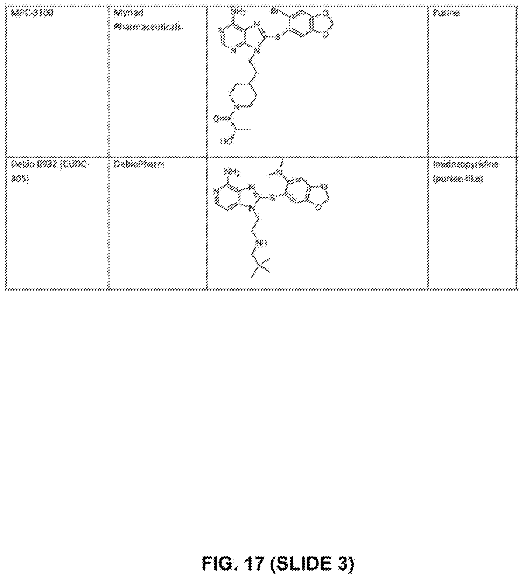

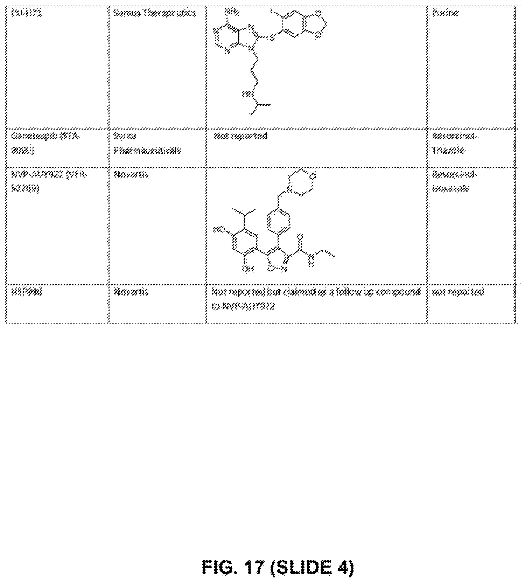

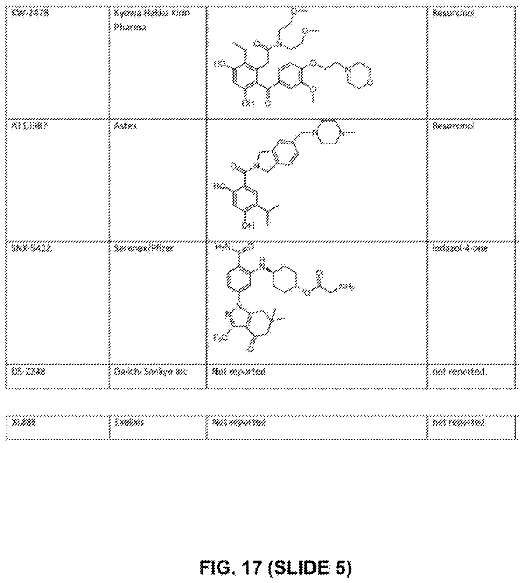

**FIGURES 16** **and** **17** show structures of HSP90 inhibitors administered in accordance with methods of the disclosure.

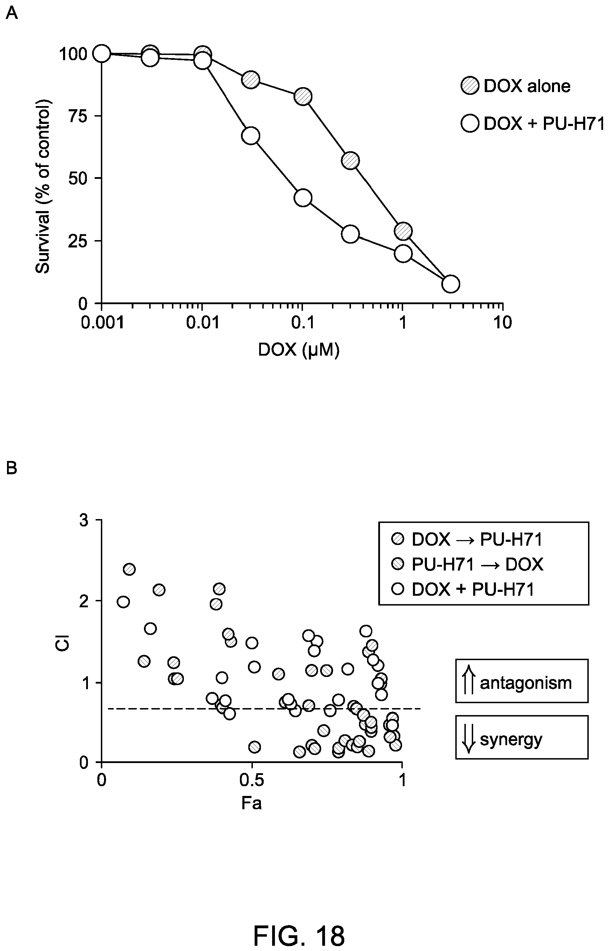

**FIGURES 18a-18c** further demonstrate the efficacy of an Hsp90 inhibitor administered subsequent to a chemoproteomic stressor. **18a** shows viability of Burkitt lymphoma cells exposed to serial dilutions of doxorubicin (DOX) prior to addition of PU-H71. **18b** shows combination index values for dosing combinations of DOX followed by PU-H71 (brown circles), PU-H71 followed by DOX (blue circles) or concurrent administration (black circles) to DLBCL cell. **18c** shows caspase-3 activation as measured by flow cytometry in DLBCL cells exposed to the indicated drug combinations.

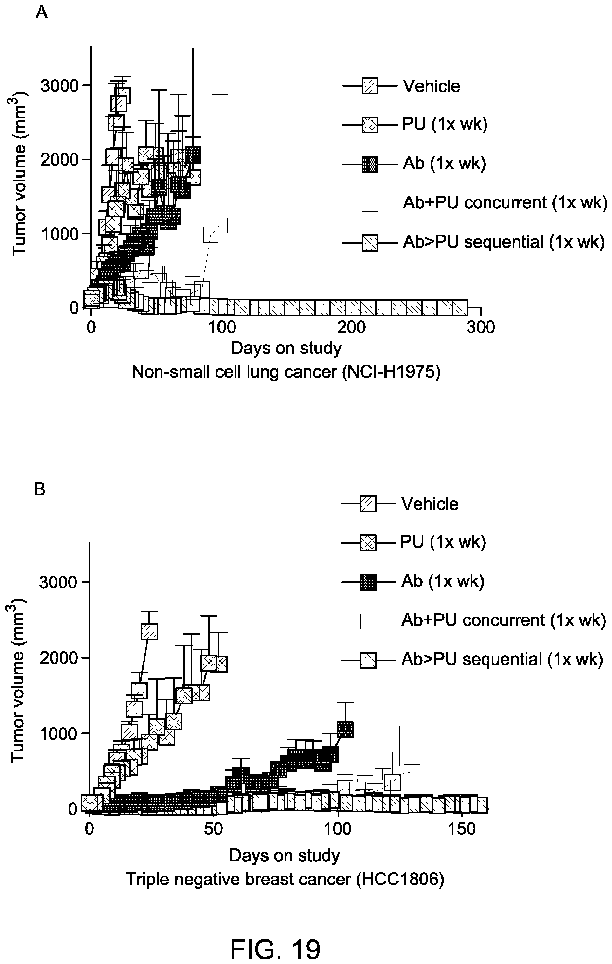

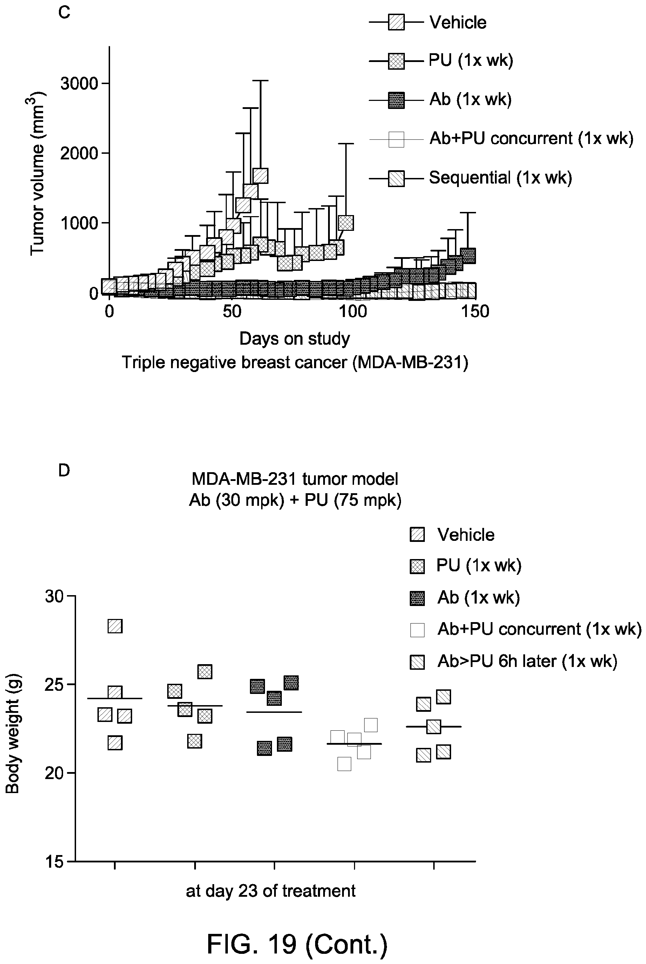

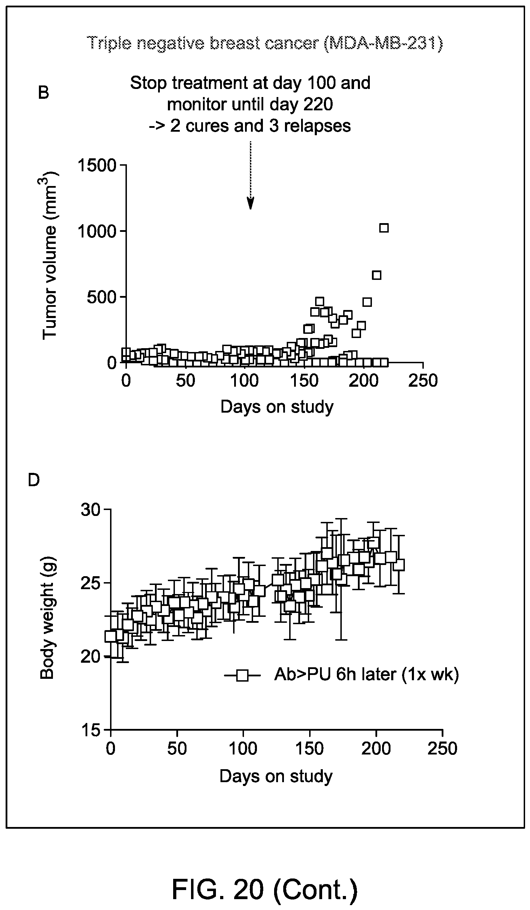

**FIGURES 19a-19d** further demonstrate the efficacy and safety of PU-H71 and Abraxane^{®} when administered under the indicated treatment paradigms to mice bearing indicated xenografted tumors (n=3-5). Tumor volume is indicated as mean +/- SD or for individual mice. Agents were administered once a week (1xwk). PU; PU-H71 at 75mpk, ip; Ab, Abraxane^{®} at 30mpk, ip; Ab->PU sequential denotes Abraxane^{®} followed by PU-H71 at 6h. Tumor growth (19a-19c) under the above treatment paradigms and body weight (19d, presented in the following order "Vehicle", left most column, followed by "PU (1x wk)", "Ab(1x wk)", Ab+ PU concurrent (1x wk)", and right most "Ab>PU 6h later (1x wk)") was monitored in mice bearing the indicated tumors a, NCI-H1975 lung cancer, b, HCC1806, triple negative breast cancer, c, MDA-MB-231, triple negative breast cancer. For long-term body weight monitoring see Figures 20c and 20d.

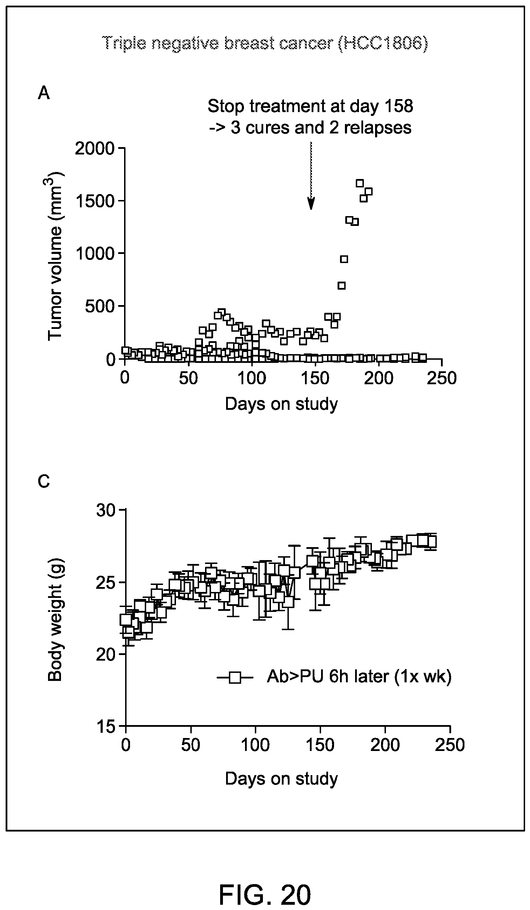

**FIGURES 20a-20d** further demonstrate the efficacy and safety of PU-H71 and Abraxane^{®} when administered under the indicated treatment paradigms to mice bearing indicated xenografted tumors (n=5). Tumor volume is indicated for individual mice (A,B) and mouse body weight as mean±SEM (C,D). Agents were administered once a week (1xwk). PU; PU-H71 at 75mpk, ip; Ab, Abraxane^{®} at 30 mpk, ip; Ab->PU sequential denotes Abraxane^{®} followed by PU-H71 at 6h. Mice received >20 consecutive cycles of weekly sequential Abraxane^{®}/PU-H71 with no clinical signs of toxicity observed and no weight loss.

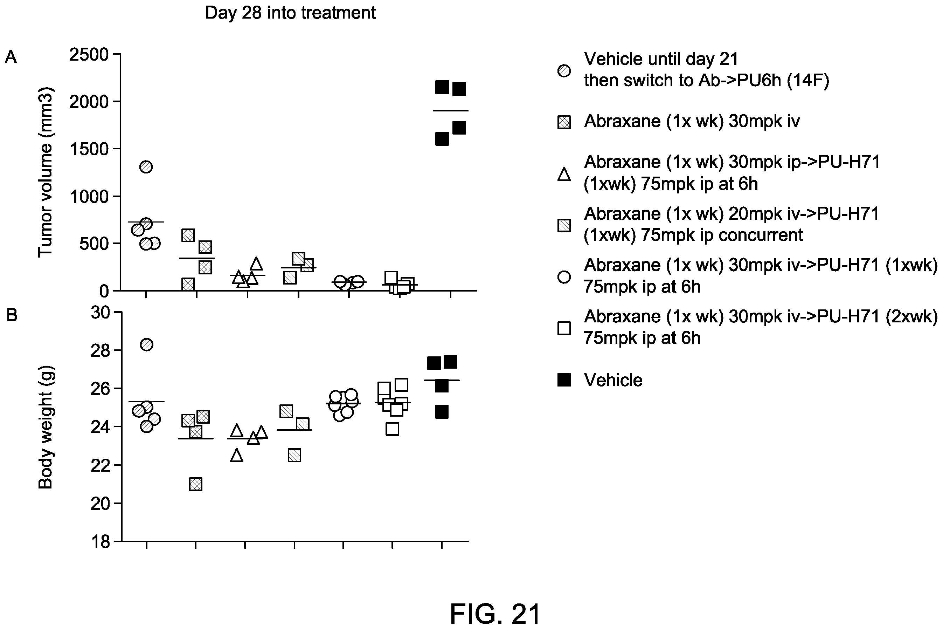

**FIGURES 21a and 21b** further demonstrate the efficacy and safety of PU-H71 (75 mg/kg) and Abraxane^{®} (30 mg/kg) when administered under the indicated treatment paradigm to mice (n=4-5) bearing xenografted MiaPaCa2 4-pancreatic cancer tumors. Tumor volume (a) and mouse weight (b) values at day 28 into treatment are presented as mean +/-SEM. The data are presented in the following order from left column to right column: "Vehicle until day 21 then switch to AB>PU 6h (14F)", "Abraxane (1x wk) 30mpk iv", "Abraxane (1x wk) 30mpk iv", "Abraxane (1x wk) 30mpk ip>PU-H71 (1x wk) 75mpk ip at 6h", "Abraxane (1x wk) 30mpk iv>PU-H71 (1x wk) 75mpk ip concurrent", "Abraxane (1x wk) 30mpk iv>PU-H71 (1x wk) 75mpk ip at 6h", Abraxane (1x wk) 30mpk iv>PU-H71 (2x wk) 75mpk ip at 6h", "Vehicle".

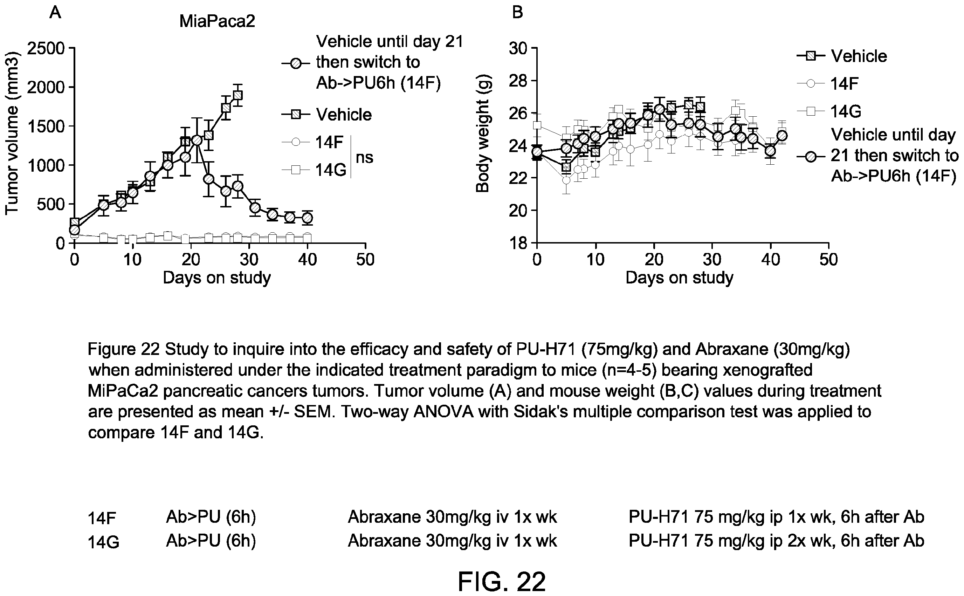

**FIGURES 22a- 22c** further demonstrate the efficacy and safety of PU-H71 and Abraxane^{®} when administered under the indicated treatment paradigm to mice (n=4 5) bearing xenografted MiaPaCa2 4-pancreatic cancer tumors. Tumor volume (a) and mouse weight (b,c) values during treatment are presented as mean +/- SEM. Two-way ANOVA with Sidak's multiple comparisons test was applied to compare 14F and14G.14F =Ab>PU (6
h) Abraxane^{®} 30 mg/kg iv 1x wk PU-H71 75 mg/kg ip 1x wk, 6 h after Ab ; 14G =Ab>PU (6
h) Abraxane^{®} 30 mg/kg iv 1x wk PU-H71 75 mg/kg ip 2x wk, 6 h after Ab.

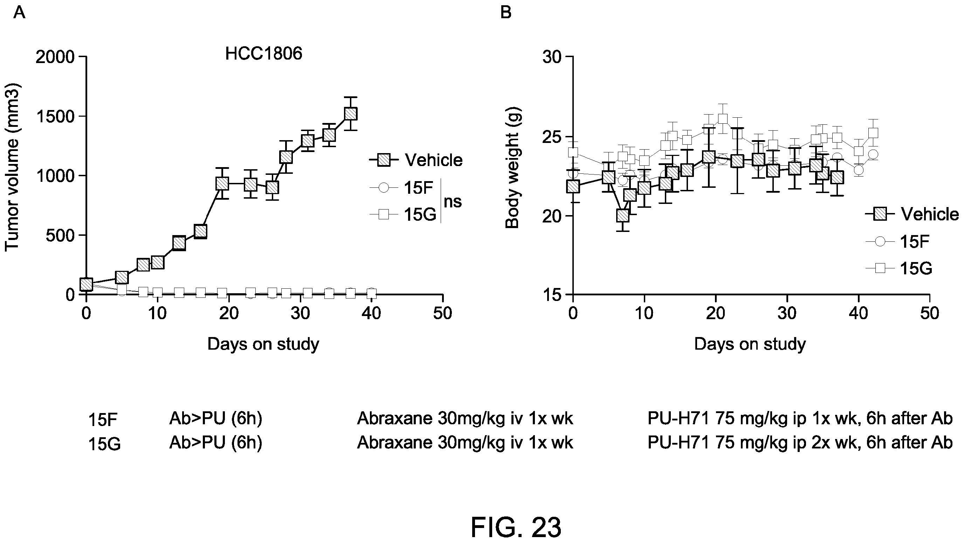

**FIGURES 23a** and **23b** further demonstrate the efficacy and safety of PU-H71 (75 mg/kg) and Abraxane^{®} (30mg/kg) when administered under the indicated treatment paradigm to mice (n=5) bearing xenografted HCC1806 triple negative breast cancer tumors. Tumor volume (a) and mouse weight (b) values during treatment are presented as mean +/- SEM. Two-way ANOVA with Sidak's multiple comparisons test was applied to compare 15F and 15G. 15F= Ab>PU (6 h) Abraxane^{®} 30 mg/kg iv 1x wk PU-H71 75 mg/kg ip 1x wk, 6 h after Ab; 15G = Ab>PU (6 h) Abraxane^{®} 30 mg/kg iv 1x wk PU-H71 75 mg/kg ip 2x wk, 6 h after Ab

### 4. DETAILED DESCRIPTION OF CERTAIN EMBODIMENTS

The present disclosure provides compounds for use in methods for treating cancer by administering to a cancer patient a therapeutically effective amount of an inhibitor of an HSP90 following pretreatment with a proteotoxic stressor.

As used in this application, the term "treatment" refers to delaying the onset of symptoms, reducing the severity or delaying the symptomatic progression of cancer. A cure of the disease is not required to fall within the scope of treatment. Further, it will be appreciated that the specific results of these treatment goals will vary from individual to individual, and that some individuals may obtain greater or lesser benefits than the statistical average for a representative population. Thus, treatment refers to administration of composition to an individual in need, with the expectation that they will obtain a therapeutic benefit.

The term "administering" refers to the act of introducing into the individual the therapeutic compound. In general, any route of administration can be used. Thus, administration by oral, intrathecal, intravenous, intramuscular or parenteral injection is appropriate depending on the nature of the condition to be treated. Administration may also be done to the brain by inhalation because there is a compartment at the upper side of the nose that connects with the brain without having the BBB capillaries. Compounds that cross the blood brain barrier are preferred for this mode of administration, although this characteristic is not strictly required.

The term "therapeutically effective amount" encompasses both the amount of the compound administered and the schedule of administration that on a statistical basis obtains the result of preventing, reducing the severity or delaying the progression of the disease in the individual. As will be appreciated, preferred amounts will vary from compound to compound in order to balance toxicity/tolerance with therapeutic efficacy and the mode of administration. Determination of maximum tolerated dose and of the treatment regime in terms of number and frequency of dosing is a routine part of early clinical evaluation of a compound.

As described in Examples 1-5, we have identified and characterized the epichaperome, a modified chaperome that occurs in over half of tumors, irrespective of tissue of origin or causal genetic mutation. We demonstrate that biochemical modifications differentiate the epichaperome from the housekeeping chaperome characteristic of normal cells. While transient chaperome complexes found in normal cells form and break down rapidly, their relatively brief temporal existence reflects a kinetic profile matching their role to fold and stabilize proteins at rates that meet the growth demands of non-transformed cells. Permanent chaperome complexes (*i.e.,* the epichaperome) in transformed cells, on the other hand, have a relatively long half-life, making them better suited for the role of the cancer chaperome that maintains signaling and transcriptional complexes in an active configuration to accommodate continuous growth and metabolism. As described in Examples 1-3, the epichaperome is enriched in chaperone and co-chaperone proteins including HSP90, HSP70, HSC70, HOP, AHA-1, CDC37, HSP40 and HSP110. Moreover, as shown in Example 3, the stable multimeric epichaperome complexes nucleate on HSP90, and physically and functionally bring together the components of the HSP70 and HSP90 machineries.

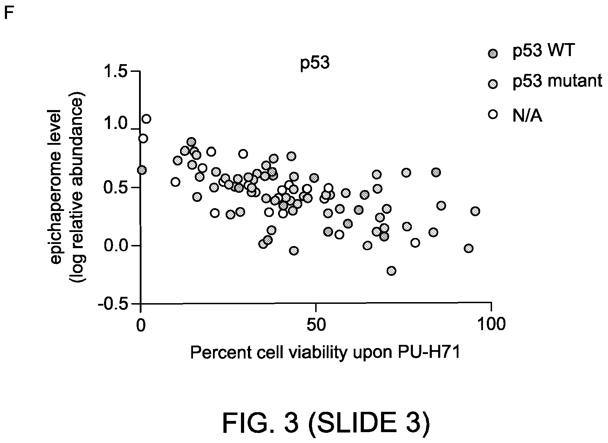

Additionally, we show in Examples 6 and 7 that HSP90 inhibitors significantly increase apoptosis in epichaperome-high cells, signifying their dependence on this cellular machinery. In other words, we show that there is a significant correlation between the abundance of the epichaperome and the susceptibility of these cancer cells to HSP90 inhibition. As discussed in Examples 6 and 7, lowering the abundance of the epichaperome resulted in cells less amenable to killing by an HSP90 inhibitor. From these experiments, we found that cells enriched in the epichaperome were more likely to die when exposed to an inhibitor of HSP90 in comparison to cells with lower levels of the epichaperome. In over 90 cancer cells lines encompassing breast cancer, lung cancer, pancreatic and gastric cancers, and leukemia and lymphomas, we found a significant correlation (P < 0.0001) between the abundance of the epichaperome and the susceptibility of these cancer cells to HSP90 inhibition. **(****FIGS. 3d** and **3f****).**

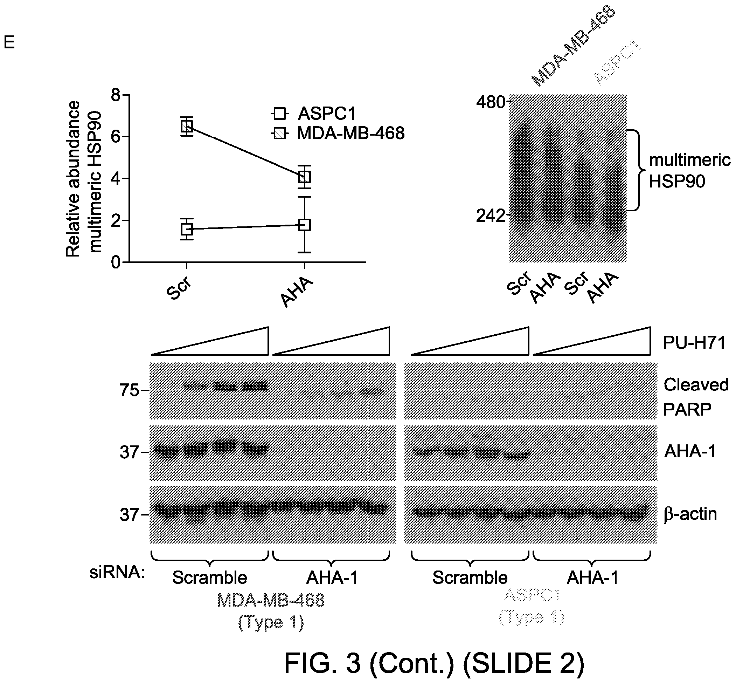

Moreover, lowering the abundance of the epichaperome by reducing the levels of AHA-1 protein, and thus reducing the stability of the epichaperome protein, resulted in cells less amenable to killing by HSP90 inhibition. **(****FIG. 3e****).**

These findings have led to the development of a new biomarker that is used to determine patients that would be amenable to treatment with an inhibitor of HSP90. The epichaperome presents a biochemical signature that is used as such a biomarker. Due to its occurrence in several tumor types irrespective of genetic or tissue attributes, this biomarker provides a means of patient stratification for patients that will respond to HSP90 therapy, or alternatively, therapy that relies on inhibition of another chaperone or co-chaperone protein.

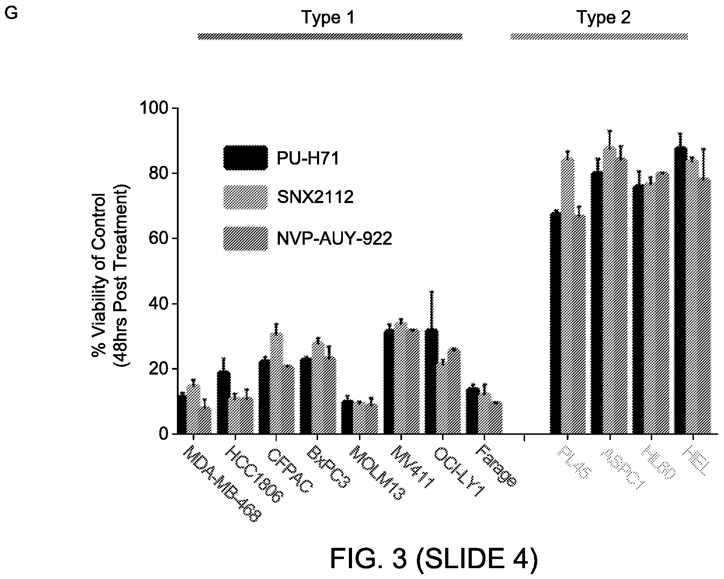

Our proteome and genetic studies have not uncovered the existence of a lesion specifically linked to the observed enrichment of the epichaperome in particular cancer cells. While p53-, ras-, myc-, HER-, PI3K/AKT-, and JAK- cell cycle-related defects were found in tumors that were sensitive to PU-H71, they were also evident in PU-H71 resistant cells **(****FIG. 3f****).** This suggests that proteome alterations that lead to chaperome reconfiguration and cellular dependence on the epichaperome characteristic of epichaperome-dependant tumors may have a varied genetic provenience or may not be dependent on a genetic background. The effect was not confined to PU-H71 treatment, since chemically distinct HSP90-directed agents of various selectivity for HSP90 complexes (SNX2112) and (NVP-AUY-922) recapitulated the profile of PU-H71. *See* Figure 16 for structures of these HSP90 inhibitors. Type 1 tumors were also effectively killed by these agents, whereas type 2 tumors remained refractory **(****FIG. 3g****).** Together, these observations confirm the epichaperome as a means of facilitating survival in these tumors.

These observed effects are in contrast to some reports in the literature, such as in vitro studies with cancer cell lines with various combinations of chemotherapeutics (e.g. Taxol^{®} or doxorubicin) with the Hsp90 inhibitor 17-AAG (see, Münster et al.Clin. Cancer Res. Vol. 7, 2228-2236, Aug.2001). As discussed above, this reference reports that sensitization of cells to apoptosis by a combination of Taxol^{®} and 17-AAG was most pronounced when the compounds were given at the same time or 17-AAG was given immediately after Taxol^{®}. Furthermore, the effects observed by Münster *et al.* with regard to the combination of Taxol^{®} and 17-AAG were Rb-dependent. Additionally, in contrast to results discussed below, Münster *et al.* report that sensitization of cells to the combination of doxorubincin and 17-AAG was not dependent on order or timing of addition of the compounds.

We next validated the functional significance of the epichaperome in primary tumors using *ex vivo* studies (*see* Example 6 for primary breast tumors and for acute myeloid leukemias). When primary breast tumors (n=4) were treated *ex vivo* with PU-H71 (treatment schematic shown in **FIG. 4****),** we found that those expressing the stable multimeric epichaperome forms to be most effectively killed by PU-H71. For instance, a study on four patients (patient 1066, patient 1067, patient 1068, patient 1069) with breast tumors was conducted **(****FIG. 5a****, left).** The top left shows the biochemical signature of four breast tumors as measured by isoelectric focusing. The middle, left shows the sensitivity of these samples by HSP90 inhibition. The bottom left shows the receptor status of the four analyzed breast cancer tumors. Patient 1066 in the study had a tumor that was enriched in the stable multimeric epichaperome forms as determined by charge-based native gel (isoelectric focusing) (*see* **FIG. 5a****).** The cancer cells of Patient 1066 readily underwent apoptosis following introduction of PU-H71. As shown in **FIG. 5a****,** the degree of apoptosis relative to the control was dependant on the concentration of PU-H71. In contrast, and similar to our findings in cultured cells, tumors not expressing the stable multimeric epichaperome forms remained mostly unaffected. For instance, patients 1067, 1068 and 1069 did not have tumors enriched in the stable epichaperome complexes and these tumors were far less sensitive to PU-H71 inhibition than the tumor of Patient 1066 **(****FIG. 5a****).** Additionally, adjacent benign tissue in the case of the breast specimens contained little to none of the epichaperome, and were accordingly insensitive to PU-H71.

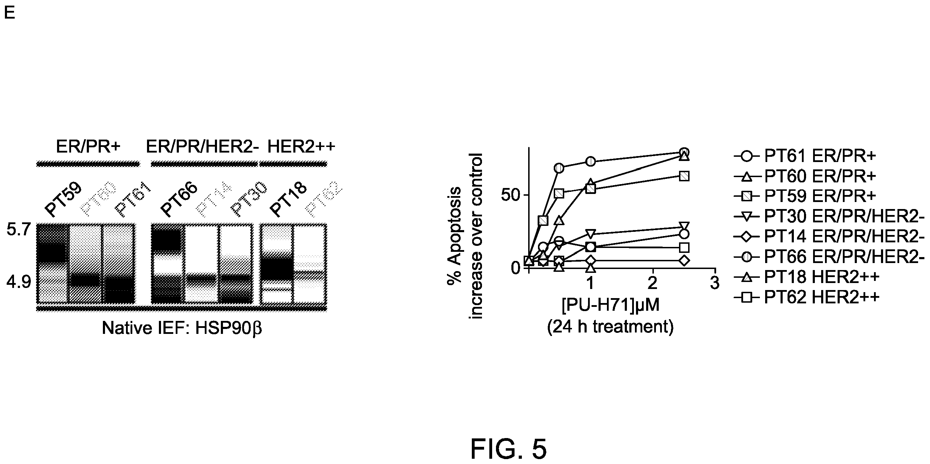

Studies on additional patient derived breast tumor samples furthered the observations described above **(****Fig. 5e****).** The left panel of Fig. 5e shows analysis of the biochemical signature of 8 patient breast tumor samples (PT59, PT60, PT61, PT66, PT14, PT30, PT18, and PT62) by isoelectric focusing. Samples from PT59, PT66, and PT18 demonstrated enrichment in the stable multimeric epichaperome forms as determined by charge-based native gel (isoelectric focusing). Notably, the cancer cells of these patients readily underwent apoptosis following introduction of PU-H71 (Fig. 5e, right panel). Also, confirming previous results, tumors not expressing the stable multimeric epichaperome forms remained mostly unaffected. For instance, samples from PT60, PT61, PT14, PT30, and PT62 did not have tumors enriched in the stable epichaperome complexes and these tumors were far less sensitive to PU-H71 treatment (Fig. 5e, right panel).

In a another experiment, the biochemical signature of three primary triple negative breast tumors (TNBC#1, TNBC#2 and TNBC#3) were analyzed by size-based native gel and the abundance of the epichaperome containing HSP90, HSC70, AHA1 and CD36 were measured **(****FIG. 5a****, right panels).** Based on the biochemical signatures, multimeric complexes were observed to a significant extent in only one of three tumors (TNBC#1) and to a moderate extent in another (TNBC#3). Notably, TNBC#2 showed very minimal to no formation of multimeric complexes indicative of epichaperome formation. As shown on the bottom right panel of **FIG. 5a****,** the tumor that was most sensitive to HSP90 inhibition was the tumor that contained the most stable multimeric HSP90-centric complexes. Notably, TNBC#2, which displayed minimal epichaperome formation, was resistant to HSP90 therapy.

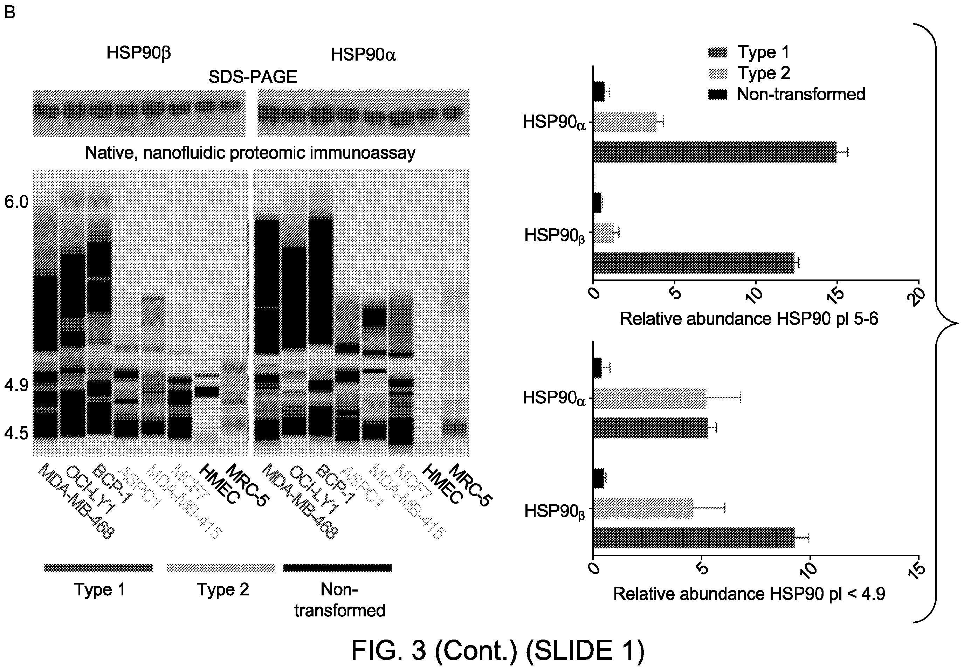

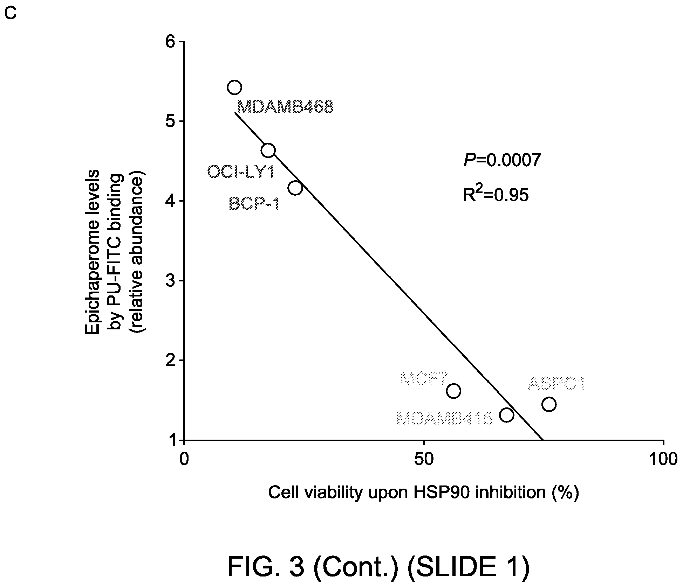

As shown in **Fig. 3a****,** PU-H71 binds with higher affinity and selectivity to HSP90 when in the epichaperome complex. For example, a solid support immobilized PU-H71 was incubated with cell homogenates to capture the most PU-H71 sensitive HSP90 complexes. The supernatant (*i.e.* leftover or least sensitive to PU-H71) was then analyzed by isolecetric focusing. **FIG. 3a** shows that HSP90 in the epichaperome complexes is most sensitive to PU-H71 (*see* **FIG. 3a****,** MDAMB468 cells where low amounts of PU-beads deplete the high molecular weight, stable HSP90 complexes but not the HSP90 dimer) whereas the housekeeping HSP90 remains unaltered (*see* HMEC cells, no change in the HSP90 signature by isolectric focusing). **FIG. 3b** explains how this property of PU-H71 can be used as an alternative method to measure the epichaperome levels. For example, **FIG. 3b** shows that in cells with high epichaperome (Type 1), a labeled PU-H71 (such as a fluorescently labeled PU-FITC) captures more HSP90 than in Type 2 cells (Fig 3c, y-axis) even though the TOTAL HSP90 among these cells is similar **(****FIG. 3b****,** SDS PAGE) **FIG. 3c** also shows that when one measures apoptosis induced in these cells by an HSP90 inhibitor (x-axis, Annexin v staining) cells with high epichaperome (as measured by isolectric focusing **FIG. 3b****,** and/or PU-FITC staining, **FIG. 3c**) are more prone to die when treated with an HSP90 inhibitor. **FIG.** 3d shows measurement of the epichaperome in a panel of primary acute myeloid leukemias using PU-FITC staining. It also shows the viability of these cells when treated with PU-H71 (apoptosis measurement by Annexin V staining). Finally, it correlates epichaperome levels with apoptosis to show that the more the epichaperome levels in these cells the more they are prone to die when cells are treated with an HSP90 inhibitor.

Based on the foregoing discoveries, we hypothesized that cancer cells can be induced to form the aforementioned stable, multi-chaperome conglomerates (epichaperome). One means of epichaperome induction is by the addition of an appropriate proteotoxic stress. Consequently, cancer cells that have little to no epichaperome can be induced to contain the epichaperome and thus to increase their sensitivity to epichaperome inhibitors, such as HSP90 inhibitors. Additionally, cancer cells that are already somewhat dependant on formation of the epichaperome can be induced to become heavily dependent on the epichaperome. We also hypothesized that the stability of the epichaperome complexes formed will be maximized at some point following administration or introduction of the proteotoxic stressor when the cancer cells are maximally dependent on epichaperome (e.g., inhibition of HSP90 and HSP70) to maintain survival, signalling and support continuous growth and metabolism. As such, following the time of maximum dependence of the cells on the chaperone proteins rewired to form the epichaperome, the stability of the epichaperome complex is gradually mitigated and the chaperone proteins assume their traditional roles of folding and stabilizing proteins.

Moreover, we hypothesized that an inhibitor that targets a particular protein comprising the epichaperome complex can be administered at a point in time following administration or introduction of the proteotoxic stressor when the cancer cells are in a state of substantial stress. The inhibitor can reduce or eliminate the function of the epichaperome in the cancer cells, thereby destroying the viability of the cells and rendering the cells vulnerable to apoptosis. The basic concept is depicted in **FIG.** 7. As shown in the top left of **FIG.** 7, cancer cells that are merely dependent on chaperone and co-chaperone proteins to perform normal "housekeeping" or other functions can be transformed into cells addicted to the epichaperome for survival. The addicted state is predicated on formation of the epichaperome complex. Moreover, as shown on the bottom right of **FIG. 7****,** cells in the addicted state are far more vulnerable to HSP90 inhibition than cells that are in the dependent state. Hence, increasing the amount of epichaperome in the cancer cells prior to HSP90 inhibition therapy by administration of a proteotoxic stressor has a profound effect on the efficacy of the HSP90 therapy. Moreover, the proteotoxic stressor has its own inherent activity that comprises the viability of the cancer cells. Accordingly, the combination of the proteotoxic stressor and the HSP90 inhibitor displays synergistic activity when administered in accordance with methods of the disclosure.

To study the influence of the proteotoxic stressor on cancer cells, we applied the biochemical techniques discussed in Section 5, Example 1. As shown in Example 8, various cancer cells were cultured with or without the presence of a chemotherapeutic agent as a proteotoxic stressor. Cells that were pre-treated with the chemotherapeutic agent and cells that were not pre-treated (vehicle) were subjected to Native PAGE to determine the nature of the chaperome complex. As shown in **FIGS. 7-9** and described in Example 8, several of the chemotherapeutic agents were capable of inducing the formation of stable multimeric chaperome complexes consistent with the formation of a more stable epichaperome. Not all of the chemotherapeutic agents induced formation of stable multimeric chaperome complexes. **FIG.10** displays a table of the particular chemotherapeutic reagents that were tested for their ability to induce the cancer tells towards formation of the epichaperome complex.

Alternative to adding a proteotoxic stressor, the stability of the epichaperome complex can be increased by pre-treating cancer cells with modulators of the post-translational modification of HSP90 and other chaperone and co-chaperone proteins. One means of influencing the post-translational modification of chaperone and co-chaperone proteins is by influencing phosphorylation. We demonstrate that phosphorylation inhibits the formation of the epichaperome complex. As shown in **FIG. 9A****,** reducing the amount of phosphate groups via the introduction of an appropriate phosphatase increases the amount of epichaperome formation. As shown in **FIG. 9B****,** reducing phosphorylation by pre-treating cells with particular kinase inhibitors also led to an increase in epichaperome formation, although the effect was not observed with all kinase inhibitors suggesting that specific epitopes are involved in regulating epichaperome formation.

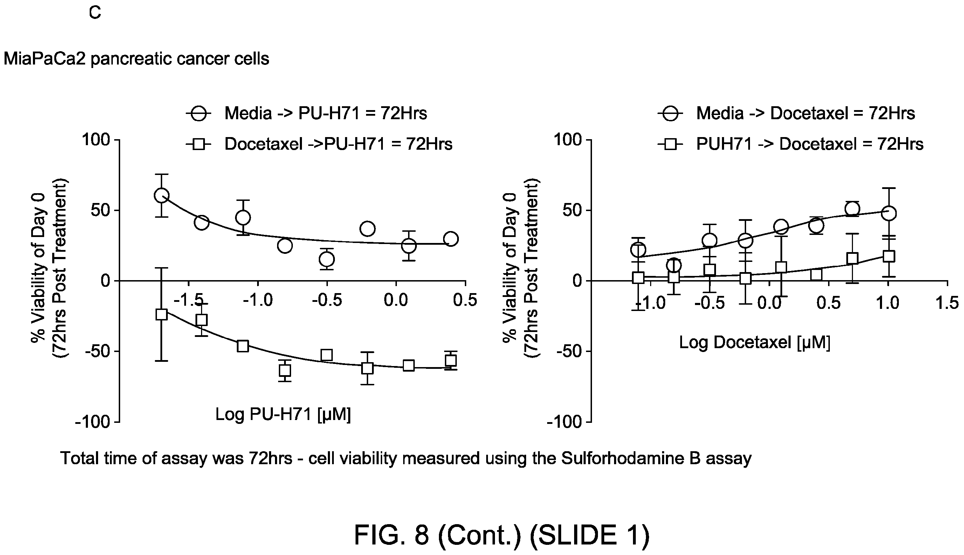

Having established that particular proteotoxic agents are capable of inducing the formation of a more stable epichaperome complex, we next developed an *in vitro* assay to measure the viability of MiaPaCa2 pancreatic cancer cells when treated with PU-H71, docetaxel, or combinations of PU-H71/docetaxel. (*see* Examples 9 and 14). With respect to the combination, two assays were performed, each at varying concentrations of the PU-H71 and the docetaxel. In one assay, the docetaxel was administered six hours prior to administration of the PU-H71. As shown in **FIG. 8c****,** pretreatment of the pancreatic cancer cells with docetaxel six hours prior to the administration of PU-H71 was significantly more potent than pretreatment of the cancer cells with PU-H71 prior to administration of the docetaxel. In fact, the later dosing regimen proved to be antagonistic.

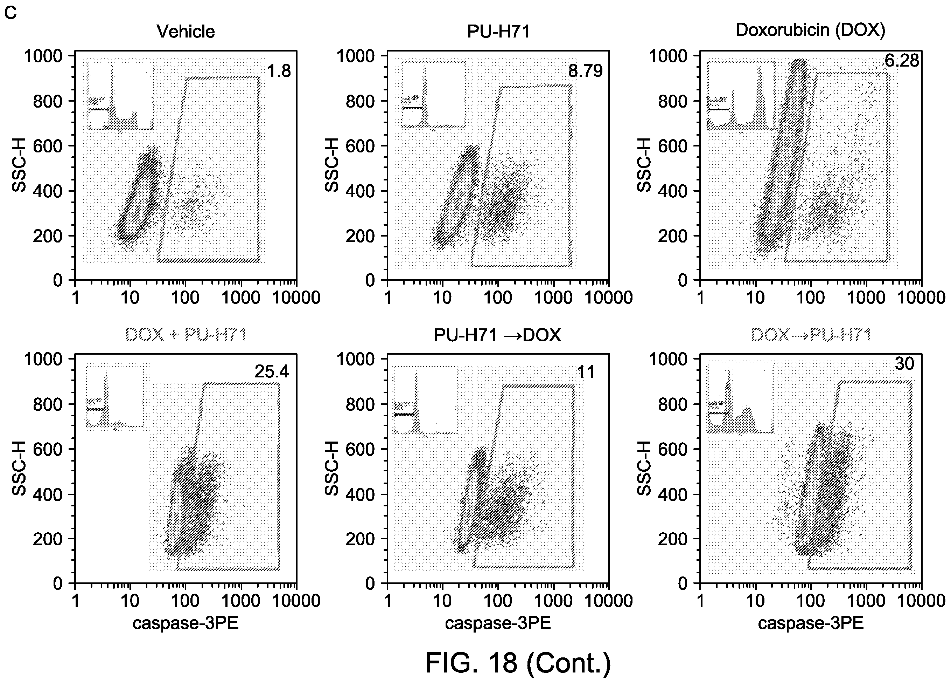

To further establish the potency of a combination of a proteotoxic agent and an Hsp90 inhibitor, *in vitro* assays were performed on lymphoma cells treated with Doxorubicin (DOX). DOX is a topoisomerase inhibitor that intercalates into DNA to induce DNA damage and oxidative stress. The interaction of DOX and PU-H71 was evaluated using standard criteria for synergy, the median effect/ Combination Index (CI) method. A dose-response matrix was used as a means to systematically test multiple ratios of DOX:PU-H71 for interaction in sequential or simultaneous schedules of exposure. Addition of PU-H71 to DLBCL and Burkitt lymphoma cells that had been preexposed to DOX (DOX→PU-H71) was synergistic at multiple drug ratios, whereas concurrent administration of drugs (DOX + PU-H71), or the exposure to PU-H71 prior to DOX (PU-H71→DOX) resulted in additive-to-antagonistic interaction (Fig. 18a, b and not shown). The effect of each drug and their combination on cell cycle and apoptosis in lymphoma cells with low to medium epichaperome levels, such as the SUDHL4 DLBCL cells was evaluated (Fig. 18c). In these cells, a 24 h-exposure to 1 µM PU-H71 resulted in G1/S arrest. A 24 h exposure to doses of DOX below the IC50 led to loss of S-phase fraction and accumulation in G2/M, with a small fraction of cells showing sub-G1 DNA content, indicative of apoptosis. When PU-H71 was added to DOX-exposed cells with a 24 h-delay, a loss of cells with G2/M DNA content and appearance of cells with fragmented sub-G1 DNA content was observed. In all, the combination of PU-H71 and DOX was most effective in inducing caspase-3 activation and apoptosis when cells were pre-exposed to DOX chemotherapy prior to PU-H71.

As described in Examples 10,13, and 14, *in vivo* animal studies were conducted to determine the efficacy of the disclosed combination dosing regimens. Animal models included an H1975 tumor model to assess lung cancer, a MiaPaca2 tumor model to assess pancreatic cancer, and HCC-1806 and MDA-MB-231 tumor models to assess triple negative breast cancer. In the models descried in Example 10, PU-H71 was used as an HSP90 inhibitor and Abraxane^{®} was used as a chemotherapeutic agent. The animals in the various studies were administered either a control vehicle, PU-H71, Abraxne or a combination of PU-H71 and Abraxane^{®} either concurrently or sequentially. For sequential administration, either Abraxane^{®} was administered first followed by administration of PU-H71 or PU-H71 was administered first followed by administration of Abraxane^{®}. Tumor volumes of the animals were evaluated at pre-selected points in time.

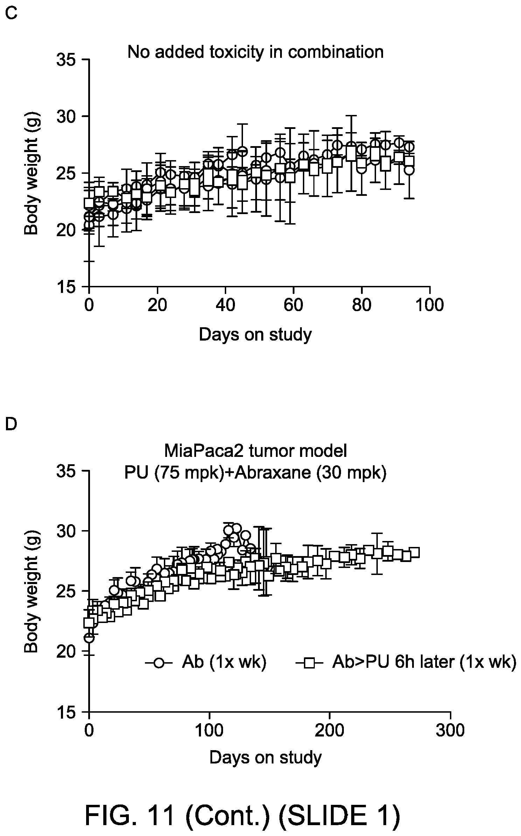

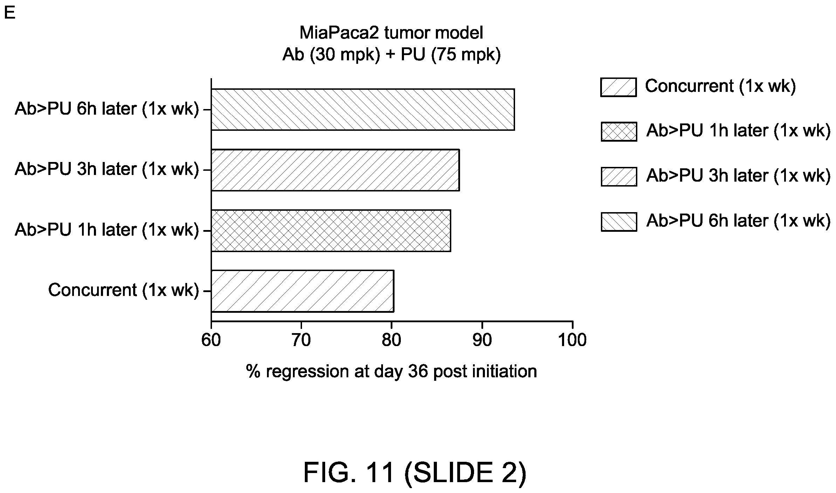

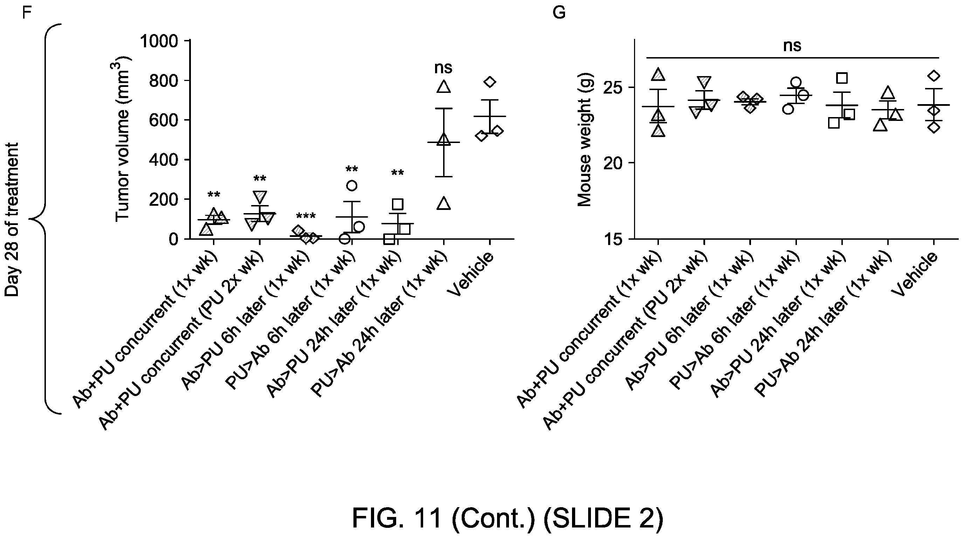

As shown in **FIGS. 11-14** **and 19-23,** in the *in vivo* animal models, administration of PU-H71 six hours following the administration of Abraxane^{®} showed significantly enhanced efficacy compared to concurrent administration of PU-H71 and Abraxane^{®} or PU-H71 followed by Abraxane^{®} six hours later. Moreover, as shown in **FIG. 13a** (H1975 tumor model), administration of Abraxane^{®} to mice six hours prior to administration of PU-H71 showed significantly enhanced efficacy relative to administration to mice twenty four hours prior to administration of PU-H71. This result indicates that the stability of the multimeric epichaperome complex is maximized at a particular time following administration of the stressor (e.g., chemotherapeutic agent) and gradually dissipates thereafter. For pre-administration with Abraxane^{®}, the proteotoxic stress on the cell begins to diminish at some point between six hours and twenty four hours.

The time dependency of epichaperome stability is also shown using the MiaPAca2 tumor model (*see* **FIG. 11e****).** In this case, PU-H71 was administered on a weekly basis either concurrently with Abraxane^{®} or administered at a time one hour, three hours or six hours following the administration of Abraxane^{®}. The percent regression of the tumor was measured thirty six days after initiation. In all cases where Abraxane^{®} was administered prior to PU-H71, the percent regression after thirty six days was greater than when PUH71 and Abraxane^{®} were administered concurrently. There was an increase in the percent regression as the timing interval between Abraxane^{®} and PU-H71 increased between one hour and six hours (*see* **FIG. 11e****).**

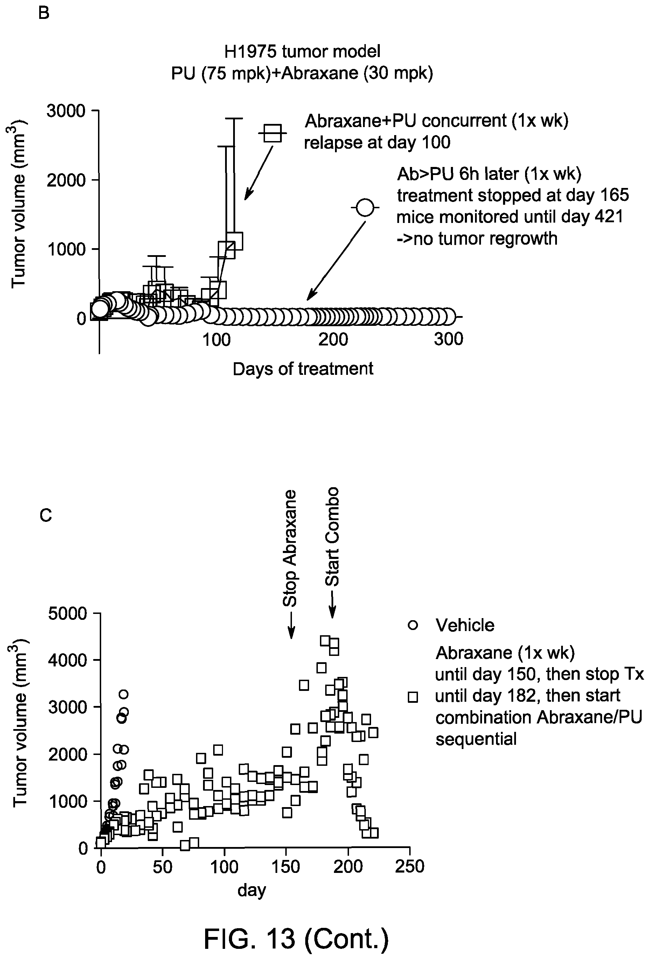

Prior studies co-administering HSP90 inhibitors and taxanes have achieved modest results, such as stasis or regression (see, for example, Proia et al, Clin Cancer Res; 20(2); 413-24). The present invention represents the first demonstration of a curative treatment regimen using HSP90 inhibitors and taxanes in the disclosed combinations. As shown in the Example 8 and 13 and **FIGS. 11-14** **and 19-23** ,superior results were obtained when mice bearing xenografts of H1975 lung cancer, MiaPaca2 pancreatic cancer, HCC-1806 triple negative breast cancer, or MDA-MB-231 triple negative breast cancer are treated first with Abraxane^{®} followed by administration of PU-H71 six hours afterwards. Importantly, concurrent administration of Abraxane^{®} and PU-H71 in the models generally results in relapse of the tumor after a certain time period. For instance, in the H1975 tumor model, the HCC-1806 tumor model and the MiaPaca2 tumor model, relapse is observed approximately 100 days following concurrent administration of Abraxan^{®}e and PU-H71 (*see* **FIG. 11b****).** However, in these same models, when Abraxane^{®} is administered six hours prior to PU-H71, relapse is generally not observed (*see* **FIG. 11b****).** For instance, in the H1975 tumor model, no tumor growth was observed in mice treated with Abraxane^{®} followed by PU-H71 administered six hours later. In this case, treatment of Abraxane^{®} and PU-H71 were stopped on day 165 following initial administration of the drugs and mice were monitored until day 421 following initial administration of the drugs and no tumor growth was observed. The result shows that using the disclosed dosing regimen can result in cures rather than simply achieving stasis. To demonstrate the point, **FIG.12** shows pictures of representative mice taken five weeks into the treatment regimen of PU-H71 and Abraxane^{®} when administered under the indicated treatment paradigms to mice bearing xenografted MiaPaCa2 pancreatic cancer tumors. Sequential treatment of PU-H71 6h after Abraxane^{®} administration resulted in a cure while concurrent treatment of PU-H71 and Abraxane^{®} resulted in stasis or regression. Similar results were obtained in the H1975 lung cancer model (*see* **FIG. 13b****)** and the HCC-1806 and MDA-MB-231 tumor models (*see* **FIGS. 14a** and **14b****).**

Accordingly, the disclosure provides compounds for use in methods of treating cancer using rational combination therapy of a proteotoxic stressor and an inhibitor of a chaperone or co- chaperone protein that is part of the epichaperome complex. Administration relies on appropriate timing of the proteotoxic stressor and the chaperone or co-chaperone inhibitor. Specific inhibitors of proteins forming the epichaperome complex include HSP90 inhibitors,
HSP70 inhibitors, AHA-1 inhibitors, CDC37 inhibitors, HOP inhibitors, HSP40 inhibitors and HSP110 inhibitors or combinations thereof. In the invention, the inhibitor of a chaperone or co-chaperone protein is an HSP90 inhibitor. In one embodiment, the inhibitor of a chaperone or co-chaperone protein is the HSP90 paralog referred to as glucose-regulated protein 94 (Grp94). In another embodiment, an inhibitor of a chaperone or co-chaperone protein is an HSP70 inhibitor. In another embodiment, an inhibitor of a chaperone or co-chaperone protein is an HSP70 inhibitor disclosed in WO2011/022440 or WO2015/l75707. In another embodiment, inhibitor of a chaperone or co-chaperone protein is a combination of an HSP90 inhibitor and an HSP70 inhibitor. In another embodiment, inhibitor of a chaperone or co- chaperone protein is an AHA-1 inhibitor. In another embodiment, inhibitor of a chaperone or co-chaperone protein is a CDC37 inhibitor. In another embodiment, inhibitor of a chaperone or co-chaperone protein is a HOP inhibitor.

In some embodiments, provided compounds for use in methods result in partial or complete remission of a cancer being treated. In some embodiments, remission is characterized in that the signs and symptoms of a cancer are reduced or not detectable. In some embodiments, provided compounds for use in methods cause all signs and symptoms of the cancer to disappear. In some embodiments, provided compounds for use in methods are characterized in that relapse of the cancer is not observed following treatment. In some embodiments, provided compounds for use in methods of treatment are characterized as providing substantially improved results beyond mere tumor statis or regression. In some embodiments, provided compounds for use in methods result in a cure of a cancer being treated. In some embodiments, provided compounds for use in methods result in no detectable tumor being present following treatment.

In one aspect, the disclosure provides compounds for use in methods for treating cancer by administering to a cancer patient an inhibitor of a chaperone or co-chaperone protein that is part of the epichaperome complex following pretreatment with a proteotoxic stressor. The proteotoxic stressor is administered at a sufficient time prior to administration of the chaperone or co- chaperone inhibitor to increase or maximize the formation of the epichaperome complex, thereby rendering the tumor more vulnerable to chaperone or co-chaperone inhibition therapy. It will be understood that different proteotoxic stressors have different pharmacokinetic and pharmacodynamic profiles that can impact their timing of administration. For instance, factors such as biological half-life, metabolism and tissue distribution will impact the onset and duration of action of the proteotoxic stressor and thereby influence the timing in forming stabilized epichaperome complexes. Other factors such as mode of administration (e.g., intravenous vs. oral), will also impact the formation of stable of epichaperome complexes. Likewise, the pharmacokinetic and pharmacodynamic profile of the chaperone or co-chaperone inhibitor can influence the timing and administration of such inhibitor.

In certain embodiments, the chaperone or co-chaperone protein inhibitor is administered at least one hour after administering an agent that induces a proteotoxic stress on the tumor cells. In other embodiments, the chaperone or co-chaperone inhibitor is administered at least two hours after administering an agent that induces a proteotoxic stress on the tumor cells. In other embodiments, the chaperone or co-chaperone inhibitor is administered at least three hours after administering an agent that induces a proteotoxic stress on the tumor cells. In other embodiments, the chaperone or co-chaperone inhibitor is administered at least three four hours after administering an agent that induces a proteotoxic stress on the tumor cells. In other embodiments, the chaperone or co-chaperone inhibitor is administered at least three five hours after administering an agent that induces a proteotoxic stress on the tumor cells. In other embodiments, the chaperone or co-chaperone inhibitor is administered at least six hours after administering an agent that induces a proteotoxic stress on the tumor cells. In other embodiments, the chaperone or co-chaperone inhibitor is administered at least seven hours after administering an agent that induces a proteotoxic stress on the tumor cells. In other embodiments, the chaperone or co-chaperone inhibitor is administered at least eight hours after administering an agent that induces a proteotoxic stress on the tumor cells. In other embodiments, the chaperone or co-chaperone inhibitor is administered at least nine hours after administering an agent that induces a proteotoxic stress on the tumor cells. In other embodiments, the chaperone or co-chaperone inhibitor is administered at least ten hours after administering an agent that induces a proteotoxic stress on the tumor cells. In other embodiments, the chaperone or co-chaperone inhibitor is administered at least twelve hours after administering an agent that induces a proteotoxic stress on the tumor cells. In other embodiments, the chaperone or co-chaperone inhibitor is administered at least eighteen hours after administering an agent that induces a proteotoxic stress on the tumor cells. In other embodiments, the chaperone or co-chaperone inhibitor is administered twenty four hours after administering an agent that induces a proteotoxic stress on the tumor cells. In certain embodiments, the proteotoxic stressor is administered at a time in the range between any of the foregoing embodiments, e.g., between about one and three hours prior to the administration of the chaperone or co-chaperone inhibitor, between about two and four hours prior to the administration of the chaperone or co-chaperone inhibitor, between about three and five hours prior to the administration of the chaperone or co-chaperone inhibitor, between about two and six hours prior the administration of the chaperone or co-chaperone inhibitor, between about three and six hours prior to the administration of the HSP90 inhibitor, between about four and six hours prior to the administration of the chaperone or co-chaperone inhibitor, between about four and eight hours prior to the administration of the chaperone or co-chaperone inhibitor, between about four and ten hours prior to the administration of the chaperone or co-chaperone inhibitor, between about five and seven hours prior to the administration of the chaperone or co-chaperone inhibitor and so on, and so forth.

In particular embodiments, the proteotoxic agent is administered parenterally (*e.g.,* intravenously). In such embodiments, the chaperone or co-chaperone inhibitor is administered at a time following completion of the parenteral administration. For instance, the chaperone or co-chaperone inhibitor can be administered one hour, two hours, three hours, four hours, five hours, six hours, seven hours, eight hours, nine hours, ten hours, eleven hours or 12 hours following completion of the parenteral administration of the proteotoxic agent. In certain embodiments, the chaperone or co-chaperone inhibitor is administered at a time in the range between any of the foregoing embodiments, *e.g.*, about one to three hours following completion of the parenteral administration of the proteotoxic agent, about two to four hours following completion of the parenteral administration of the proteotoxic agent, about three to five hours following completion of the parenteral administration of the proteotoxic agent, about two to six hours following completion of the parenteral administration of the proteotoxic agent, about three to six hours following completion of the parenteral administration of the proteotoxic agent, about four to six hours following completion of the parenteral administration of the proteotoxic agent, about four to eight hours following completion of the parenteral administration of the proteotoxic agent, about four to ten hours following completion of the parenteral administration of the proteotoxic agent, about five to seven hours following completion of the parenteral administration of the proteotoxic agent and so on, and so forth.

In the invention, the chaperone or co-chaperone inhibitor to be administered following administration of the proteotoxic stressor is an HSP90 inhibitor. In the invention, the HSP90 inhibitor is 8-(6-Iodo-benzo[1,3]dioxol-5-ylsulfanyl)-9-(3-isopropylamino-propyl)-9H-purin-6-ylamine (PU-H71), or a pharmaceutically acceptable salt thereof.

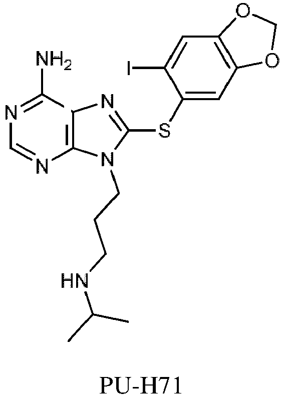

PU-H71 can be administered following administration of the proteotoxic stressor is 8-(6-Iodo-benzo[1,3]dioxol-5-ylsulfanyl)-9-(3-isopropylamino-propyl)-9H-purin-6-ylamine (PU-H71) or pharmaceutically acceptable salt thereof (*e.g.,* HCl salt). PU-H71 can be administered intravenously to a human patient at a dosage ranging from about 5 mg/m² to about 350 mg/m² according to a dosing schedule selected from once weekly, twice weekly, three times weekly, four times weekly or five times weekly. In accordance with the disclosure, the proteotoxic stressor is generally administered at a predetermined time prior to each administration of the PU-H71. In particular embodiments, PU-H71 is administered intravenously to a human patient at a dosage from about 20 mg/m² to about 60 mg/m² according to a dosing schedule selected from once weekly, twice weekly, three times weekly, four times weekly or five times weekly. In other embodiments, PU-H71 is administered intravenously to a human patient at a dosage from about 60 mg/m² to about 150 mg/m² according to a dosing schedule selected from once weekly, twice weekly, three times weekly, four times weekly or five times weekly. In other embodiments, PU-H71 is administered intravenously to a human patient at a dosage from about 200 mg/m² to about 350 mg/m² according to a dosing schedule selected from once weekly, twice weekly, three times weekly, four times weekly or five times weekly. In other embodiments, PU-H71 is administered intravenously to a human patient at a dosage from about 250 mg/m² to about 300 mg/m² according to a dosing schedule selected from once weekly, twice weekly, three times weekly, four times weekly or five times weekly. In other embodiments, PU-H71 is administered intravenously to a human patient at a dosage from about 250 mg/m² to about 300 mg/m² according to a twice weekly dosing schedule. In other embodiments, PU-H71 is administered intravenously to a human patient at a dosage from about 300 mg/m² to about 350 mg/m² according to a once weekly dosing schedule.

In the invention, the proteotoxic stressor to be administered prior to the chaperone or co-chaperone protein inhibitor is a chemotherapeutic agent. The chemotherapeutic reagents are microtubule stabilizing agents. The chemotherapeutic agent is provided at a dose that is capable of increasing the levels of epichamerome formed in the cells. In one embodiment, the chemotherapeutic agent may be given at a dosage that is typically administered to cancer patients. In another embodiment, the chemotherapeutic agent may be given at a dosage that less than the amount typically administered to cancer patients.

In the invention, the chemotherapeutic agent to be administered prior to the chaperone or co-chaperone inhibitor is a microtubule stabilizing agent. Particular microtubule stabilizing agents include but are not limited to docetaxel, paclitaxel, cabazitaxel, ixabepilone, vincristine, laulimalide, discodermolids and epothilones. In one embodiment the proteotoxic stressor is a protein-bound paclitaxel composition such as Abraxane^{®}.

In one embodiment of the invention, PU-H71 is administered following the administration of paclitaxel or docetaxel. In one particular embodiment, PU-H71 is administered at a particular time following administration of Taxol^{®} (paclitaxel in cremophor). In another embodiment, paclitaxel is administered in a liposomal formulation such as LEP-ETU (NeoPharm), EndoTAG^{®}-1 (Medigene) or; Lipusu^{®} (Luye Pharma Group). In one embodiment, the paclitaxel is administered as a nanodispersion. One such example of a nanodispersion-based paclitaxel injection is PICN (paclitaxel injection for nanodispersion) (Sun Pharma).

In one particular embodiment, PU-H71 is administered at a specified time following administration of paclitaxel. Paclitaxel can be formulated by various methods. For instance, paclitaxel can be formulated as a protein-bound paclitaxel composition such as Abraxane^{®} or a cremophor-based formulation such as Taxol^{®}. In such embodiments, the paclitaxel is generally administered intravenously. Each intravenous administration of the paclitaxel is over a pre-determined period of time that may be patient and dose dependent. For instance, the paclitaxel may be infused over a time period ranging from 1 hour to 96 hours. In particular embodiments, the paclitaxel is infused for 1, 3, or 24 hours. PU-H71 can then be administered at a specified time following completion of the intravenous dose of the paclitaxel. For instance, the PU-H71 can be administered at least two hours, at least three hours, at least four hours, at least five hours, at least six hours, at least seven hours, at least eight hours or at least twelve hours following completing the administration of the paclitaxel. In some embodiments, the PU-H71 can be administered at least eighteen or twenty four hours following completing the administration of the paclitaxel. In a particular embodiment, the PU-H71 is administered no more than twelve hours following administration of the paclitaxel. In a particular embodiment, the PU- H71 is administered no more than twenty four hours following administration of the paclitaxel. In another particular embodiment, PU-H71 is administered between five and seven hours following administration of the paclitaxel.

In embodiments where PU-H71 is administered following administration of Abraxane^{®}, the dosage and timing of administration of Abraxane^{®} can generally follow the schedule indicated on the prescribing information for Abraxane^{®}, as long as PU-H71 is administered at a specified time following intravenous administration of the Abraxane^{®}. For instance, for metastatic breast cancer, the recommended dosage of Abraxane^{®} is 260 mg/m² intravenously for 30 minutes every three weeks. For non-small lung cancer, the recommended dosage for Abraxane^{®} is 100 mg/m² intravenously over 30 minutes on days 1, 8 and '5 of each 21-day cycle. For adenocarcinoma of the pancreas, the recommended dosage of Abraxane^{®} is 125 mg/m² administered intravenously on days 1, 8, and 15 of a 28 day cycle. It will be understood, however, the dosage and timing of administration reflected in the prescribing information can be diverged from. For instance, the synergistic effect displayed when PU-H71 is administered after Abraxane^{®} may warrant a dose reduction of Abraxane^{®} relative to the dosages reflected in the prescribing information for Abraxane^{®}. As such, the dosage of Abraxane^{®} administered to cancer patients on a regimen of Abraxane^{®} and PU-H71 administered in accordance with methods of the disclosure can be 80% of the amount, 70% of the amount, 60% of the amount, 50% of the amount, 40% of the amount, 30% of the amount or 20% of the amount reflected in the prescribing information for Abraxane^{®}. In certain embodiments, the Abraxane^{®} can be administered in an amount between any of the foregoing embodiments, e.g., between 20% and 100% of the amount reflected in the prescribing information for Abraxane^{®}, between 40% and 100% of the amount reflected in the prescribing information for Abraxane^{®}, between 60% and 100% of the amount reflected in the prescribing information for Abraxane^{®}, between 20% and 80% of the amount reflected in the prescribing information for Abraxane^{®}, between 40% and 80% of the amount reflected in the prescribing information for Abraxane^{®}, between 50% and 70% of the amount reflected in the prescribing information for Abraxane^{®}, between 50% and 60% of the amount reflected in the prescribing information for Abraxane^{®}, and so on, and so forth.

In embodiments where the PU-H71 is administered following the administration of Taxo^{®}l, the Taxol^{®} and the PU-H71 can be administered using various dosing schedules, including but not limited to a single dose every 3 weeks, a single dose every 2 weeks or a single dose every 1 week. The 3 week and 2 week doing schedule generally relies on a dosage of Taxo^{®}l ranging from 135-250 mg/m² over a 3 hour, 24 hour or 96 hour infusion. The 1 week dosing schedule generally relies on a 1 hour infusion of Taxol^{®} ranging from 40 -100 mg/m². For instance, Taxol^{®} may be administered weekly at a dosage of 40 mg/m2, 50 mg/m², 60 mg/m², 70 mg/m², 80 mg/m², 90 mg/m² or100 mg/m².

The proteotoxic stressor and the chaperone or co-chaperone protein inhibitor can be administered at a regular interval, referred to as a treatment cycle. The treatment cycle is defined as the number of days in which the dosing schedule begins to repeat itself. The proteotoxic stressor and the chaperone or co-chaperone protein inhibitor are generally administered on day 1 of the treatment cycle and may be administered at other days of the treatment cycle. For instance, the proteotoxic stressor (or modulator of the post- translational modification of the chaperone or co-chaperone protein) and the chaperone or co- chaperone protein inhibitor can be administered once, twice, three times, four times, five times, six times, seven times, eight times, nine times or ten times over a 7 day, 10 day, 14 day, 21 day, 28 day or 30 day dosing schedule. If the drugs are prescribed to be administered once a week, the treatment cycle is defined as a 7 day period with the proteotoxic stressor and the chaperone or co-chaperone protein being administered on day 1 of the cycle and no proteotoxic stressor or chaperone or co-chaperone protein inhibitor being administered on days 2-6 of the treatment cycle. If the drugs are prescribed to be administered once every three weeks, the treatment cycle is defined as a 21 day period with the proteotoxic stressor and the chaperone or co-chaperone protein inhibitor being administered on day 1 of the cycle and no proteotoxic stressor or chaperone or co- chaperone protein inhibitor being administered on days 2-21 of the treatment cycle. If the proteotoxic stressor and the chaperone or co-chaperone protein are prescribed to be administered at days 1, 8 and 15 of a 28 day dosing schedule, then no proteotoxic stressor or chaperone or co-chaperone protein is to be administered between days 16 and 28 of each cycle.

In one aspect, the disclosure provides compounds for use in methods of treating cancer by administering a combination of a proteotoxic stressor and a chaperone or co-chaperone protein inhibitor over a treatment cycle of between 7 days and 31 days, wherein the proteotoxic stressor and the chaperone or co-chaperone protein inhibitor are administered at least once over said cycle, and wherein each administration of said proteotoxic stressor is followed by administration of said chaperone or co-chaperone protein inhibitor. In accordance with the disclosure, administration of the chaperone or co-chaperone protein commences following an increase in epichaperome formation induced by the proteotoxic stressor. In some embodiments, the chaperone or co-chaperone protein inhibitor is administered at least one hour after administering an agent that induces a proteotoxic stress on the tumor cells. In other embodiments, the chaperone or co-chaperone protein inhibitor is administered at least two hours after administering an agent that induces a proteotoxic stress on the tumor cells. In other embodiments, the chaperone or co-chaperone protein inhibitor is administered at least three hours after administering an agent that induces a proteotoxic stress on the tumor cells. In other embodiments, the chaperone or co-chaperone protein inhibitor is administered at least three four hours after administering an agent that induces a proteotoxic stress on the tumor cells. In other embodiments, the chaperone or co-chaperone protein inhibitor is administered at least five hours after administering an agent that induces a proteotoxic stress on the tumor cells. In other embodiments, the chaperone or co-chaperone protein inhibitor is administered at least six hours after administering an agent that induces a proteotoxic stress on the tumor cells. In other embodiments, the chaperone or co-chaperone protein inhibitor is administered at least seven hours after administering an agent that induces a proteotoxic stress on the tumor cells. In other embodiments, the chaperone or co-chaperone protein inhibitor is administered at least eight hours after administering an agent that induces a proteotoxic stress on the tumor cells. In other embodiments, the chaperone or co-chaperone protein inhibitor is administered at least nine hours after administering an agent that induces a proteotoxic stress on the tumor cells. In other embodiments, the chaperone or co-chaperone protein inhibitor is administered at least ten hours after administering an agent that induces a proteotoxic stress on the tumor cells. In other embodiments, the chaperone or co-chaperone protein inhibitor is administered at least twelve hours after administering an agent that induces a proteotoxic stress on the tumor cells. In other embodiments, the chaperone or co-chaperone protein inhibitor is administered twenty four hours after administering an agent that induces a proteotoxic stress on the tumor cells. In certain embodiments, the proteotoxic stressor is administered at a time in the range between any of the foregoing embodiments, e.g., between about one and three hours prior to the administration of the chaperone or co-chaperone protein inhibitor, between about two and four hours prior to the administration of the the chaperone or co-chaperone protein inhibitor, between about three and five hours prior to the administration of the chaperone or co-chaperone protein, between about two and six hours prior the administration of the chaperone or co-chaperone protein, between about three and six hours prior to the administration of the chaperone or co-chaperone protein, between about four and six hours prior to the administration of the chaperone or co-chaperone protein, between about four and eight hours prior to the administration of the chaperone or co- chaperone protein inhibitor, between about four and ten hours prior to the administration of the chaperone or co-chaperone protein inhibitor, between about five and seven hours prior to the administration of the chaperone or co-chaperone protein inhibitor and so on, and so forth.

In some embodiments, the treatment cycle may be a 21 day cycle, with the proteotoxic stressor and the chaperone or co-chaperone protein inhibitor administered only on day 1 of the cycle. In some embodiments, the treatment cycle may be a 21 day cycle, with the proteotoxic stressor and the chaperone or co-chaperone protein inhibitor to be administered twice, three times, four times, five times, six times, seven times or eight times during the cycle. In some embodiments, the treatment cycle may be a 14 day cycle, with the proteotoxic stressor and the chaperone or co-chaperone protein inhibitor administered once, twice, three times, four times five times, or six times over the cycle. In some embodiments, the treatment cycle may be a 28 day cycle, with the proteotoxic stressor and the chaperone or co-chaperone protein inhibitor to be administered twice, three times, four times, five times, six times, seven times, eight times, nine times or ten times during the cycle. In some such embodiments, the proteotoxic stressor and the chaperone or co- chaperone protein inhibitor may be administered on days 1, 8 and 15 of the 28 treatment cycle. In other embodiments, the treatment cycle may be a 7 day cycle, with the proteotoxic stressor and the chaperone or co-chaperone protein inhibitor to be administered on day 1 of the cycle.

In certain embodiments, the chaperone or co-chaperone protein inhibitor is administered only on days when the proteotoxic stressor is administered. In other embodiments, the chaperone or co-chaperone protein inhibitor is administered on days when the proteotoxic stressor is administered and on days when the proteotoxic stressor is not administered. For instance, in a two week cycle, the chaperone or co-chaperone protein inhibitor and the proteotoxic stressor may be administered on days 1 and 8 of the cycle and the chaperone or co-chaperone protein inhibitor can be administered by itself on days 4 and 11 of the cycle.

Methods of administration of the proteotoxic stressor and the chaperone protein inhibitor or co-chaperone protein inhibitor include, but are not limited to, intradermal, intramuscular, intraperitoneal, parenteral, intravenous, subcutaneous, intranasal, epidural, oral, sublingual, intracerebral, intravaginal, transdermal, rectal, by inhalation, or topical, particularly to the ears, nose, eyes, or skin.

The proteotoxic stessor and the chaperone protein inhibitor or co-chaperone protein inhibitor can each be administered as pharmaceutically acceptable compositions. The compositions can optionally comprise a suitable amount of a pharmaceutically acceptable excipient so as to provide the form for proper administration to the patient undergoing therapy. Such a pharmaceutical excipient can be a diluent, suspending agent, solubilizer, binder, disintegrant, preservative, coloring agent, lubricant, and the like. The pharmaceutical excipient can be a liquid, such as water or an oil, including those of petroleum, animal, vegetable, or synthetic origin, such as peanut oil, soybean oil, mineral oil, sesame oil, and the like. The pharmaceutical excipient can be saline, gum acacia, gelatin, starch paste, talc, keratin, colloidal silica, urea, and the like. In addition, auxiliary, stabilizing, thickening, lubricating, and coloring agents can be used. In one embodiment, the pharmaceutically acceptable excipient is sterile when administered to an animal.

Saline solutions and aqueous dextrose and glycerol solutions can also be employed as liquid excipients, particularly for injectable solutions. Suitable pharmaceutical excipients also include starch, glucose, lactose, sucrose, gelatin, malt, rice, flour, chalk, silica gel, sodium stearate, glycerol monostearate, talc, sodium chloride, dried skim milk, glycerol, propylene glycol, water, EtOH, and the like. The compositions, if desired, can also contain minor amounts of wetting or emulsifying agents, or pH buffering agents. Specific examples of pharmaceutically acceptable carriers and excipients that can be used to formulate oral dosage forms are described in the Handbook of Pharmaceutical Excipients, (Amer. Pharmaceutical Ass'n, Washington, DC, 1986).

The compositions can take the form of solutions, suspensions, emulsions, tablets, pills, pellets, capsules, capsules containing liquids, powders, sustained-release formulations, suppositories, emulsions, aerosols, sprays, suspensions, or any other form suitable for use.

When a compound is to be injected parenterally, it can be, *e.g.,* in the form of an isotonic sterile solution. Alternatively, when a compound of the disclosure is to be inhaled, it can be formulated into a dry aerosol or can be formulated into an aqueous or partially aqueous solution.

In one embodiment, the proteotoxic stressor and the chaperone protein inhibitor or co-chaperone protein inhibitor can be individually formulated for intravenous administration. In certain embodiments, compositions for intravenous administration comprise sterile isotonic aqueous buffer. Where necessary, the compositions can also include a solubilizing agent. Generally, the ingredients are supplied either separately or mixed together in unit dosage form, for example, as a dry lyophilized powder or water free concentrate in a hermetically sealed container such as an ampule or sachette indicating the quantity of active agent. When a compound is to be administered by infusion, it can be dispensed, for example, with an infusion bottle containing sterile pharmaceutical grade water or saline. When a compound is administered by injection, an ampule of sterile water for injection or saline can be provided so that the ingredients can be mixed prior to administration.

### 5. EXAMPLES

### 5.1 Example 1: Identifying Chaperome Complexes

### 5.1.1 Materials and Methods

**Cell Lines.** Cell lines were obtained from laboratories at WCMC or MSKCC, and were originally purchased from the American Type Culture Collection (ATCC) or DSMZ. Cells were cultured as per the providers' recommended culture conditions. Cells were authenticated using short tandem repeat profiling and tested for mycoplasma.

**Primary breast cancer specimens.** Patient tissue procurement was authorized through institutionally review board-approved bio-specimen protocol#09-121 at Memorial Sloan Kettering Cancer Centre (New York, NY). Specimens were treated for 24 h with the indicated concentrations of PU-H71. Following treatment, slices were fixed in 4% formalin solution for 1 h then stored in 70% ethanol. For tissue analysis, slices were embedded in paraffin, sectioned, slide-mounted, and stained with haematoxylin and eosin. Tissue slides were assessed blindly by a breast cancer pathologist who gauged the apoptosis present in the tumor as well as any effect to normal tissue.

**Protein analysis by the NanoPro capillary-based immunoassay platform.** Cultured cells were lysed in 20 mM HEPES pH 7.5, 50 mM KCl, 5 mM MgCl₂, 0.01% NP40, 20 mM Na₂MoO₄ buffer, containing protease and phosphatase inhibitors. Total protein assay was performed on an automated system, NanoPro**^{™}** 1000 Simple Western (ProteinSimple^{®}), for charge-based separation. Briefly, total cell lysates were diluted to a final protein concentration of 250 ng/µl using a master mix containing 1x Premix G2 pH 3-10 separation gradient (Protein simple^{®}) and 1x isoelectric point standard ladders (ProteinSimple^{®}). Samples diluted in this manner maintained their native charge state, and were loaded into capillaries (ProteinSimple^{®}) and separated based on their isoelectric points at a constant power of 21,000 **µ**Watts for 40 min. Immobilization was performed by UV-light embedded in the Simple Western system, followed by incubations with anti-HSP90**β** (SMC-107A, StressMarq Biosciences) or with anti-HSP90**α** (ab2928, Abcam), and subsequently with HRP-conjugated anti-Mouse IgG (1030-05, SouthernBiotech) or with HRP-conjugated anti-Rabbit IgG (4010-05, SouthernBiotech). Protein signals were quantitated by chemiluminescence using SuperSignal^{®} West Dura Extended Duration Substrate (Thermo Scientific), and digital imaging and associated software (Compass) in the Simple Western system.

The experimental data were collected and analyzed by the software Compass as provided by the system. Distinct HSP90 complexes were detected in MDA-MB-468 (sensitive to Hsp90 inhibitor; high epichaperome levels), Aspc1 (resistant to Hsp90 inhibitor; low to no epichaperome) and HMEC (non-transformed) cells.

Several nanopro conditions have been tested. A mixture of 3-10+5-8 ampholytes had a similar profile to the standard conditions, but resulted in a broader peak separation then desired.

20 mM Bicine pH 7.6, 0.6% CHAPS buffer (buffer 1), recommended by the manufacturer, and Tris buffered saline (buffer 2) have been tested as a protein extraction buffer. The picks above p1 5 (standard p1 for Hsp90) were significantly decreased in MDA-MB-468 cells lysed in buffer 1 and 2, suggesting that these conditions are not suitable for efficient extraction of these protein species.

Several Hsp90-specific antibodies have been tested including ab2927 from Abcam, resulting in a comparable to H90-10 profile. Enzo SPA-830 and SPA-845 antibodies provided no detectable signal.

**Western blotting.** Protein was extracted from cultured cells in 20 mM Tris pH 7.4, 150 mM NaCl, 1 % NP-40 buffer with protease and phosphatase inhibitors added. Ten to fifty µg of total protein was subjected to SDS-PAGE, transferred onto nitrocellulose membrane, and incubated with indicated antibodies. HSP90β and HSP110 antibodies were purchased from Stressmarq; HSP70, HSC70, HIP, HOP, and HSP40 from Enzo; HSP90β, HSP90**α**, and AHA-1 from Abcam; cleaved PARP from Promega; CDC37 from Cell Signaling Technology; and **β**-actin from Sigma-Aldrich. The blots were washed with TBS/0.1% tween 20 and incubated with appropriate HRP-conjugated secondary antibodies. Chemiluminescent signal was detected with Enhanced Chemiluminescence Detection System (GE Healthcare) according to the manufacturer's instructions.

**Native gel electrophoresis.** A lysis buffer (20 mM Tris pH 7.4, 20 mM KCl, 5 mM MgC12, 0.01% NP40 and 10% glycerol) with a low amount of detergent has been chosen for extraction of native Hsp90 complexes in order to preserve them during the lysis procedure. Cells were lysed by a freeze-thaw procedure. 50-100 g of protein was loaded onto 4-10% native gradient gel and resolved at 4°C. The proteins were transferred onto nitrocellulose membrane in 0.1% SDS-containing transfer buffer for 1 hour and immuno-blotted with the anti-Hsp90 antibody. Several transferring conditions have been tested to determine the one allowing for the transfer and detection of the high molecular weight HSP90 complexes. We have incubated the gels in 25 mM Tris, 192 mM glycin, 0.1% SDS for 15 min at room temperature (condition #1) before the transfer in standard conditions; performed the transfer in 0.05% SDS-containing transfer buffer (condition #2) and performed the transfer in 0.1% SDS-containing transfer buffer (condition #3). Specificity of the signal was tested by the use of a positive control and of AHA1 knock-down samples.

Several Hsp90 specific antibodies have been tested for their ability to recognize the high molecular weight Hsp90 species. Among these were H90-10 clone (SMC-107A) from StressMarq, ab2928 from Abcam, 610418 from BD Transduction laboratories and ab2927 from Abcam.

### 5.1.2 Results

To overcome the limitations of protein separation for resolving complexes of similar composition and size, we took advantage of a capillary-based platform that combines isoelectric focusing with immunoblotting capabilities. Fan, A. C. et al. Nanofluidic proteomic assay for serial analysis of oncoprotein activation in clinical specimens Nat Med 15, 566-571 (2009). This methodology uses an immobilized pH gradient to separate native multimeric protein complexes based on their isoelectric point (pI), and then allows for probing of immobilized complexes with specific antibodies. Moreover, it does so using only minute amounts of sample, enabling the investigation of primary specimens.

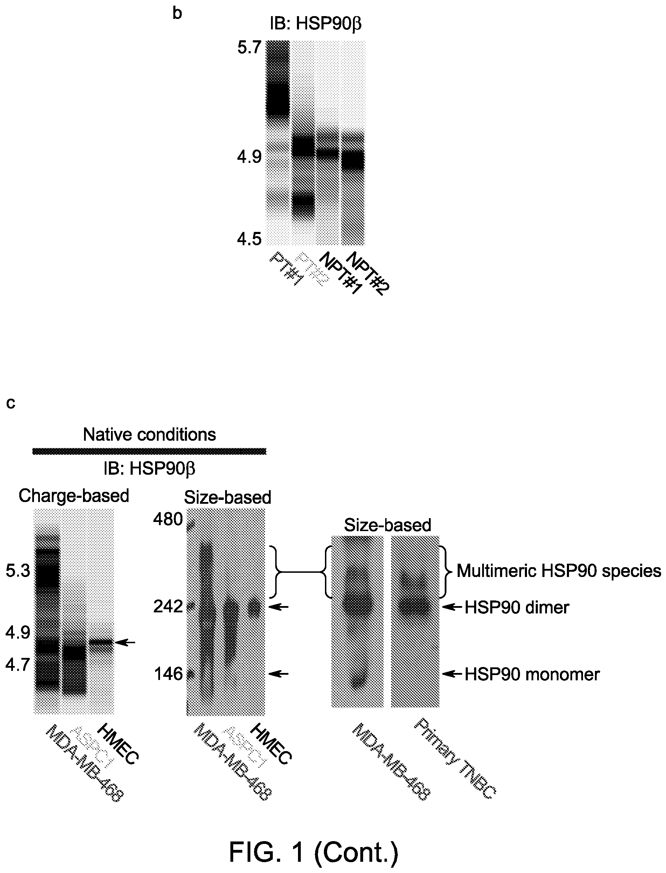

Using this method, we first analyzed HSP90, the most abundant chaperome member in human cells. In cultured non-transformed cells **(****FIG. 1a****)** and in primary normal breast tissue **(****FIG.1b****),** HSP90 focused primarily as a single species at the predicted pI of 4.9. Conversely, cancer cell lines analyzed by this method contained a complex mixture of HSP90 species spanning a pI range of 4.5 to 6. Both the HSP90**α** and HSP90**β** isoforms were part of these complexes. Furthermore, while all cancer cell lines contained a number of HSP90 complexes with pI values below 4.9, a subset of cell lines was enriched in HSP90 complexes with the unusual pI of 5 and above, from herein referred to as "type 1" cells (Fig. 1a). This distinction in HSP90 complexes of high (type 1) and low (type 2) pI was also evident in primary cancer specimens, as seen in two breast tumors **(****FIG 1b****).** Interestingly, the total levels of HSP90, as measured by SDS-PAGE, were essentially identical among all analyzed samples irrespective of whether they were type 1 or type 2 cells **(****FIG. 1a****, top).**

Two types of protein modifications, chemical and biochemical, can change the isoelectric point for a given protein. HSP90 is known to interact with several co-chaperones including HSP70 and HSC70 (which are inducible and constitutive cytosolic HSP70 family members, respectively), HSP70-HSP90 organizing protein (HOP) (also known as stress-inducible phosphoprotein 1 [STIP1]), activator of Hsp90 ATPase 1 (AHA-1 or AHSA-1), and cell division cycle 37 (CDC37). Each of these co-chaperones has a distinct role, with CDC37 facilitating activation of kinases by HSP90, AHA-1 augmenting its ATPase activity, and HSP70 and HOP participating with HSP90 in the chaperoning of a variety of proteins. Indeed, upon separating HSP90 complexes by size in a native PAGE, we observed that cells enriched in the high pI HSP90 species were also enriched in the high molecular weight HSP90-containing complexes **(****FIG. 1c****).** Under similar conditions, we detected one major species in non-transformed cells. This is presumably the HSP90 dimer in agreement with a previous study that found transient HSP90 oligomeric forms in normal tissue, which dissociated to smaller dimers and monomers under native electrophoretic conditions.

### 5.2 Example 2: Identifying Multimeric Forms of Chaperome Members

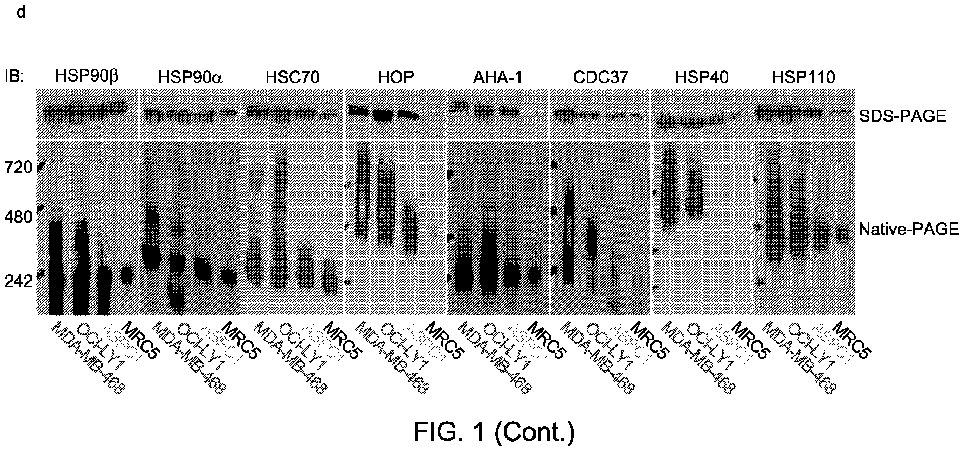

We next screened a panel of anti-chaperome antibodies for those that interacted with the target protein in its native form. We reasoned that these antibodies were more likely to capture stable multimeric forms of the chaperome members. Using these native-cognate antibodies, we observed that the more the cell content of high molecular weight HSP90 complexes, the more it was enriched in multimeric forms of other essential chaperome members, such as HSC70, HOP, AHA-1, CDC37, HSP40 and HSP110 **(FIG. Id,** bottom). While the quaternary state of the chaperome varied, the overall levels of each chaperome member remained relatively constant **(****FIG. 1d****,** top).

### 5.3 Example 3: HSP90 and HSC70 nucleate multi-chaperome complexes

### 5.3.1 Materials and Methods

**siRNA knock-down.** Cells were plated at 1×10⁶ per 6 well-plate and transfected with an siRNA against human AHSA1 (Qiagen) or a negative control with Lipofectamine RNAiMAX reagent (Invitrogen), incubated for 72 h and subjected to further analysis.

**Protein depletion.** Protein lysates were immunoprecipitated consecutively three times with either an HSP70 (Enzo), HSC70 (Enzo) or HOP or with the same species normal antibody as a negative control (Santa Cruz). The resulting supernatant was collected and run on a native or a denaturing gel.

### 5.3.2 Results

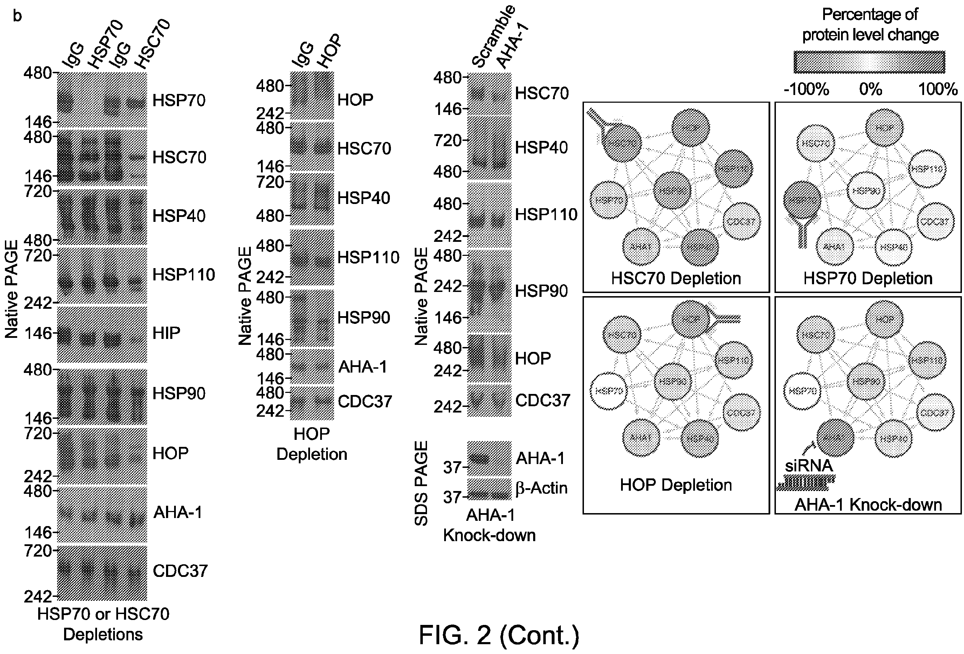

To define the composition of the stable multimeric chaperome complexes in type 1 tumors and investigate the relationship between their components, we either altered the cellular expression of individual chaperome members, namely HSP70, HSC70, HOP, and AHA-1, or captured complex participants using baits specific for HSP90 and HSP70 *(see* Example 4). When we reduced the levels of HSP70 or HSC70 by immunodepletion, the reduction of HSC70 had the most robust effect and resulted in a reconfiguration of the high molecular weight chaperome species **(****FIG. 2b****).** Within these complexes, immunoblotting confirmed changes in the amount of HSP40, Heat Shock 105kDa/110kDa Protein 1 (HS105 or HSP110), and HSC70-interacting protein (HIP) (alternatively named ST13, suppressor of tumourigenicity), all of which are known HSP70-interacting co-chaperones. We also observed changes in the levels of HSP90, HOP, and surprisingly of AHA-1 and CDC37, which are co-chaperones associated with HSP90 activity **(****FIG. 2b****).** Substantial reconfiguration of the chaperome only modestly affected total chaperome levels. We found similar results in lysates immunodepleted for HOP, where changes in HSP40, HSP110, and CDC37 could be measured, and in those depleted for AHA-1 where a significant remodelling in the HSP70 co-chaperone machinery was noted. Together, these data point to a functional integration of the HSP90 and HSP70 chaperone machineries in type 1 cells **(****FIG. 2a****, b).**

### 5.4. Example 4: Capturing Proteins of the Epichaperome Using Affinity-Based Proteomics

### 5.4.1 Materials and Methods

**Chemical bait proteomics.** PU-H71 beads (Moulick, K. et al. Affinity-based proteomics reveal cancer-specific networks coordinated by Hsp90. Nature chemical biology 7, 818-826 (2011) and YK beads (Rodina et al., ACS Chem Biol. 2014 Aug 15;9(8):1698-705) were generated as described elsewhere. Protein extracts were prepared either in 20 mM HEPES pH 7.5, 50 mM KCl, 5 mM MgCl₂, 1% NP40, and 20 mM Na₂MoO₄ for PU-H71 beads pull-down, or in 20 mM Tris pH 7.4, 150 mM NaCl, and 1 % NP40 for YK beads pull-down. Samples were incubated with PU-H71 beads for 3-4 h or with YK beads overnight, at 4°C, then washed and subjected to SDS-PAGE with subsequent immunoblotting and Western blot analysis. Proteomic analyses were performed using the published protocol (Moulick, K. et al. Affinity-based proteomics reveal cancer-specific networks coordinated by Hsp90. Nature chemical biology 7, 818-826 (2011) and Nayar, U. et al. Targeting the Hsp90-associated viral oncoproteome in gammaherpesvirus-associated malignancies. Blood 122, 2837-2847 (2013). Control beads contained an inert molecule.

**Bioinformatics analyses.** The exclusive spectrum count values, an alternative for quantitative proteomic measurements, were used for protein analyses. CHIP and PP5 were examined and used as internal quality controls among the samples. Statistical analyses were performed using R (version 3.1.3). Differential protein enrichment analyses were performed using the moderated linear model from the limma package of Bioconductor. Logarithmic values for protein abundance were used in the differential protein enrichment analyses. The empirical Bayesian statistics using a contrast fit model was used to calculate the *p*-value to reflect the differential protein abundance between type 1 cells and combined type 2 and non-transformed cells. A heatmap was created to display the shortlisted proteins using the package "gplots" and "lattice".

**The protein-protein interaction (PPI) network.** Proteins displayed in the heatmap were uploaded in STRING database to generate the PPI networks. The thickness of the edges represents the confidence score of a functional association. The score was calculated based on four criteria: co-expression, experimental and biochemical validation, association in curated databases, and co-mentioning in PubMed abstracts. Proteins with no adjacent interactions were not shown. The color scale in nodes indicates the average enrichment of the protein (measured as exclusive spectral counts) in type 1, type 2, and non-transformed cells, respectively. The network layout for type 1 tumours was generated using edge-weighted spring-electric layout in Cytoscape with slight adjustments of marginal nodes for better visualization. The layout for type 2 and non-transformed cells retains that of type 1 for better comparison. Proteins with average relative abundance values less than 1 were deleted from analyses. The biological processes in which they participate and the functionality of proteins enriched in type 1 tumours were assigned based on gene ontology terms and based on their designated interactome from UniProtKB, STRING, and/or I2D databases

### 5.4.2 Results

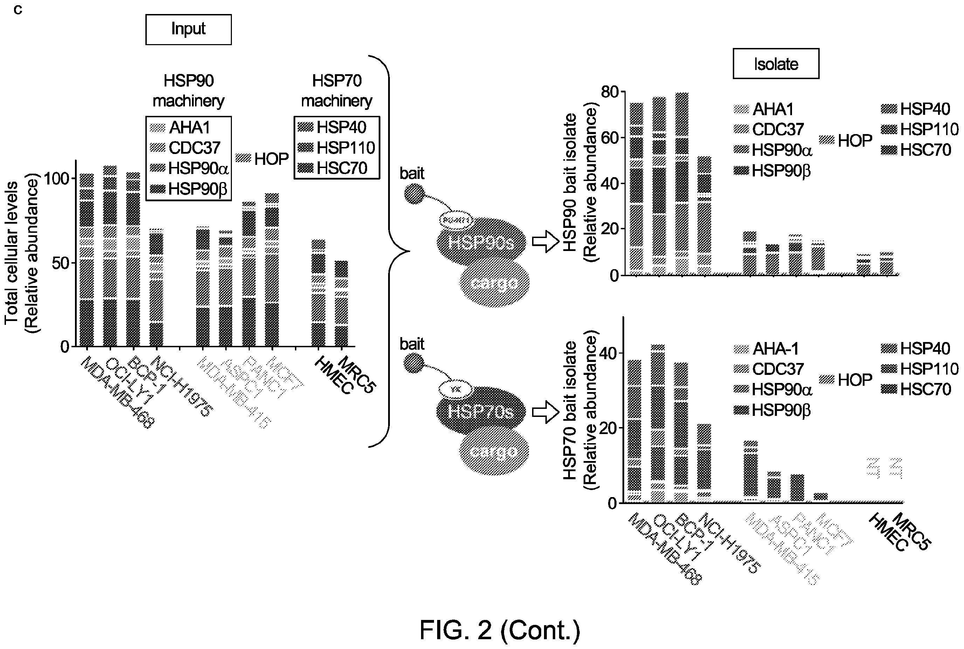

We isolated the endogenous chaperome complexes from type 1, type 2, and non-transformed cells through the use of chemical baits that capture HSP90 and its associated interactome (Moulick, K. et al. Affinity-based proteomics reveal cancer-specific networks coordinated by Hsp90. Nature chemical biology 7, 818-826 (2011)), or HSC70/HSP70 and its interactome (Rodina, A. et al. Affinity purification probes of potential use to investigate the endogenous Hsp70 interactome in cancer. ACS Chem Biol 9, 1698-1705 (2014)). We then probed the isolates for major chaperome members, especially known regulators of the HSP70s such as HSP40 and HSP110, and of the HSP90s such as AHA-1 and CDC37. We also probed for HOP, a bridging co-chaperone of the HSP70 and HSP90 machineries (**FIG. 2a****, c).** Two major findings emerged from these experiments. First, we found an enrichment of HSP90- and HSC70/HSP70-nucleating complexes in type 1 tumors. In contrast, under identical experimental conditions, fewer such complexes were isolated in type 2 cancer cells and in non-transformed cells. Second, in type 1 cells we unexpectedly found HSP70 co-chaperones, such as HSP110 and HSP40, to be enriched on the HSP90-directed bait, while HSP90 co-chaperones, AHA-1 and CDC37, were enriched on the HSP70-directed bait. These observations indicate a physical integration of HSP90 and HSP70 machineries **(****FIG. 2a****, c).**

The currently accepted mechanism for the action of the HSP90 machinery is a sequential one involving the temporal assembly and disassembly of early, intermediate, and late stage chaperone complexes. In the early stages, HSP70 together with one of the HSP40 co-chaperones captures nascent or denatured proteins. Next, an intermediate complex is formed in which the client protein is transferred from the HSP70 complex to the HSP90 complex. Conformational changes regulated by nucleotides and co-chaperones shift this to the mature complex, where the active client protein is released. HOP and CDC37 are intermediate stage co-chaperones controlling the entry of clients into the pathway, while p23 and AHA-1 are involved in the later stages of the cycle that lead to client protein maturation (Rehn, A. B. & Buchner, J. in The Networking of Chaperones by Co-chaperones 113-131 (Springer, 2015)).

The model of transitory HSP90 and HSP70 chaperome complexes acting sequentially to fold proteins during homeostasis does not, however, explain the observed co-existence of several HSP90- and HSP70-centric complexes in type 1 tumors. Instead, our data indicate that a unique reconfiguration occurs in type 1 tumors to yield a chaperome that is distinct from that seen in type 2 tumors and in normal cells. The type 1 chaperome is characterized by the existence of stable HSP90 and HSP70-centric complexes that concomitantly incorporate the co-chaperones of both machineries; this is evidenced by their stability under native PAGE conditions and also by their capture by both baits.

To understand the biochemical mechanism behind the specific reconfiguring of the chaperome in type 1 tumors and to identify the components of these multimeric complexes, we performed a non-biased proteomic analysis. Specifically, we used the HSP90 bait to probe homogenates from type 1 (n=6) and type 2 (n=3) cancer cells, and from non-transformed (n=3) cells. To ensure that we captured a majority of HSP90 complexes, we performed these studies under conditions of HSP90-bait saturation. We then subjected the protein isolates to mass spectrum analyses and found a variety of HSP90 interactions, including client proteins and modulators. Of these, we chose 110 proteins for bioinformatics analyses, encompassing proteins known to function as chaperone, co-chaperone, scaffolding, foldase, and isomerase proteins, and thus more likely to participate in the chaperome reconfiguration observed in type 1 tumors.

### 5.5. Example 5: The Epichaperome Facilitates Cancer Cell Survival

### 5.5.1 Materials and Methods

Reagents. HSP90 and HSP70 inhibitor drugs used in this study including PU-H71, NVP-AUY-922, SNX-2112, and YK were synthesized as previously reported (Moulick, K. et al. Affinity-based proteomics reveal cancer-specific networks coordinated by Hsp90. Nature chemical biology 7, 818-826 (2011) and Taldone, T. et al. Heat shock protein 70 inhibitors. 2. 2,5'-thiodipyrimidines, 5-(phenylthio)pyrimidines, 2-(pyridin-3-ylthio)pyrimidines, and 3-(phenylthio)pyridines as reversible binders to an allosteric site on heat shock protein Journal of medicinal chemistry 57, 1208-1224 (2014)). STA-9090 was purchased from MedKoo Biosciences and CUDC-305 from ChemieTek.

Lysates of the indicated cells MDAMB468 (Type 1, high epichaperome, HSP90 addicted, sensitive to HSP90 inhibitors), ASPC1 (Type 2, low to no epichaperome, HSP90 dependent, resistant to HSP90 inhibitors) and HMEC (non-transformed) were incubated with increasing amounts of PU-H71-beads (PU-H71 attached to a solid support) or with control beads (beads with an inert chemical attached) **(****FIG. 3a****).** The supernatant was applied to native gel separation followed by immunoblotting with HSP90 and other epichaperome complex components (left, example for MDAMB468) or nanopro isoelectric focusing and immunoblotting (left, shown for all 3 cells). Duplicates of each experimental condition are presented. Data were graphed to indicate the relative binding affinity of PU-H71 to the HSP90 species as expressed in the 3 cell types.

The biochemical profile of HSP90 under native and denaturing gel conditions in the indicated cancer cells (breast, lymphoma, pancreatic cancer are shown). Isoelectric focusing via nanopro shows the abundance of the epichaperome complexes in Type 1 (addicted, sensitive to HSP90 inhibitors) and Type 2 (dependent, resistant to HSP90 inhibitors) cancer cells **(****FIG. 3b****).**

Epichaperome abundance determined by the PU-FITC flow cytometry assay. **(****FIG. 3c****).** Cells were treated with 1 gM PU-H71-FITC. At 4 h post treatment, cells were washed twice with FACS buffer. To measure PU-H71-FITC binding in live cells, cells were stained with 7-AAD in FACS buffer at room temperature for 10 min, and analyzed by flow cytometry (BD Biosciences). Cell viability was determined using Annexin v staining. At 48h post 1 **µ** M PUH71 treatment, cells were stained with Annexin V-V450 (BD Biosciences) and 7-AAD in Annexin V buffer, and subjected to flow cytometry to measure viability determined by AnnexinV/7AAD double negative gates.

### 5.5.2 Results

As shown in FIG. **3a****,** PU-H71 attached to a solid support was able to effectively reduce the amount of epichaperome formation, as measured by isoelectric focusing. As the concentration of PU-H71 was increased, there was a clear dose-dependent reduction in the amount of epichaperome in the lysates. In FIG. **3b****,** we show that the abundance of epichaperome complexes in cells sensitive to HSP90 inhibitors is significantly greater than in cells not sensitive to HSP90 inhibitors. In **Fig. 3c****,** we show that the HSP90 inhibitor PU-H71 has a higher affinity for the stress HSP90 species versus the housekeeping, normal cell HSP90 and thus a properly labeled PU-H71 can be used to quantify the abundance of stress HSP90 in live cells.

Moreover, we found that cells enriched in the epichaperome were more likely to die when exposed to PU-H71 in comparison to cells with lower levels of the epichaperome **(****FIG. 3d****).** In over 90 cancer cells lines encompassing breast cancer, lung cancer, pancreatic and gastric cancers, and leukemia and lymphomas, we found a significant correlation (P < 0.0001) between the abundance of the epichaperome and the susceptibility of these cancer cells to HSP90 inhibition. Lowering the abundance of the epichaperome by reducing the levels of AHA-1 protein resulted in cells less amenable to killing by PU-H71 **(****FIG. 3e****).** The effect was not confined to PU-H71 treatment, since chemically distinct HSP90-directed agents of various selectivities for HSP90 complexes recapitulated the profile of PU-H71. Type 1 tumors were also effectively killed by these agents, whereas type 2 tumors remained refractory **(****FIG. 3g****).** Together, these observations confirm the epichaperome as a means of facilitating survival in these tumors.

### 5.6. Example 6: Ex Vivo Studies

### 5.6.1 Materials and Methods

**Primary breast tumor protocol.** The *ex vivo* protocol employed on this study is depicted schematically in **FIG. 4****.** Sample preparation and treatment are detailed in Ex vivo treatment response of primary tumors and/or associated metastases for preclinical and clinical development of therapeutics. Corben AD, Uddin MM, Crawford B, Farooq M, Modi S, Gerecitano J, Chiosis G, Alpaugh ML.J Vis Exp. 2014 Oct 2;(92):e52157.

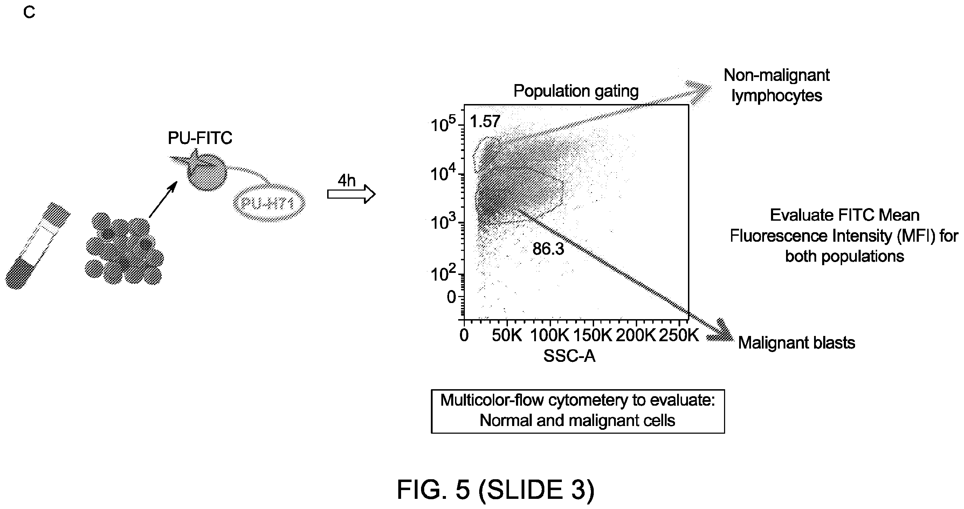

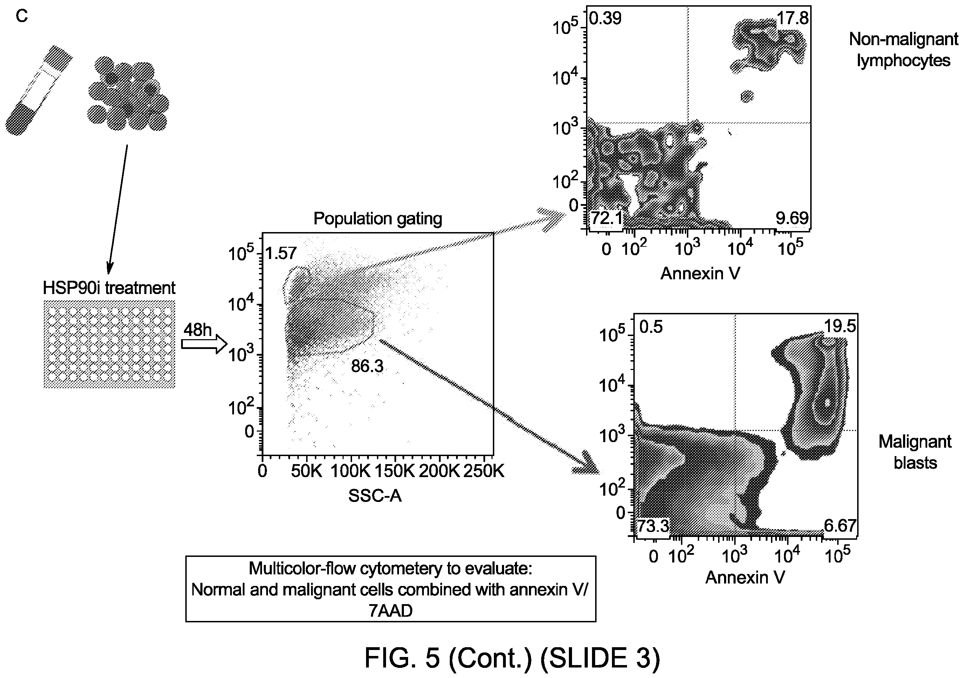

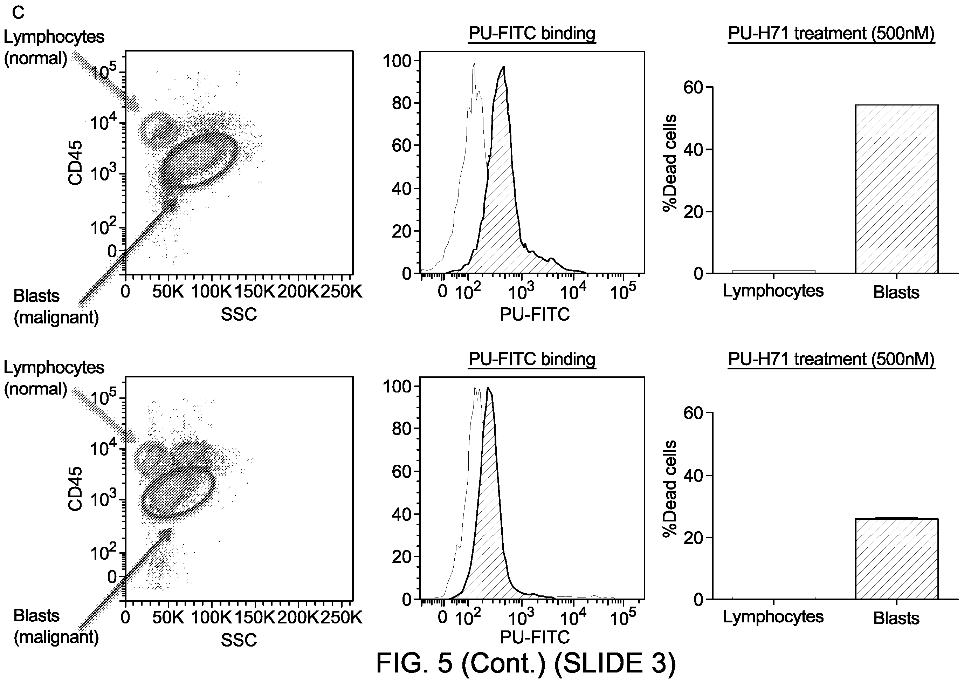

**PU-FITC flow assay in leukemia samples (****FIG. 5c****).** PU-FITC assay was performed as previously described (Taldone, T. et al. Synthesis of purine-scaffold fluorescent probes for heat shock protein 90 with use in flow cytometry and fluorescence microscopy. Bioorganic & medicinal chemistry letters 21, 5347-5352 (2011). Briefly, cells were incubated with 1 **µ**M PU-FITC at 37°C for 4 h. Then cells were washed twice with FACS buffer (PBS/0.5% FBS), and re-suspended in FACS buffer containing 1 **µ**g/ml DAPI. The mean fluorescence intensity (MFI) of PU-FITC in treated viable AML cells (DAPI-ve) was evaluated by flow cytometry. For primary AML specimens, cells were also stained with anti-CD45-APC-H7, to identify blasts and lymphocyte populations (BD biosciences). Blasts and lymphocyte populations were gated based on SSC vs CD45. The FITC derivative FITC9 was used as a negative control.

**Primary breast cancer specimens.** Patient tissue procurement was authorized through institutionally review board-approved bio-specimen protocol#09-121 at Memorial Sloan Kettering Cancer Centre (New York, NY). Specimens were treated for 24 h with the indicated concentrations of PU-H71. Following treatment, slices were fixed in 4% formalin solution for 1 h then stored in 70% ethanol. For tissue analysis, slices were embedded in paraffin, sectioned, slide-mounted, and stained with haematoxylin and eosin. Tissue slides were assessed blindly by a breast cancer pathologist who gauged the apoptosis present in the tumor as well as any effect to normal tissue.

**Primary acute myeloid leukemia.** Cryopreserved primary AML samples were obtained with informed consent and Weill Cornell Medical College institutional review board approval, and from the University of Pennsylvania Stem Cell & Xenograft Core Facility. Samples were thawed and cultured for 1 h at 37°C followed by treatment as described previously (Hassane, D. C. et al. Chemical genomic screening reveals synergism between parthenolide and inhibitors of the PI-3 kinase and mTOR pathways. Blood 116, 5983-5990 (2010)).

**Epichaperome abundance determined by the PU-FITC flow cytometry assay.** Cells were treated with 1 µM PU-H71-FITC. At 4 h post treatment, cells were washed twice with FACS buffer (PBS-0.5% FBS). To measure PU-H71-FITC binding in live cells, cells were stained with 7-AAD in FACS buffer at room temperature for 10 min, and analyzed by using an BD-LSR-II or BD-Canto cytometry (BD Biosciences). Cell viability was determined using Annexin-V staining. At 48h post 1 µM PUH71 treatment, cells were stained with Annexin-V BD Horizon-V450 (BD Biosciences) and 7-AAD in Annexin-V buffer, and subjected to flow cytometry to measure viability determined by AnnexinV/7AAD double negative gates.

### 5.6.2 Results

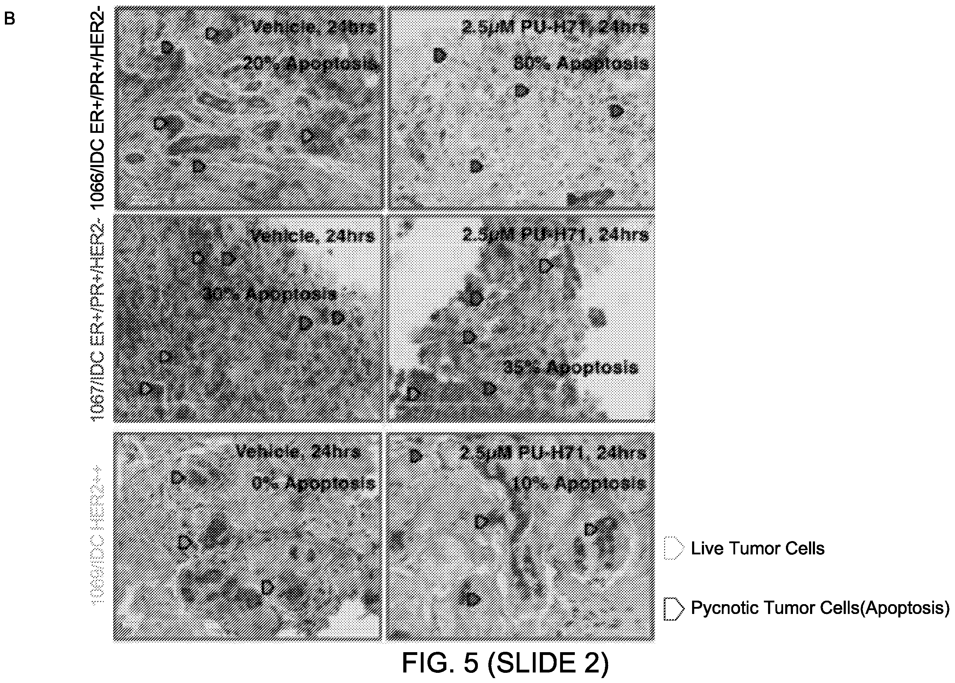

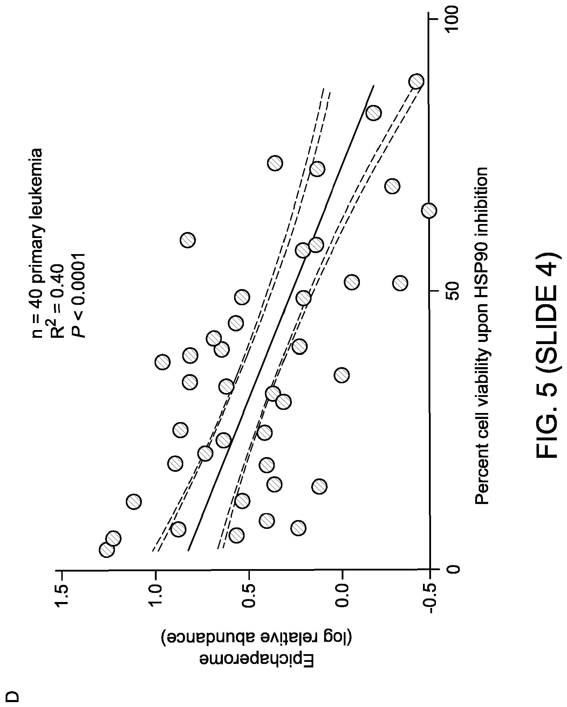

We validated the functional significance of the epichaperome in primary tumors *ex vivo* **(****FIGS. 5a-5d****).** When primary breast tumors (n=4) **(****FIGS. 5a****, 5b)** and acute myeloid leukemias (n=40) **(****FIG. 5c****, 5d)** were treated *ex vivo* with PU-H71, we found that those expressing the stable multimeric epichaperome forms to be most effectively killed by PU-H71. In contrast, and similar to our findings in cultured cells, type 2 tumors remained mostly unaffected. Adjacent benign tissue in the case of the breast specimens and non-malignant lymphocytes in the AML samples contained little to none of the epichaperome, and were accordingly insensitive to PU-H71 **(****FIGS 5a** **and** **5b****).**

### 5.7 Example 7: Dependence of Tumors on the Epichaperome

### 5.7.1 Materials and Methods

Flow cytometry assay: PU-FITC assay was performed as previously described (Taldone, T. et al. Synthesis of purine-scaffold fluorescent probes for heat shock protein 90 with use in flow cytometry and fluorescence microscopy. Bioorganic & medicinal chemistry letters 21, 5347-5352 (2011)). Briefly, cells were incubated with 1 **µ**M PU-FITC at 37°C for 4 h. Then cells were washed twice with FACS buffer (PBS/0.5% FBS), and re-suspended in FACS buffer containing 1 **µ**g/ml DAPI. The mean fluorescence intensity (MFI) of PU-FITC in treated viable AML cells (DAPI-ve) was evaluated by flow cytometry.

**Flow cytometry analysis for Circulating tumor cells (CTCs).** Peripheral blood was collected in EDTA tubes (10ml) pre- and post-PU-H71 treatment as per NCT01393509 clinical study. Buffy coat was obtained by ficoll gradient separation. PU-FITC binding assay was performed as described above. Briefly, 2 million cells were treated for 6 h with 1**µ**M PU-FITC or controls at 37°C. Cells were then washed (1×PBS/5%FBS) and stained with EpCAM-PE (BD Biosciences), CD14-APC-Cy7 and CD45-APC (ebiosciences, CA) for 45mins on ice. Cells were washed and stained with DAPI (1 **µ**g/ml) for 30 minutes at 4°C. At least 1 million events were acquired using a BD LSRII flow cytometer (BD Biosciences).

### 5.7.2 Results

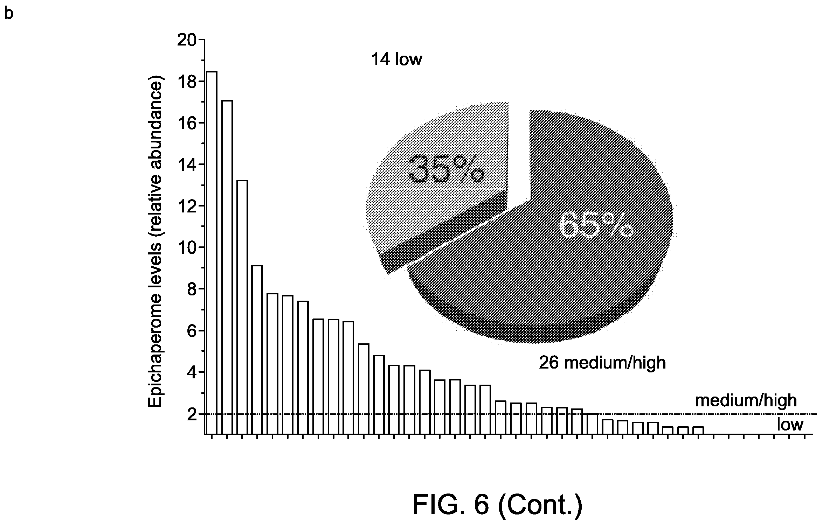

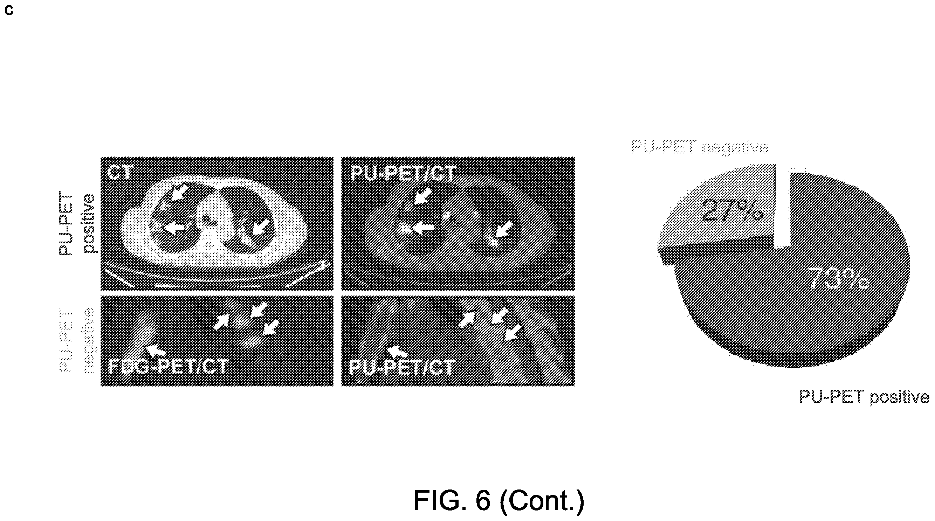

We assessed the prevalence of tumors enriched for epichaperome complexes. We performed flow cytometry with PU-FITC on 95 cancer cell lines encompassing pancreatic, gastric, lung, breast cancers, lymphomas, and leukemias, and found approximately 60-70% presented medium to high levels of epichaperome complexes **FIG. 6a****.** When this study was repeated on patient tumor samples, similar results were obtained with primary liquid tumors (n=40), and solid tumors including lymphomas **(****FIGS. 6b** **and** **6c****).** This establishes that over half of tumors use the epichaperome irrespective of their subtype, provenience, and genetic background. It also established that tumors express distinct levels of the epichaperome, and thus distinct sensitivity to inhibition of epichaperome components, *i.e.* to single agent administration of an agent such as an HSP90 inhibitor.

### 5.8 Example 8: Inducing Formation of Epichaperome

### 5.8.1 Materials and Methods

Cells were cultured using the procedure described in Example 1. The proteotoxic stressor (e.g., paclitaxel or bortezomib) was added for 6 hours and then the cells were lysed. The homogenate was applied for separation on a gel under native conditions.

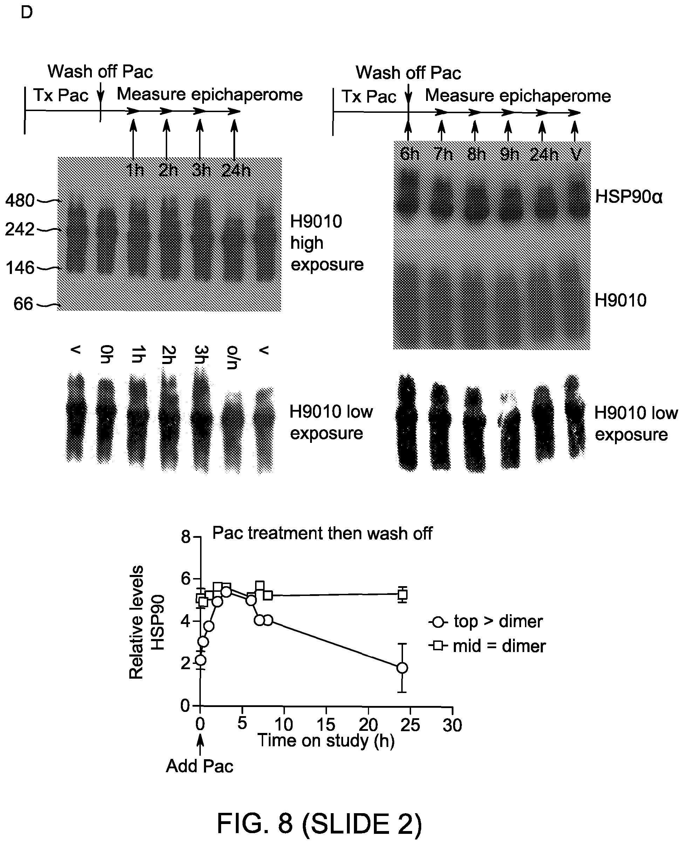

For the wash off studies **(****FIG. 8d****),** cells were cultured and then treated with paclitaxel (referred to as PAC in figure) for 1-2 hours to mimic *in vivo* conditions. The paclitaxel was then washed off to determine how long the epichaperome is maintained *in vitro.*

### 5.8.2 Results

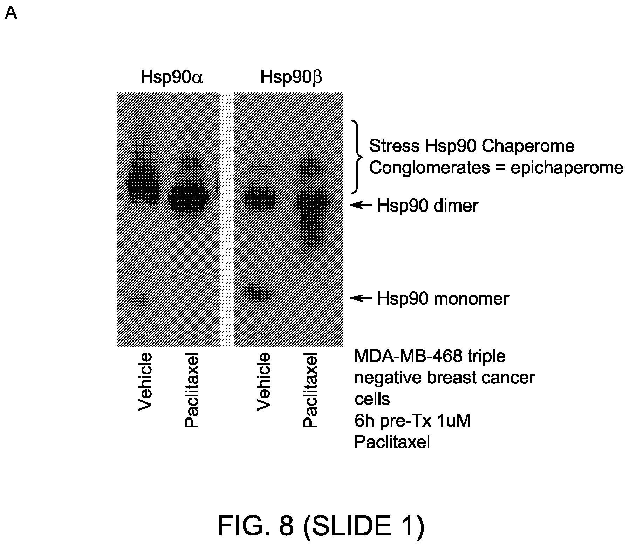

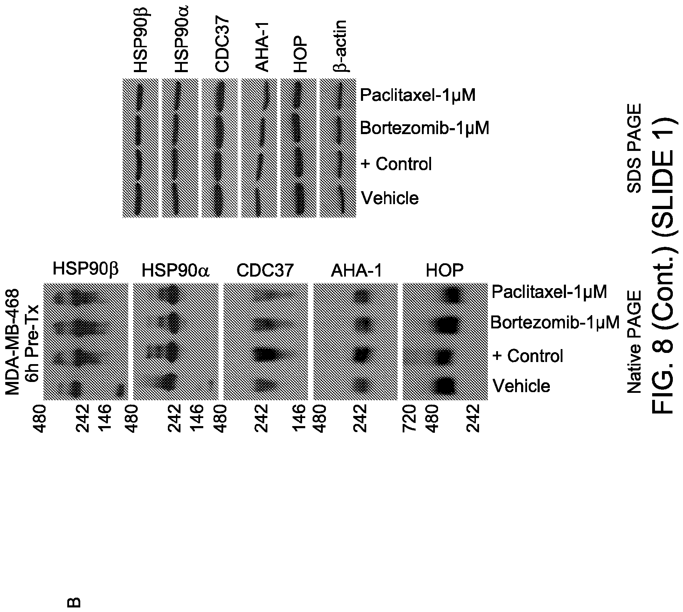

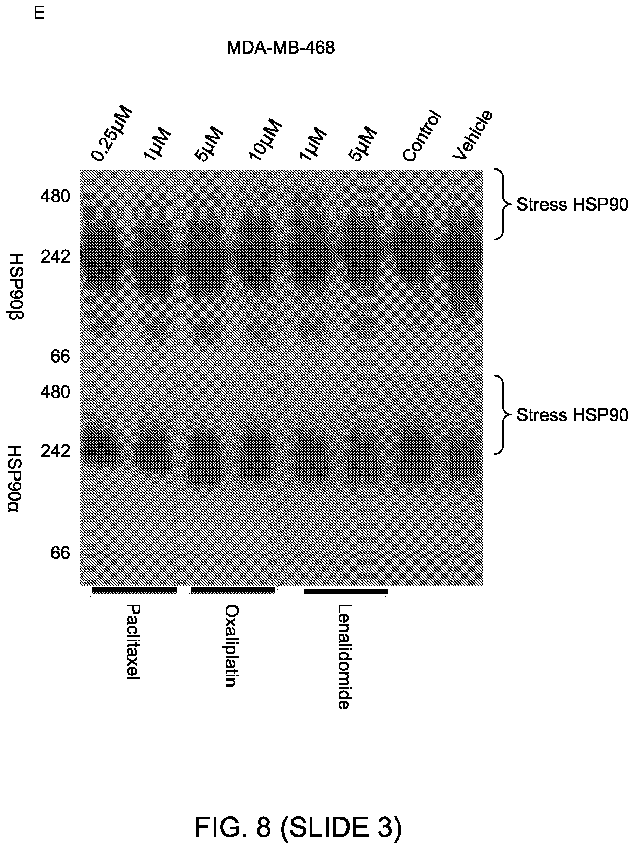

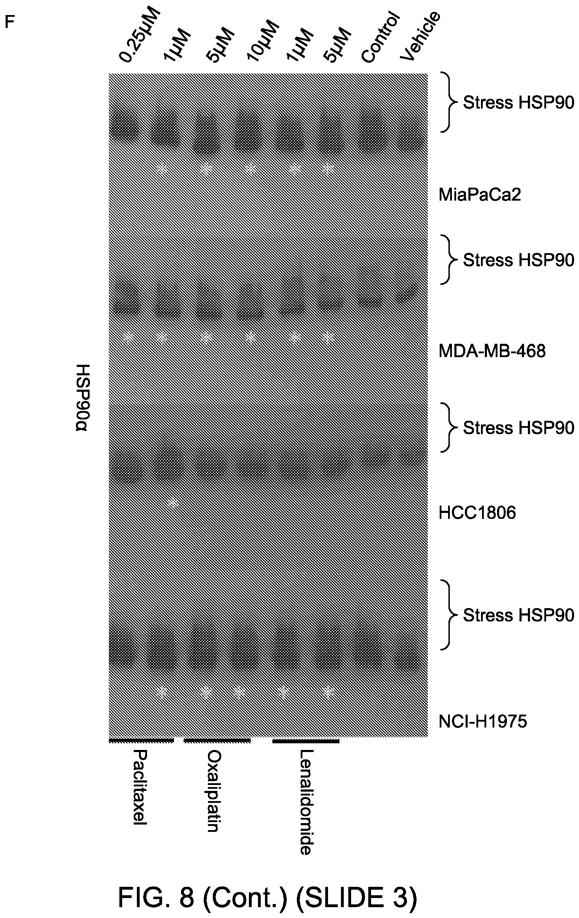

The results of studies using chemotherapeutic agents as proteotoxic stressors are depicted in **FIGS. 8a****,** **8b****,** **8e****,** **8f** and **10****.** The results were discussed in Section 4 above. With respect to **FIG. 8d****,** the biochemical profile of HSP90 was assessed under native conditions. Cells were pretreated with paclitaxel for 1h then drug was washed off to mimic *in vivo* conditions. The epichaperome levels (labeled top > dimer) follow a bell shape with a peak noted at 5-7h, then decrease to endogenous levels by 24h.

The results of studying the effects of phosphatase or phosphorylation inhibitors are depicted in **FIGS 9a** and **9b****,** respectively. These results were discussed in Section 4 above.

### 5.9 Example 9: Combination of Docetaxel/PU-H71 (MiaCa2 Pancreatic Cancer Cells)

### 5.9.1 Materials and Methods

Viability of MiaPaCa2 pancreatic cancer cells treated with the sequential combinations of vehicle, PU-H71 and Docetaxel as indicated. Cell viability at 72h was measured using the Sulforhodamine B assay.

### 5.9.2 Results

The results of the study with MiaPaCa2 pancreatic cells are depicted in **FIG. 8c****.** These results were discussed in Section 4 above.

### 5.10 Example 10: In vivo animal studies

### 5.10.1 Materials and Methods

4- to 6-week-old *nu*/*nu* athymic female mice were obtained from Harlan Laboratories. All experiments were carried out under a protocol approved by the Institutional Animal Care and Use Committee at MSKCC and institutional guidelines for the proper and humane use of animals in research were followed. H1975 (3 X 10⁶ cells) or Mia-Paca2 (5 X 10⁶ cells) were subcutaneously implanted in the right flank of mice using a 22-gauge needle and allowed to grow. All mice received Doxycyclin in their feed while on therapy. Tumors were allowed to reach 50-150 mm³ in volume prior to treatment. The mice were randomly grouped into: vehicle, Abraxane^{®}, PU-H71 or various Abraxane^{®} + PU-H71 combinations. Before administration, a solution of PU-H71 was formulated in 10 mM phosphate buffer (pH ~6.4). Pre-formulated Abraxane^{®} (5 mg/ml) was used. Mice bearing H1975 or MiaPaca2 tumors were administered Abraxane^{®} (30 mg/kg) and/or PU-H71 (75 mg/kg) alone or in combination by intraperitoneal (i.p.) injections. Tumor size was measured twice weekly using Vernier calipers and tumor volume was calculated as the product of its (length x width² )/2. Body weights were also measured twice weekly to ensure that there was no visible toxicity associated with treatment.

### 5.10.2 Results

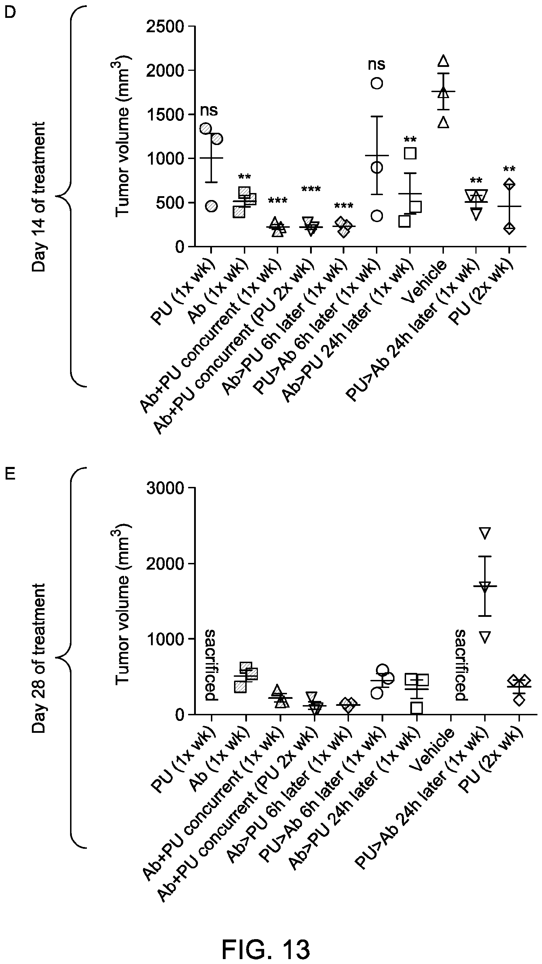

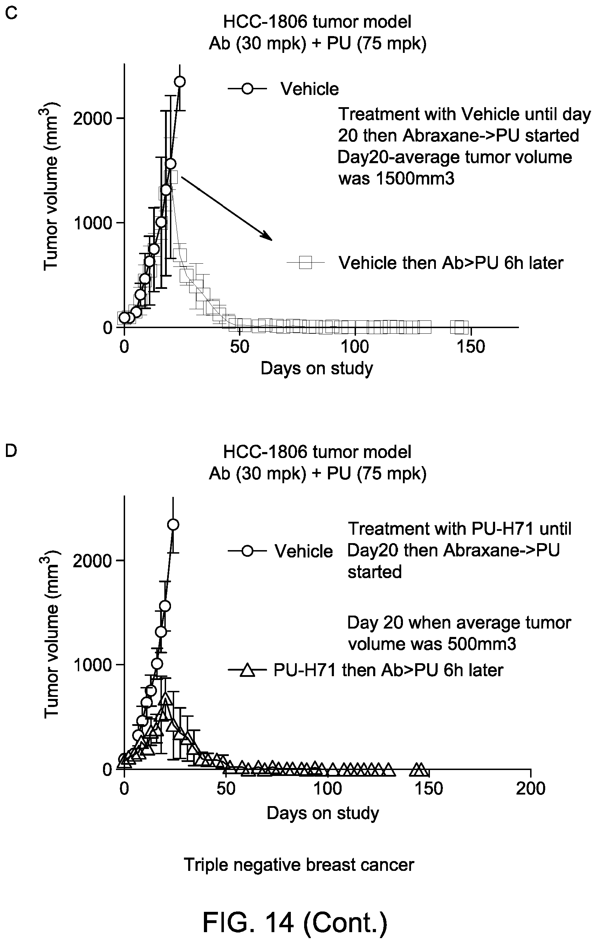

Mice bearing xenografted MiaPaCa2 (pancreatic cancer, gemcitabine resistant) or H1975 non-small lung cancer (mut EGFR, erlotinib resistant) tumors were treated on a once weekly schedule with the indicated agents alone or administered co-mixed or in the sequence Abraxane^{®} followed at 6h by PU-H71 (Abraxane^{®} (30 mg/kg) and/or PU-H71 (75 mg/kg) alone or in combination by intraperitoneal (i.p.) injections; n=5 mice group). Tumor volume was monitored and graphed against the time of treatment. In **FIG. 11****,** the upper panels show averages of 5 mice in each group, while lower panels show data for individual mice monitored. Tumor volumes for the mice treated with PU-H71 six hours after administration of Abraxane^{®} were substantially reduced relative to monotherapy and concurrent administration of abraxane^{®} and PU-H71. The pictures of **FIG. 12** were taken 5 weeks into the treatment regimen and shows representative mice from each treatment arm. While concurrent administration resulted in stasis regression of the tumor, sequential administration of Abraxane^{®} followed by PU-H71 led to a cure, with no detectable tumor evident in the mice. **FIG. 13** shows results for the H1975 lung cancer model. **FIG. 14** shows results for the HCC-1806 and MDA-MB-231 triple negative breast cancer model. The results obtained from these models are consistent with the results obtained in the MiaPaCa2 pancreatic cancer model.

### 5.11 Example 11: Incucyte kinetic growth assay

### 5.11.1 Materials and Methods

MDA-MD-231 and BT20 triple negative breast cancer cells were seeded in tissue culture treated dishes. Once confluent to approximately 50% of the vessel surface area, the cells were treated with medium containing 2 **µ**M paclitaxel, 5 **µ**M PU-H71, vehicle (DMSO), or a combination of 2 **µ**M paclitaxel and 5 **µ**M PU-H71 (together or sequential). Cells were grown in these conditions in an Essen IncuCyte housed in a standard cell culture incubator. Nine images per condition were collected every 2 hours for a total of 316 hours. After 24 hours under the aforementioned treatment conditions, paclitaxel treated cells were treated with medium containing 5 **µ**M PU-H71 or the control vehicle, PU-H71 treated cells were treated with medium containing 2 **µ**M paclitaxel or control vehicle, and the cells treated with vehicle and the combination of PU-H71 with paclitaxel were treated with medium containing control vehicle. All secondary drug additions were added to existing medium containing the original treatment. The existing medium was additionally diluted twice more with medium containing the control vehicle; each dilution occurring 24 hours after the previous. After 96 hours in culture, the medium was aspirated from each well and replaced with cell-appropriate medium with no pharmacological agents. As stated above, cells were monitored for a total of 316 hours (*i.e.,* 200 hours after complete removal of pharmacological agents).

### 5.11.2 Results

While *in vitro* experiments cannot recapitulate the *in vivo* treatment paradigms, this experiment shows, similarly to the Example in vivo above, that sequential addition of paclitaxel -HSP90 inhibitor is more cytotoxic than either agent alone or combination of the two agents added together. BT20 triple negative breast cancer cells have mid-level epichaperome (as measured above) and are sensitive to taxanes. As seen in **FIG. 15****,** each agent alone has a cytotoxic effect in these cells as monitored by microscopy that records cell confluency (IncuCyte system). At approximately 130h, the media is replaced, removing dead cells and cell debris (floating cells, cell debris are removed by wash, indicated by the sudden drop in the signal). Between 130h and approximately 230h the instrument records whether there are remaining live cells in each treatment group and whether these have the ability to grow. As see for PU-H71 and Pac alone, there are a few live cells and these cells start to regrow (curve moves upward). There are lesser live cells for the group receiving sequential Pac->PU than Pac and PU added concomitantly. Data collected after 230h [second sudden signal drop following media change] are not reliable due to the sensitivity limit of the instrument.

### 5.12 Example 12: Combination of Doxirubicin/PU-H71 (Lymphoma)

### 5.12.1 Materials and Methods

BJAB Burkitt lymphoma cells were exposed to serial dilutions of DOX for 24 h prior to addition of 500 nM PU-H71. Viability was measured 24 h later using standard MTS assay. Sextuplicate data points were averaged and normalized against untreated (DOX alone) or PU-H71-treated (DOX followed by PU-H71) controls. Data is presented as mean ± SD. Note that the IC50 for DOX shifts from 400nM (closed circles) to 75 nM (open circles) when 500 nM PU-H71 is added sequentially after DOX (Fig 18a).

Pairwise drug interactions were systematically evaluated on a 6-by-6 dose-response matrix analyzed using CompuSyn, yielding 25 individual combinations at multiple drug ratios. Farage DLBCL cells were exposed to the following combinations: DOX followed by PU-H71 added with a 24 h-delay (brown circles, DOX→PU-H71), PU-H71 followed by DOX added with a 24 h-delay (blue circles, PU-H71→DOX), or concurrent administration (black circles, DOX + PU-H71). Drug interactions were evaluated in Fa-CI plots, where additivity, synergy, and antagonism are defined as CI=1, CI < 1, and CI > 1, respectively. Combination Index (CI) values were calculated from experimental survival data using Compusyn Software and are shown as a function of the Fraction Affected (Fa, fraction of cells killed by the combination). The Fa-CI plot was constructed by simulating CI values over the entire range of Fa values from 5% to 95% (lines) using CompuSyn (FIG. 18b).

SUDHL4 DLBCL cells were exposed to PU-H71 and DOX or combinations in different schedules as indicated. Caspase-3 activation was measured by flow cytometry using PE-conjugated anti-active Caspase-3 antibody (FIG. 18C). Cell cycle analysis was performed in parallel and is shown in the inset at top left of each scatterplot. Frequency of hypodiploid population is indicated.

### 5.12.2 Results

The results of the study with lymphoma cells are depicted in **FIG. 18****.** These results are discussed in Section 4 above.

### 5.13 Example 13: Combination of Abraxane^{®}/PU-H71 (Lung, TNBC)

These experiments further demonstrate the effect and safety of the Sequential (Abraxane^{®}->PU-H71@6h) treatment under long-term administration, and to evaluate tumor relapse or the lack of following treatment cessation. The effect on tumor growth (Figure 19a-c and Figure 20a,b) and mouse weight (Figure 19d and Figures 20 c,d) of Abraxane^{®} and PU-H71 administered alone, concurrently or in the sequence Abraxane^{®} followed by PU-H71 at 6h was analyzed. Mice (n=3-5) bearing xenografted NCI-H1975 lung cancer, HCC1806, triple negative breast cancer or MDA-MB-231, triple negative breast cancer tumors were administered intraperitoneally, once a week PU-H71 (75mg/kg) and Abraxane^{®} (30mg/kg) alone or in combination. No adverse effects were observed during the study. All animals survived to scheduled endpoints and appeared healthy, maintained a normal body weight, and behaved normally from the time of receipt through the end of the study. Although, both concurrent and sequential combination therapies significantly suppressed tumor growth, all tumors treated with concurrent Abraxane^{®}+PU-H71 relapsed during and/or following treatment cessation. Several mice treated with Sequential (Abraxane^{®}->PU-H71@6h) remained tumor-free several months after treatment cessation (Figures 20a,b). For example, 3 out of five HCC1806 bearing mice were tumor-free 100 days after treatment cessation, and two out of five MDA-MB-231 mice were tumor free 120 days after treatment cessation.

### 5.14 Example 14: Combination of Abraxane^{®}/PU-H71 (Pancreatic)

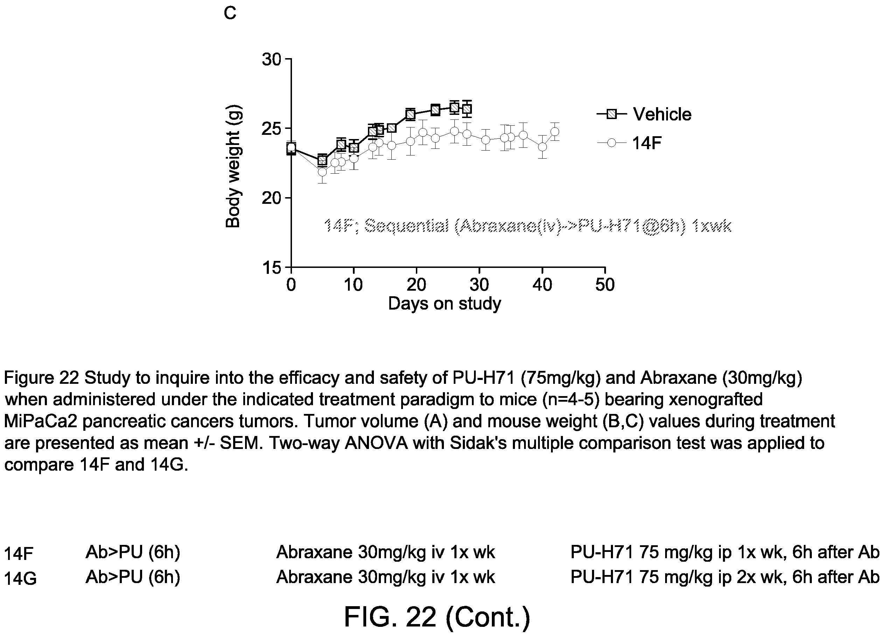

These experiments further demonstrate the effect and safety of the Sequential (Abraxane^{®}->PU-H71@6h) treatment when Abraxane^{®} is administered intravenously. It is also designed to compare the effect of PU-H71 given once or twice a week in sequential administration with Abraxane^{®}. Mice (n=5) bearing xenografted MiaPaca2 pancreatic cancer tumors were treated with Abraxane^{®} and PU-H71 as indicated below. Tumor volume and mouse body weight were monitored during treatment and are graphed in Figure 21a and Figure 21b, respectively, over the course of the 40-day treatment period (see also Figures 22b,c). PU-H71 (75mpk) was administered alone or in combination once a week (1xwk) or twice per week (2xwk, Mon-Thu) by ip injection. Abraxane^{®} was given alone or in combination 1xwk at 30mpk, intravenously or intraperitoneally, as indicated. When combined, PU-H71 and Abraxane^{®} were given concurrently, or using the Ab->PU 6h sequencing strategy. Nine mice were administered the vehicle control - at day 21 of treatment, five of the eight mice were switched to Sequential (Abraxane^{®} (iv)->PU-H71@6h) and the remaining four were continued on vehicle.

No adverse effects were observed during the study. All animals survived to scheduled endpoints and appeared healthy, maintained a normal body weight, and behaved normally from the time of receipt through the end of the study.

No difference in efficacy was observed when PU-H71 was given with Abraxane^{®} once or twice a week.

Large tumors (1319±583mm3) that received a one dose of Sequential (Abraxane^{®} (iv)->PU-H71@6h), regressed in two days to 817.9±515mm3 (38% regression), and after the second dose to 452.1±236mm3 (65% regression), and continued to decrease as treatment continued (Figure 22a).

### Example 15: Combination of Abraxane^{®}/PU-H71 (TNBC)

These experiments further demonstrate the effect and safety of the Sequential (Abraxan^{®}->PU-H71@6h) treatment when Abraxane^{®} is administered intravenously. It is also designed to compare the effect of PU-H71 given once or twice a week in sequential administration with Abraxane^{®}. Mice (n=5) bearing xenografted HCC1806 triple negative breast cancer tumors were treated with Abraxane^{®} and PU-H71 as indicated below. Tumor volume and mouse body weight were monitored during treatment and are graphed in Figure 23a and Figure 23b, respectively, over the course of the 40-day treatment period. PUH71 (75mpk) was administered alone or in combination once a week (1xwk) or twice per week (2xwk, Mon- Thu) by ip injection. Abraxane^{®} was given alone or in combination 1xwk at 30mpk, intravenously, as indicated. When combined, PU-H71 and Abraxane^{®} were given concurrently, or using the Ab->PU 6h sequencing strategy. Five mice were administered the vehicle control.

No adverse effects were observed during the study. All animals survived to scheduled endpoints and appeared healthy, maintained a normal body weight, and behaved normally from the time of receipt through the end of the study.

No difference in efficacy was observed when PU-H71 was given with Abraxane^{®} once or twice a week.

## Claims

1. An inhibitor of HSP90 for use in a method of treating cancer, the method comprising administering to a cancer patient the inhibitor of HSP90 following pretreatment with a proteotoxic stressor, wherein the proteotoxic stressor is administered at a sufficient time prior to administration of the HSP90 inhibitor to increase the formation of the epichaperome, wherein the proteotoxic stressor is administered no more than 24 hours prior to administration of the HSP90 inhibitor; wherein the inhibitor of HSP90 is 8-(6-Iodo-benzo[1,3]dioxol-5-ylsulfanyl)-9-(3-isopropylamino-propyl)-9H-purin-6-ylamine, or a pharmaceutically acceptable salt thereof, and wherein the proteotoxic stressor is a microtubule stabilizing agent.

2. A proteotoxic stressor for use in a method of treating cancer, the method comprising administering to a cancer patient an inhibitor of HSP90 following pretreatment with the proteotoxic stressor, wherein the proteotoxic stressor is administered at a sufficient time prior to administration of the HSP90 inhibitor to increase the formation of the epichaperome, wherein the proteotoxic stressor is administered no more than 24 hours prior to administration of the HSP90 inhibitor; wherein the inhibitor of HSP90 is 8-(6-Iodo-benzo[1,3]dioxol-5-ylsulfanyl)-9-(3-isopropylamino-propyl)-9H-purin-6-ylamine, or a pharmaceutically acceptable salt thereof, and wherein the proteotoxic stressor is a microtubule stabilizing agent.

3. An inhibitor of HSP90 and a proteotoxic stressor for use in a method of treating cancer, the method comprising administering to a cancer patient the inhibitor of HSP90 following pretreatment with the proteotoxic stressor, wherein the proteotoxic stressor is administered at a sufficient time prior to administration of the HSP90 inhibitor to increase the formation of the epichaperome, wherein the proteotoxic stressor is administered no more than 24 hours prior to administration of the HSP90 inhibitor; wherein the inhibitor of HSP90 is 8-(6-Iodo-benzo[l,3]dioxol-5-ylsulfanyl)-9-(3-isopropylamino-propyl)-9H-purin-6-ylamine, or a pharmaceutically acceptable salt thereof, and wherein the proteotoxic stressor is amicrotubule stabilizing agent.

4. The compound or compounds for use according to any one of claims 1-3, wherein 8-(6-Iodo-benzo[1,3]dioxol-5-ylsulfanyl)-9-(3-isopropylaminopropyl)-9H-purin-6-ylamine, or a pharmaceutically acceptable salt thereof: is administered at least one hour after administering the microtubule stabilizing agent.

5. The compound or compounds for use according to any one of claims 1-3, the method comprising administering a combination of the proteotoxic stressor and the HSP90 inhibitor over a cycle of between 7 and 31 days, wherein the proteotoxic stressor and the HSP90 inhibitor are administered at least once over said cycle, and wherein each administration of said proteotoxic stressor is followed by administration of said HSP90 inhibitor.

6. The compound or compounds for use according to claim 5, wherein the treatment cycle is 7 days.

7. The compound or compounds for use according to claim 6, wherein the microtubule stabilizing agent and 8-(6-1odo-benzo[1,3]dioxol-5-ylsulfanyl)-9-(3-isopropylamino-propyl)-9H-purin-6-ylamine, or a pharmaceutically acceptable salt thereof, are administered only on day 1 of the treatment cycle.

8. The compound or compounds for use according to claim 5, wherein the treatment cycle is 21 days.

9. The compound or compounds for use according to claim 8, wherein the microtubule stabilizing agent and 8-(6-Iodo-benzo[1,3]dioxol-5-ylsulfanyl)-9-(3-isopropylamino-propyl)-9H-purin-6-ylamine, or a pharmaceutically acceptable salt thereof, are administered only on day 1 of the treatment cycle.

10. The compound or compounds for use according to any preceding claim, wherein the salt of 8-(6-lodo-benzo[1,3]dioxol-5-ylsulfanyl)-9-(3-isopropylaminopropyl)-9H-purin-6-ylamine is an HCl salt.

11. The compound or compounds for use according to any preceding claim wherein the microtubule stabilizing agent is selected from docetaxel, paclitaxel, cabazitaxel, ixabepilone, vincristine, laulimalide, discodermolids and epothilones.

12. The compound or compounds for use according to any preceding claim, further comprising administering an HSP70 inhibitor.

13. The compound or compounds for use according to claim 12, wherein the HSP70 inhibitor is administered after the microtubule stabilizing agent.

14. The compound or compounds for use according to claim 13, wherein the HSP70 inhibitor is administered concurrently with or prior to the administration of 8-(6-Iodo-benzo[1,3]dioxol-5-ylsulfanyl)-9-(3-isopropylamino-propyl)-9H-purin-6-ylamine, or a pharmaceutically acceptable saltthereof.

15. The compound or compounds for use according to any preceding claim, wherein the cancer is selected from breast cancer, lung cancer including small cell lung cancer and non-small cell lung cancer, cervical cancer, colon cancer, choriocarcinoma, bladder cancer, cervical cancer, basal cell carcinoma, choriocarcinoma, colon cancer, colorectal cancer, endometrial cancer esophageal cancer, gastric cancer, head and neck cancer, acute lymphocytic cancer (ACL), myelogenous leukemia including acute myeloid leukemia (AML) and chronic myeloid chronic myeloid leukemia (CML), multiple myeloma, T-cell leukemia lymphoma, liver cancer, lymphomas including Hodgkin's disease, lymphocytic lymphomas, neuroblastomas follicular lymphoma and a diffuse large B-cell lymphoma, oral cancer, ovarian cancer, pancreatic cancer, prostate cancer, rectal cancer, sarcomas, skin cancers such as melanoma, testicular cancer, thyroid cancer, renal cancer, myeloproliferative disorders, gastrointestinal cancers including gastrointestinal stromal tumors, esophageal cancer, stomach cancer, a gallbladder cancer, anal cancer, brain tumors including gliomas, lymphomas including follicular lymphoma and diffuse large B-cell lymphoma.

## Patentansprüche

1. Inhibitor von HSP90 zur Verwendung bei einem Verfahren zur Behandlung von Krebs, wobei das Verfahren nach einer Vorbehandlung mit einem proteotoxischen Stressor das Verabreichen des Inhibitors von HSP90 an einen Krebspatienten umfasst, wobei der proteotoxische Stressor rechtzeitig vor der Verabreichung des HSP90-Inhibitors verabreicht wird, um die Bildung des Epichaperoms zu verstärken, wobei der proteotoxische Stressor nicht mehr als 24 Stunden vor der Verabreichung des HSP90-Inhibitors verabreicht wird; wobei der Inhibitor von HSP90 8-(6-Iodbenzo[1,3]dioxol-5-ylsulfanyl)-9-(3-isopropylaminopropyl)-9H-purin-6-ylamin oder ein pharmazeutisch annehmbares Salz davon ist und wobei der proteotoxische Stressor ein Mikrotubulus-Stabilisierungsmittel ist.

2. Proteotoxischer Stressor zur Verwendung bei einem Verfahren zur Behandlung von Krebs, wobei das Verfahren nach einer Vorbehandlung mit dem proteotoxischen Stressor das Verabreichen eines Inhibitors von HSP90 an einen Krebspatienten umfasst, wobei der proteotoxische Stressor rechtzeitig vor der Verabreichung des HSP90-Inhibitors verabreicht wird, um die Bildung des Epichaperoms zu verstärken, wobei der proteotoxische Stressor nicht mehr als 24 Stunden vor der Verabreichung des HSP90-Inhibitors verabreicht wird; wobei der Inhibitor von HSP90 8-(6-Iodbenzo[1,3]dioxol-5-ylsulfanyl)-9-(3-isopropylaminopropyl)-9H-purin-6-ylamin oder ein pharmazeutisch annehmbares Salz davon ist und wobei der proteotoxische Stressor ein Mikrotubulus-Stabilisierungsmittel ist.

3. Inhibitor von HSP90 und proteotoxischer Stressor zur Verwendung bei einem Verfahren zur Behandlung von Krebs, wobei das Verfahren nach einer Vorbehandlung mit dem proteotoxischen Stressor das Verabreichen des Inhibitors von HSP90 an einen Krebspatienten umfasst, wobei der proteotoxische Stressor rechtzeitig vor der Verabreichung des HSP90-Inhibitors verabreicht wird, um die Bildung des Epichaperoms zu verstärken, wobei der proteotoxische Stressor nicht mehr als 24 Stunden vor der Verabreichung des HSP90-Inhibitors verabreicht wird; wobei der Inhibitor von HSP90 8-(6-Iodbenzo[1,3]dioxol-5-ylsulfanyl)-9-(3-isopropylaminopropyl)-9H-purin-6-ylamin oder ein pharmazeutisch annehmbares Salz davon ist und wobei der proteotoxische Stressor ein Mikrotubulus-Stabilisierungsmittel ist.

4. Verbindung oder Verbindungen zur Verwendung nach einem der Ansprüche 1-3, wobei 8-(6-Iodbenzo[1,3]dioxol-5-ylsulfanyl)-9-(3-isopropylaminopropyl)-9H-purin-6-ylamin oder ein pharmazeutisch annehmbares Salz davon mindestens eine Stunde nach dem Verabreichen des Mikrotubulus-Stabilisierungsmittels verabreicht wird.

5. Verbindung oder Verbindungen zur Verwendung nach einem der Ansprüche 1-3, wobei das Verfahren das Verabreichen einer Kombination des proteotoxischen Stressors und des HSP90-Inhibitors über einen Zyklus zwischen 7 und 31 Tagen umfasst, wobei der proteotoxische Stressor und der HSP90-Inhibitor im Zyklus mindestens einmal verabreicht werden und wobei jeder Verabreichung des proteotoxischen Stressors eine Verabreichung des HSP90-Inhibitors folgt.

6. Verbindung oder Verbindungen zur Verwendung nach Anspruch 5, wobei der Behandlungszyklus 7 Tage beträgt.

7. Verbindung oder Verbindungen zur Verwendung nach Anspruch 6, wobei das Mikrotubulus-Stabilisierungsmittel und 8-(6-Iodbenzo[1,3]dioxol-5-ylsulfanyl)-9-(3-isopropylaminopropyl)-9H-purin-6-ylamin oder ein pharmazeutisch annehmbares Salz davon nur an Tag 1 des Behandlungszyklus verabreicht werden.

8. Verbindung oder Verbindungen zur Verwendung nach Anspruch 5, wobei der Behandlungszyklus 21 Tage beträgt.

9. Verbindung oder Verbindungen zur Verwendung nach Anspruch 8, wobei das Mikrotubulus-Stabilisierungsmittel und 8-(6-Iodbenzo[1,3]dioxol-5-ylsulfanyl)-9-(3-isopropylaminopropyl)-9H-purin-6-ylamin oder ein pharmazeutisch annehmbares Salz davon nur an Tag 1 des Behandlungszyklus verabreicht werden.

10. Verbindung oder Verbindungen zur Verwendung nach einem vorstehenden Anspruch, wobei das Salz von 8-(6-Iodbenzo[1,3]dioxol-5-ylsulfanyl)-9-(3-isopropylaminopropyl)-9H-purin-6-ylamin ein HCl-Salz ist.

11. Verbindung oder Verbindungen zur Verwendung nach einem vorstehenden Anspruch, wobei das Mikrotubulus-Stabilisierungsmittel aus Docetaxel, Paclitaxel, Cabazitaxel, Ixabepilon, Vincristin, Laulimalid, Discodermoliden und Epothilonen ausgewählt ist.

12. Verbindung oder Verbindungen zur Verwendung nach einem vorstehenden Anspruch, die weiterhin das Verabreichen eines HSP70-Inhibitors umfasst bzw. umfassen.

13. Verbindung oder Verbindungen zur Verwendung nach Anspruch 12, wobei der HSP70-Inhibitor nach dem Mikrotubulus-Stabilisierungsmittel verabreicht wird.

14. Verbindung oder Verbindungen zur Verwendung nach Anspruch 13, wobei der HSP70-Inhibitor gleichzeitig mit oder vor der Verabreichung von 8-(6-Iodbenzo[1,3]dioxol-5-ylsulfanyl)-9-(3-isopropylaminopropyl)-9H-purin-6-ylamin oder eines pharmazeutisch annehmbaren Salzes davon verabreicht wird.

15. Verbindung oder Verbindungen zur Verwendung nach einem vorstehenden Anspruch, wobei der Krebs ausgewählt ist aus Brustkrebs, Lungenkrebs einschließlich kleinzelligem Lungenkarzinom und nicht-kleinzelligem Lungenkarzinom, Zervixkarzinom, Kolonkarzinom, Choriokarzinom, Blasenkrebs, Zervixkarzinom, Basalzellkarzinom, Choriokarzinom, Kolonkarzinom, Kolorektalkarzinom, Endometriumkarzinom, ösophagealem Karzinom, Magenkrebs, Kopf-Hals-Karzinom, akutem lymphatischem Krebs (ACL), myelogener Leukämie einschließlich akuter myeloischer Leukämie (AML) und chronischer myelogener chronischer myelogener Leukämie (CML), multiplem Myelom, T-Zell-Leukämie/Lymphom, Leberkrebs, Lymphomen einschließlich Morbus Hodgkin, lymphozytischen Lymphomen, Neuroblastomen, follikulärem Lymphom und einem diffusen großzelligen B-Zell-Lymphom, Mundkrebs, Ovarialkrebs, Pankreaskrebs, Prostatakrebs, Rektalkarzinom, Sarkomen, Hautkrebserkrankungen wie einem Melanom, Hodenkrebs, Schilddrüsenkrebs, Nierenkrebs, myeloproliferativen Krankheiten, Gastrointestinalkarzinomen einschließlich Tumoren des Gastrointestinalstromas, ösophagealem Karzinom, Magenkrebs, einem Gallenblasenkarzinom, Analkarzinom, Hirnkarzinomen einschließlich Gliomen, Lymphomen einschließlich follikulärem Lymphom und diffusem großzelligem B-Zell-Lymphom.

## Revendications

1. Inhibiteur de HSP90 pour une utilisation dans un procédé de traitement d'un cancer, le procédé comprenant l'administration à un patient cancéreux de l'inhibiteur de HSP90 à la suite d'un prétraitement avec un stresseur protéotoxique, le stresseur protéotoxique étant administré à un temps suffisant avant l'administration de l'inhibiteur d'HSP90 pour augmenter la formation de l'épichapérome, le stresseur protéotoxique étant administré pas plus de 24 heures avant l'administration de l'inhibiteur de HSP90 ; l'inhibiteur de HSP90 étant la 8-(6-iodo-benzo[1,3]dioxol-5-ylsulfanyl)-9-(3-isopropylamino-propyl)-9H-purin-6-ylamine, ou un sel pharmaceutiquement acceptable correspondant, et le stresseur protéotoxique étant un agent de stabilisation de microtubules.

2. Stresseur protéotoxique pour une utilisation dans un procédé de traitement d'un cancer, le procédé comprenant l'administration à un patient cancéreux d'un inhibiteur de HSP90 à la suite d'un prétraitement avec le stresseur protéotoxique, le stresseur protéotoxique étant administré à un temps suffisant avant l'administration de l'inhibiteur d'HSP90 pour augmenter la formation de l'épichapérome, le stresseur protéotoxique étant administré pas plus de 24 heures avant l'administration de l'inhibiteur de HSP90 ; l'inhibiteur de HSP90 étant la 8-(6-iodo-benzo[1,3]dioxol-5-ylsulfanyl)-9-(3-isopropylamino-propyl)-9H-purin-6-ylamine, ou un sel pharmaceutiquement acceptable correspondant, et le stresseur protéotoxique étant un agent de stabilisation de microtubules.

3. Inhibiteur de HSP90 et stresseur protéotoxique pour une utilisation dans un procédé de traitement d'un cancer, le procédé comprenant l'administration à un patient cancéreux de l'inhibiteur de HSP90 à la suite d'un prétraitement avec le stresseur protéotoxique, le stresseur protéotoxique étant administré à un temps suffisant avant l'administration de l'inhibiteur d'HSP90 pour augmenter la formation de l'épichapérome, le stresseur protéotoxique étant administré pas plus de 24 heures avant l'administration de l'inhibiteur de HSP90 ; l'inhibiteur de HSP90 étant la 8-(6-iodo-benzo[1,3]dioxol-5-ylsulfanyl)-9-(3-isopropylamino-propyl)-9H-purin-6-ylamine, ou un sel pharmaceutiquement acceptable correspondant, et le stresseur protéotoxique étant un agent de stabilisation de microtubules.

4. Composé ou composés pour une utilisation selon l'une quelconque des revendications 1 à 3, la 8-(6-iodo-benzo[1,3]dioxol-5-ylsulfanyl)-9-(3-isopropylamino-propyl)-9H-purin-6-ylamine, ou un sel pharmaceutiquement acceptable correspondant, étant administré au moins une heure après l'administration de l'agent de stabilisation de microtubules.

5. Composé ou composés pour une utilisation selon l'une quelconque des revendications 1 à 3, le procédé comprenant l'administration d'une combinaison du stresseur protéotoxique et de l'inhibiteur de HSP90 sur un cycle d'entre 7 et 31 jours, le stresseur protéotoxique et l'inhibiteur de HSP90 étant administrés au moins une fois pendant ledit cycle, et chaque administration dudit stresseur protéotoxique étant suivie par l'administration dudit inhibiteur de HSP90.

6. Composé ou composés pour une utilisation selon la revendication 5, le cycle de traitement étant de 7 jours.

7. Composé ou composés pour une utilisation selon la revendication 6, l'agent de stabilisation de microtubules et la 8-(6-iodo-benzo[1,3]dioxol-5-ylsulfanyl)-9-(3-isopropylamino-propyl)-9H-purin-6-ylamine, ou un sel pharmaceutiquement acceptable correspondant, étant administrés seulement au jour 1 du cycle de traitement.

8. Composé ou composés pour une utilisation selon la revendication 5, le cycle de traitement étant de 21 jours.

9. Composé ou composés pour une utilisation selon la revendication 8, l'agent de stabilisation de microtubules et la 8-(6-iodo-benzo[1,3]dioxol-5-ylsulfanyl)-9-(3-isopropylamino-propyl)-9H-purin-6-ylamine, ou un sel pharmaceutiquement acceptable correspondant, étant administrés seulement au jour 1 du cycle de traitement.

10. Composé ou composés pour une utilisation selon une quelconque revendication précédente, le sel de la 8-(6-iodo-benzo[1,3]dioxol-5-ylsulfanyl)-9-(3-isopropylamino-propyl)-9H-purin-6-ylamine étant un sel de HCl.

11. Composé ou composés pour une utilisation selon une quelconque revendication précédente, l'agent de stabilisation de microtubules étant choisi parmi le docétaxel, le paclitaxel, le cabazitaxel, l'ixabépilone, la vincristine, le laulimalide, des discodermolides et des épothilones.

12. Composé ou composés pour une utilisation selon une quelconque revendication précédente, comprenant en outre l'administration d'un inhibiteur de HSP70.

13. Composé ou composés pour une utilisation selon la revendication 12, l'inhibiteur de HSP70 étant administré après l'agent de stabilisation de microtubules.

14. Composé ou composés pour une utilisation selon la revendication 13, l'inhibiteur de HSP70 étant administré simultanément avec ou avant l'administration de la 8-(6-iodo-benzo[1,3]dioxol-5-ylsulfanyl)-9-(3-isopropylamino-propyl)-9H-purin-6-ylamine, ou d'un sel pharmaceutiquement acceptable correspondant.

15. Composé ou composés pour une utilisation selon une quelconque revendication précédente, le cancer étant choisi parmi un cancer du sein, un cancer du poumon y compris un cancer du poumon à petites cellules et un cancer du poumon non à petites cellules, un cancer du col de l'utérus, un cancer du côlon, un choriocarcinome, un cancer de la vessie, un cancer du col de l'utérus, un carcinome de cellules basales, un choriocarcinome, un cancer du côlon, un cancer colorectal, un cancer endométrial, un cancer de l'œsophage, un cancer gastrique, un cancer de la tête et du cou, un cancer lymphocytaire aigu (CLA), une leucémie myélogène y compris une leucémie myéloïde aiguë (LMA) et une leucémie myéloïde chronique myéloïde chronique (LMC), un myélome multiple, une leucémie à cellules T, un lymphome, un cancer du foie, des lymphomes y compris la maladie de Hodgkin, des lymphomes lymphocytaires, des neuroblastomes, un lymphome folliculaire et un lymphome diffus à grandes cellules B, un cancer oral, un cancer de l'ovaire, un cancer pancréatique, un cancer de la prostate, un cancer rectal, des sarcomes, des cancers de la peau tels qu'un mélanome, un cancer testiculaire, un cancer de la thyroïde, un cancer rénal, des troubles myéloprolifératifs, des cancers gastro-intestinaux y compris des tumeurs stromales gastro-intestinales, un cancer de l'œsophage, un cancer de l'estomac, un cancer de la vésicule biliaire, un cancer anal, des tumeurs au cerveau y compris des gliomes, des lymphomes y compris un lymphome folliculaire et un lymphome diffus à grandes cellules B.
